# EUROPEAN PATENT SPECIFICATION

(11) **EP 1 602 718 B1**
(45) Date of publication and mention of the grant of the patent: **15.10.2008**
(21) Application number: 04717230.9
(22) Date of filing: 04.03.2004
(51) Int. Cl.: C12N 9/02, C07K 14/37, C07K 14/375, D06P 3/08

(54) **NEUTRAL PHENOL OXIDASE**
NEUTRALE PHENOLOXIDASE
PHENOL OXYDASE NEUTRE

(30) Priority: 07.03.2003 JP 2003062454
(43) Date of publication of application: 07.12.2005
(73) Proprietor: Mandom Corporation, Osaka-shi, Osaka 540-8530 (JP)
(72) Inventor: Tsujino, Yoshio, Kobe-shi, Hyogo 658-0003 (JP); Endo, Katsunori, Kobe-shi, Hyogo 658-0051 (JP); Hasan, Abul Khaer Mohamad Quamrul, Kobe-shi, Hyogo 658-0032 (JP); OTSUKA, Kaori, Kanazawa-shi, Ishikawa 921-8135 (JP)
(74) Representative: Vossius & Partner
(86) International application number: PCT/JP2004/002725
(87) International publication number: WO 2004/078958

(56) References cited:
- WO-A-97/28257
- WO-A1-20/04020617
- JP-A- 9 206 071
- JP-A- 10 501 137
- JP-A- 2001 514 513
- KIISKINEN L-L ET AL: "PURIFICATION AND CHARACTERIZATION OF A NOVEL LACCASE FROM THE ASCOMYCETE MELANOCARPUS ALBOMYCES" APPLIED MICROBIOLOGY AND BIOTECHNOLOGY, SPRINGER VERLAG, BERLIN, DE, vol. 59, no. 2/3, July 2002 (2002-07), pages 198-204, XP001179500 ISSN: 0175-7598
- PALMIERI GIANNA ET AL: "A novel white laccase from Pleurotus ostreatus" JOURNAL OF BIOLOGICAL CHEMISTRY, vol. 272, no. 50, 12 December 1997 (1997-12-12), pages 31301-31307, XP002378261 ISSN: 0021-9258
- ENDO KOHKI ET AL: "A novel extracytoplasmic phenol oxidase of Streptomyces: Its possible involvement in the onset of morphogenesis" MICROBIOLOGY (READING), vol. 148, no. 6, June 2002 (2002-06), pages 1767-1776, XP002387762 ISSN: 1350-0872
- MARQUES DE SOUZA CRISTINA GIATTI ET AL: "Purification and characterization of the main laccase produced by the white-rot fungus Pleurotus pulmonarius on wheat bran solid state medium." JOURNAL OF BASIC MICROBIOLOGY, vol. 43, no. 4, 2003, pages 278-286, XP002378262 ISSN: 0233-111X
- ENDO KOHKI ET AL: "Enzymological characterization of EpoA, a laccase-like phenol oxidase produced by Streptomyces griseus." JOURNAL OF BIOCHEMISTRY (TOKYO), vol. 133, no. 5, May 2003 (2003-05), pages 671-677, XP002387763 ISSN: 0021-924X
- GARZILLO ANNA MARIA ET AL: "Structural and kinetic characterization of native laccases from Pleurotus ostreatus, Rigidoporus lignosus, and Trametes trogii" JOURNAL OF PROTEIN CHEMISTRY, vol. 20, no. 3, April 2001 (2001-04), pages 191-201, XP002387764 ISSN: 0277-8033
- ISHIGAMI T ET AL: "CHARACTERIZATION OF POLYPHENOL OXIDASE FROM CHAETOMIUM THERMOPHILE, A THERMOPHILIC FUNGUS" JOURNAL OF GENERAL AND APPLIED MICROBIOLOGY, XX, XX, vol. 54, no. 5, 1988, pages 401-408, XP009065582
- DATABASE BIOSIS [Online] LEEE J-S. ET AL: 'Production and enzymatic properties of laccase from flammulina-velutipes', XP002903665 Database accession no. prev198580089830 & KOREAN JOURNAL OF MYCOLOGY vol. 13, no. 2, 1985, pages 111 - 114

## Description

The present invention relates to a neutral phenol oxidase showing small changes depending on a substrate in each optimum pH for various substrates and having high enzymatic activity at pH around neutral region, a method for producing the same, and an antibody raised against the neutral phenol oxidase. More specifically, the present invention relates to a neutral phenol oxidase useful for dyeing fibers or coloring hair, and the like, a method for producing the same, and an antibody capable of specifically recognizing the neutral phenol oxidase.

Oxidation enzymes having oxidizing action on various substrates such as phenolic compounds and polyphenol compounds are roughly classified into two groups, peroxidase and phenol oxidase.

The above-mentioned peroxidase catalyzes the oxidation of various substrates and requires the presence of hydrogen peroxide as a common substrate in a reaction system. On the other hand, the phenol oxidase catalyzes the oxidation of various substrates and requires the presence of molecular oxygen as a common substrate.

Therefore, among known oxidation enzymes, since the above-mentioned phenol oxidase can catalyze the oxidation of various substrates in the presence of atmospheric oxygen, the phenol oxidase is suitable for various chemical reactions such as coloring, decolorization, polymerization, and decomposition, which are caused by the generation of radical species as intermediates in the presence of oxygen.

Therefore, it is expected that such phenol oxidase will be widely applied to various fields such as clinical analysis, biosensors, bleaching of pulp and fibers, manufacture of laminated wood, improvement in the quality of wood, production of phenolic resins, production of artificial lacquer paints, production of adhesives, synthesis of organic compounds, dyeing and discharging, prevention of color transfer during washing, elimination of phenol and aniline compounds from waste water, detoxication of toxic compounds, decomposition of endocrine disrupting chemicals, prevention of turbidity in juice, removal of bitterness and astringency from food, acceleration of digestion of livestock food in domestic animals, suppression of production of an effluvium, desulfurization of fossil fuels, and decomposition treatment of hazardous environmental substances.

In addition, it becomes possible to efficiently carry out reactions that are originally difficult to catalyze (or reactions that are impossible to catalyze) (WO 96/16165) by reaction using mediators such as 1-hydroxybenzotriazole, 2,2'-azino-bis(3-ethylbenzothiazoline-6-sulfonic acid) (hereinafter, abbreviated as ABTS), and 1-nitroso-2-naphthol-3,6-disulfonic acid (hereinafter, abbreviated as NNS).

For example, it is known that when phenothiazine-10-propionic acid is used as a mediator, decomposition reaction of indigo by phenol oxidase can be efficiently carried out. In addition, it has been disclosed that decomposition of dioxin can be performed by using a phenol oxidase-containing substance and a composition containing a phenol oxidase mediator in combination (Japanese Patent Laid-open No. 2001-037465).

As for many potential industrial applications, the neutral and alkaline regions are suitable for reaction pH, from the viewpoint of reaction efficiency and the like. Particularly, there are many advantages in the applications in the neutral region such that a reaction under mild conditions that have little effect on the environment, the human body, and the like, can be carried out.

The phenol oxidase includes laccase, tyrosinase, polyphenol oxidase (catechol oxidase), ascorbic acid oxidase, bilirubin oxidase, and the like.

However, tyrosinase is characterized in that the tyrosinase catalyzes the direct oxidation (coloring) of monophenolic compounds and ortho-diphenol compounds but does not catalyze the oxidation of para-diphenol compounds and para-diamine (phenylenediamine) compounds (Mayer and Harel, Phytochem. 18, 193-215, 1979).

The phenol oxidase has been previously found in various plants, bacteria, fungi, and the like. For example, as for plants, the phenol oxidase has been found in, secretory vessels of Anacardiaceae, peach, chestnut, Podocarpaceae, and the like. As for fungi, the phenol oxidase has been found in, for example, *Aspergillus*, *Botrytis*, *Myrothecium*, *Penicillium*, *Pestalotia*, and *Rhizoctonia* belonging to the subphylum Deuteromycotina; *Pleurotus*, *Lentinus*, *Polyporus*, *Trametes*, and *Coriolus* belonging to the subphylum Basidiomycotina; and *Podospora*, *Neurospora*, and *Monocillium* belonging to the subphylum Ascomycotina. As for bacteria, the phenol oxidase has been found in, for example, *Bacillus*, *Azospirillum*, *Streptomyces*, and *Aerobacter* (Yoshida, J. Chem. Soc. 43, 472, 1883).

Among them, as for fungi belonging to Basidiomysetes (e.g., wood-rotting fungi, especially white-rot fungi), the phenol oxidase has been found in, for example, *Schizophyllum commune, Coriolus versicolor, Pycnoporus coccineus, Pleurotus octreatus,* and *Fomitella fraxinea* (Ullah and Evans, Appl. Microbiol. Biotechnol., 53, 230-234, 2000).

However, since many phenol oxidases have optimum pHs for enzymatic activity for various substances in the acidic region, there are disadvantages of the limitation in applications. In addition, even though phenol oxidases each having an optimum pH in the neutral or alkaline region are used, such phenol oxidases have disadvantages such that their respective optimum pHs are greatly varied depending on a substrate used so that the phenol oxidases do not efficiently act on various substrates at pH around neutral region.

Further, laccase has been found in *Irpex lacteus*, *Auricularia polytricha*, *Ganoderm lucidum*, *Coprinus micaceus*, *Daedaleopsis styracina*, and *Flammulina veltipes.* Among them, it has been found that a laccase from *Irpex lacteus* shows activity of oxidizing to condense 4-aminoantipyrine and phenol under the reaction condition at pH of around 6.0, resulting in color development (Japanese Patent Laid-open No. Sho 60-156385).

However, the laccase is an enzyme found by cultivation under acidic conditions.

An object of the present invention is to provide a means by which dyeing of fibers, coloring of hair or the like can be achieved, and by which reduced effects on the environment, the human body, and the like can be achieved. An object of the present invention is to provide a phenol oxidase, particularly a neutral phenol oxidase having the following characteristics: having an optimum pH at around neutral region for various compounds, especially for phenolic compounds, aminophenol compounds, and diamine compounds; showing high stability in a wide pH range from neutral to mildly alkaline pH; and showing a small changes depending on an substrate in among optimum pH. In addition, an object of the present invention is to provide a method for producing efficiently and easily the neutral phenol oxidase in large quantity at low cost. Further, an object of the present invention is to provide an antibody, by which collection and purification of the neutral phenol oxidase, and the like can be achieved. In addition, an object of the present invention is to provide a dyeing method by which dyeing with various dyes, concretely, phenolic compounds, aminophenol compounds, diamine compounds, and the like can be achieved and a dyeing composition of which handling is easy.

Namely, the gist of the present invention relates:
[1] a neutral phenol oxidase having the following properties:
   (1) optimum pH: being from 5.0 to 7.0,
   (2) substrate specificity:
      i) catalyzing a coloring reaction by an oxidation of each of N,N-dimethyl-para-phenylenediamine, ortho-aminophenol, 2,6-dimethoxyphenol, 1,3-dihydroxynaphthol and 4-hydroxyindole at a pH of around 6.5; and
      ii) catalyzing an oxidative polymerization reaction of alkali extract of lignin;
   (3) 28 kDa (calculated by SDS-PAGE),
   (4) pH stability: maintaining a relative remaining activity of at least 70 % under incubation conditions at a pH of 8.0 to 9.0 for 20 hours at 30°C,
   (5) optimum temperature: being from 30° to 50°C,
   (6) thermal stability:
      I) maintaining a relative remaining activity of at least 80 % under incubation conditions at 0° to 30°C for 1 hour at a pH of 7.0, as compared with an activity before incubation, and
      II)maintaining a relative remaining activity of at least 90 % under incubation conditions at 0 to 30°C for 1 hour at a pH of 9.0, as compared with an activity before incubation, and
   (7) isoelectric point: being 7.4.
[2] the neutral phenol oxidase according to the above item [1], wherein the neutral phenol oxidase is produced by a basidiomycete belonging to the genus *Flammulina;*
[3] the neutral phenol oxidase according to the above item [1] or [2], wherein the basidiomycete belonging to the genus *Flammulina* is a basidiomycete belonging to *Flammulina velutipes*;
[4] the neutral phenol oxidase according to any one of the above items [1] to [3], wherein the basidiomycete belonging to *Flammulina velutipes* is *Flammulina velutipes* IFO 30601 strain.
[5] a production method of a neutral phenol oxidase, characterized in that (A) a basidiomycete belonging to the genus *Flammulina* velutipes IFO 00601 is cultured at a pH of 6.0 to 12.0, a neutral phenoloxidase is separated from a culture supernatant of a culture obtained in step (A) or an extract obtained from the culture ; wherein the neutral phenol oxidase is the neutral phenol oxidase of the above item [1] ;
[6] an antibody raised against the neutral phenol oxidase of any one of the above items [1] to [4];
[7] a dyeing composition comprising the neutral phenol oxidase of any one of the above items [1] to [4];
[8] the dyeing composition according to the above item [7], further comprising a dye; and
[9] a dyeing method, characterized in that a subject to be dyed is brought into contact with a dye in the presence of the neutral phenol oxidase of any one of the above items [1] to [4], thereby dyeing the subject to be dyed.

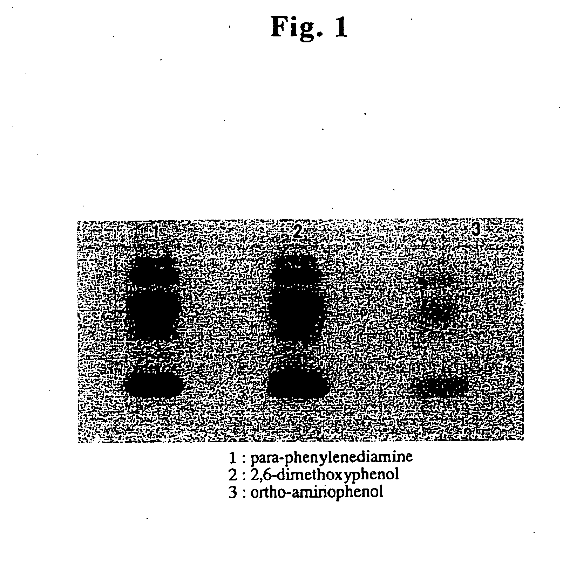

Fig. 1 shows the result of activity staining of DEAE-cellulose elution fraction from *Flammulina velutipes* IFO 30601'strain. In the figure, lane 1 shows the result of activity staining using para-phenylenediamine, lane 2 being the result of activity staining using 2,6-dimethoxyphenol, and lane 3 being the result of activity staining using ortho-aminophenol, respectively.

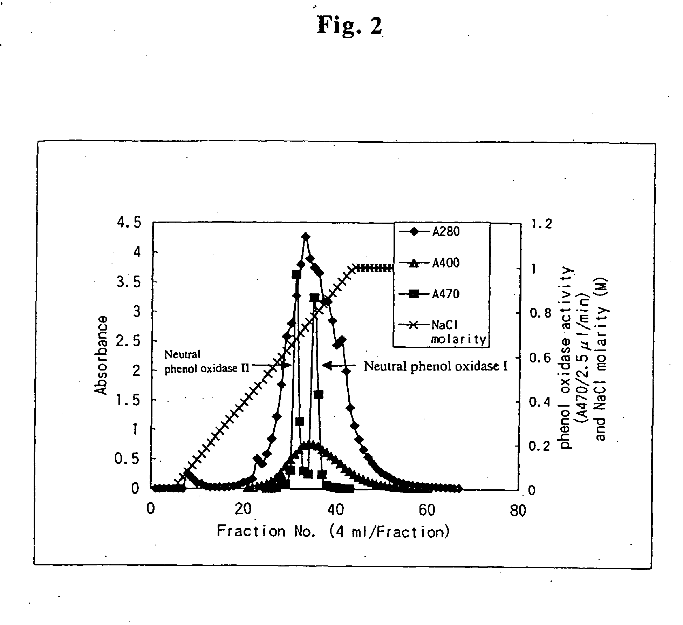

Fig. 2 shows the chromatogram of ion-exchange chromatography. In Fig. 2, the rhombus shows a change in an amount of protein contained in each fraction expressed on the basis of the absorbance at 280 nm (A280), the triangle being a change in an amount of glucide contained in each fraction expressed on the basis of the absorbance at 400 nm (A400), the square being a change in the enzymatic activity in each fraction expressed on the basis of the absorbance at 470 nm (A470), and the cross being the concentration (M) of sodium chloride.

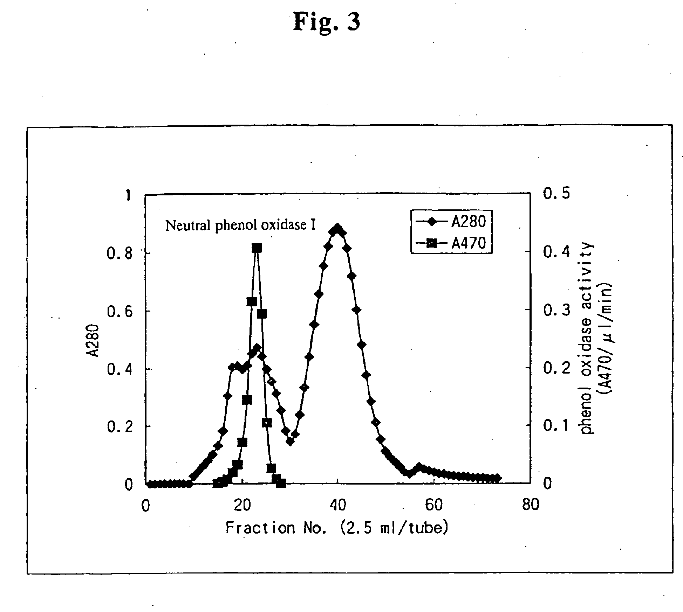

Fig. 3 shows the chromatogram of gel filtration chromatography of the neutral phenol oxidase I. In Fig. 3, the rhombus shows a change in an amount of protein contained in each fraction expressed on the basis of the absorbance at 280 nm (A280), and the square being a change in the enzymatic activity in each fraction expressed on the basis of the absorbance at 470 nm (A470).

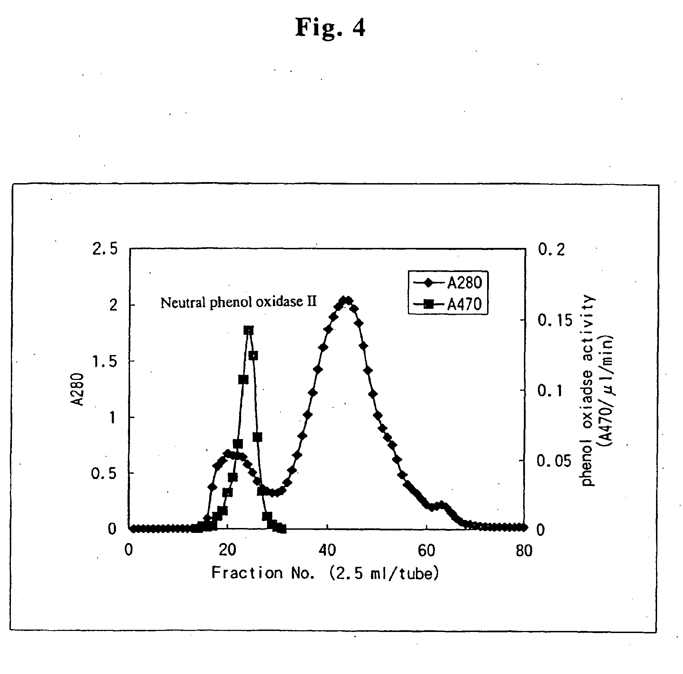

Fig. 4 shows the chromatogram of gel filtration chromatography of the neutral phenol oxidase II. In Fig. 3, the rhombus shows a change in an amount of protein contained in each fraction expressed on the basis of the absorbance at 280 nm (A280), and the square being a change in the enzymatic activity in each fraction expressed on the basis of the absorbance at 470 nm (A470).

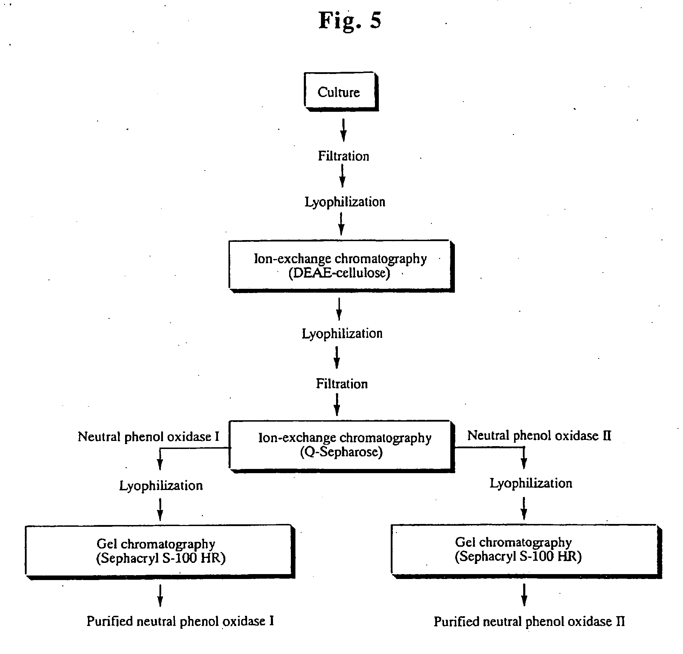

Fig. 5 shows the scheme of purification of the neutral phenol oxidase I and the neutral phenol oxidase II.

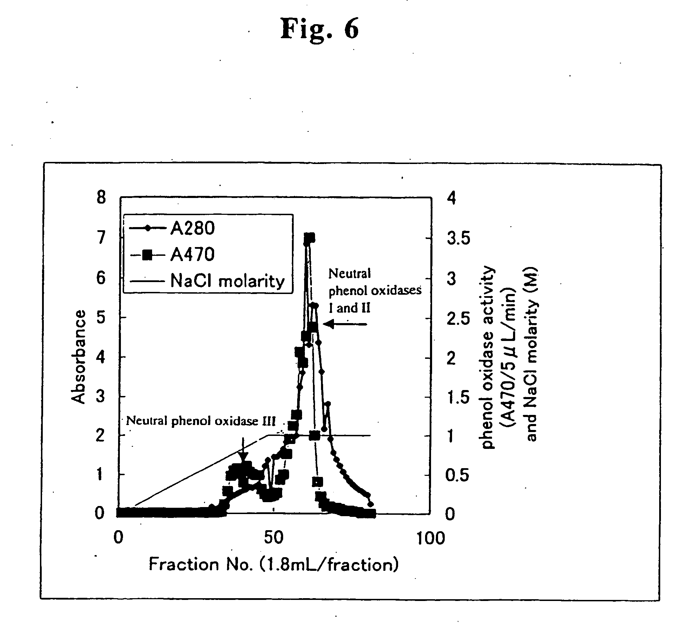

Fig. 6 shows the chromatogram of ion-exchange chromatography (DEAE-cellulose) of the neutral phenol oxidase III. In Fig. 6, the rhombus shows a change in an amount of protein contained in each fraction expressed on the basis of the absorbance at 280 nm (A280), the square being a change in the enzymatic activity in each fraction expressed on the basis of the absorbance at 470 nm (A470), and the right line being the concentration (M) of sodium chloride.

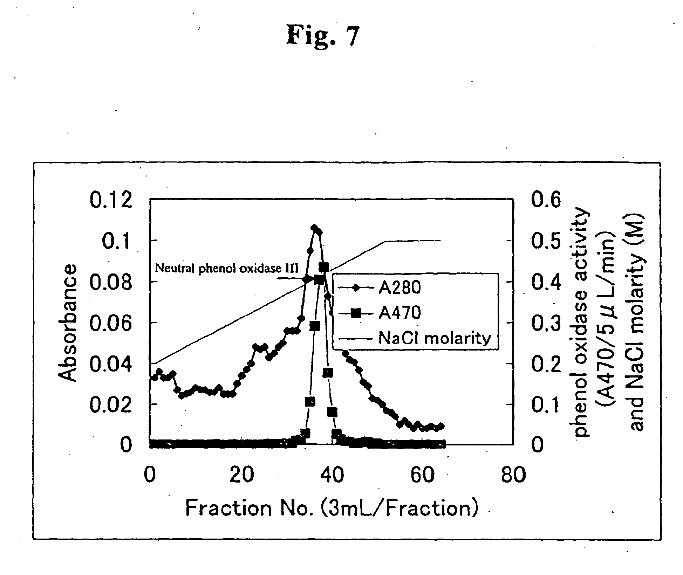

Fig. 7 shows the chromatogram of ion-exchange chromatography (Q-Sepharose) of the neutral phenol oxidase III. In Fig. 7, the rhombus shows a change in an amount of protein contained in each fraction expressed on the basis of the absorbance at 280 nm (A280), the square being a change in the enzymatic activity in each fraction expressed on the basis of the absorbance at 470 nm (A470), and the right line being the concentration (M) of sodium chloride.

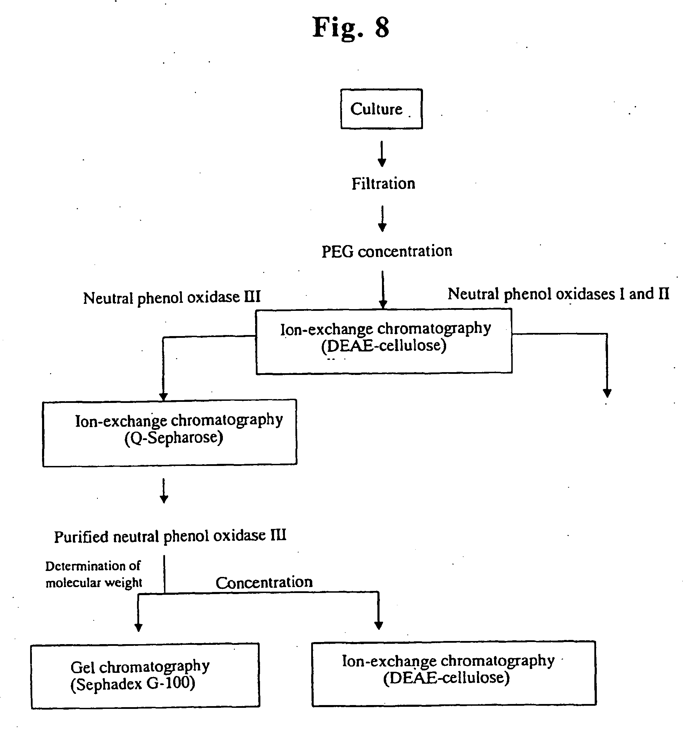

Fig. 8 shows the scheme of purification of the neutral phenol oxidase III.

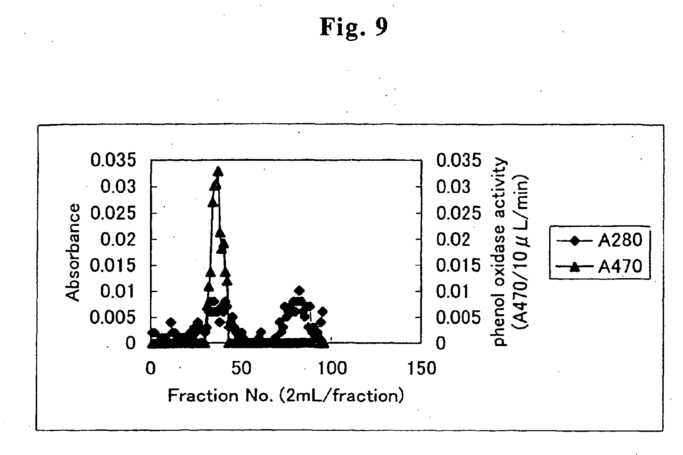

Fig. 9 shows the chromatogram of gel filtration chromatography of the neutral phenol oxidase III. In Fig. 9, the rhombus shows a change in an amount of protein contained in each fraction expressed on the basis of the absorbance at 280 nm (A280), and the square being a change in the enzymatic activity in each fraction expressed on the basis of the absorbance at 470 nm (A470).

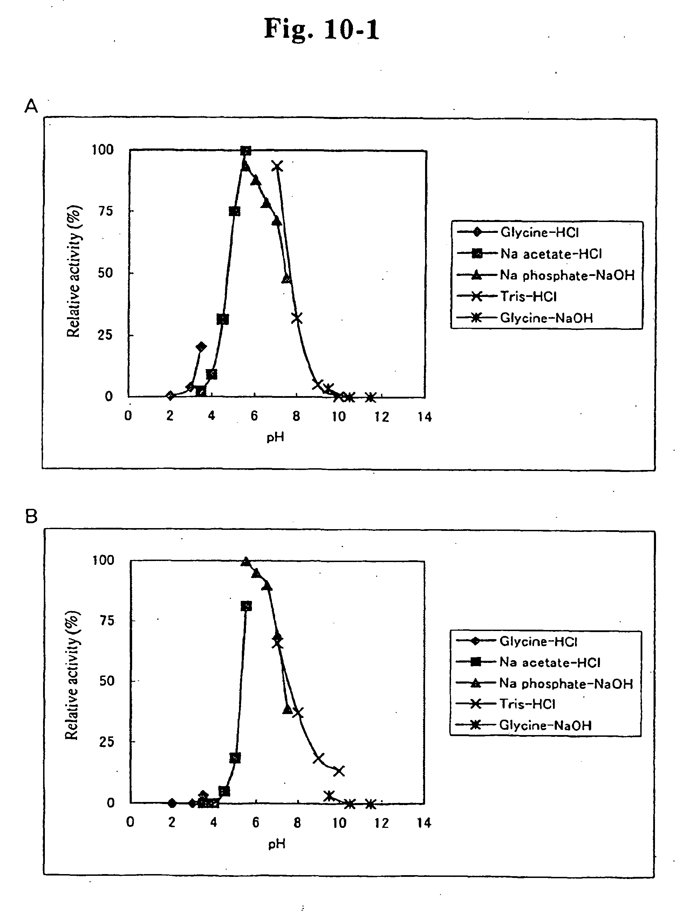

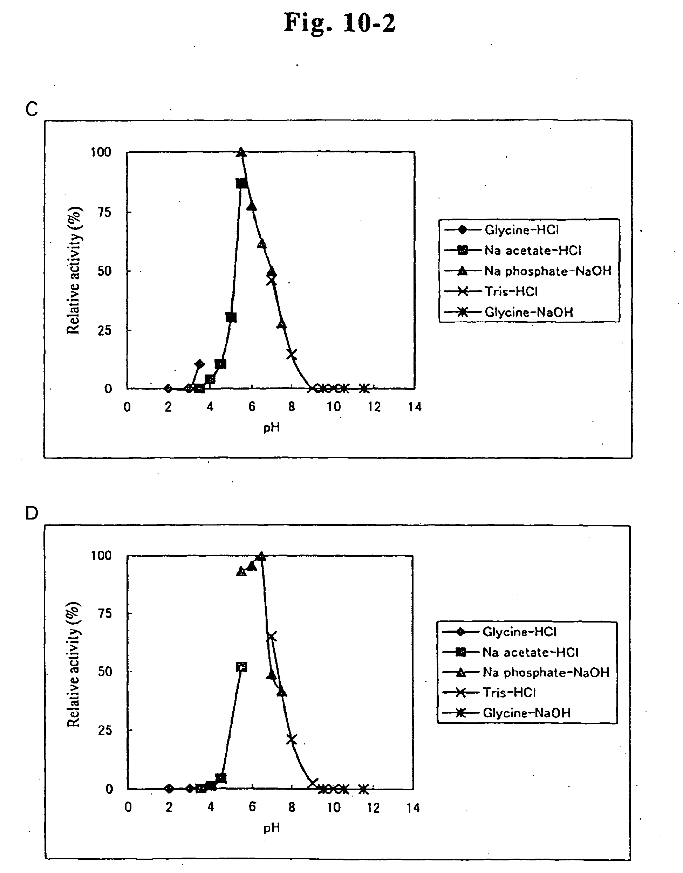

Fig. 10 shows the result of the optimum pH of the neutral phenol oxidase I. Fig. 10 is shown by a relative activity determined when the maximum activity is defined as 100. In addition, in Fig. 10, panel A shows the case where 2,6-dimethoxyphenol was used as a substrate, panel B being the case where ortho-aminophenol was used as a substrate, panel C being the case where para-phenylenediamine was used as a substrate, and panel D being the case where syringaldazine was used as a substrate.

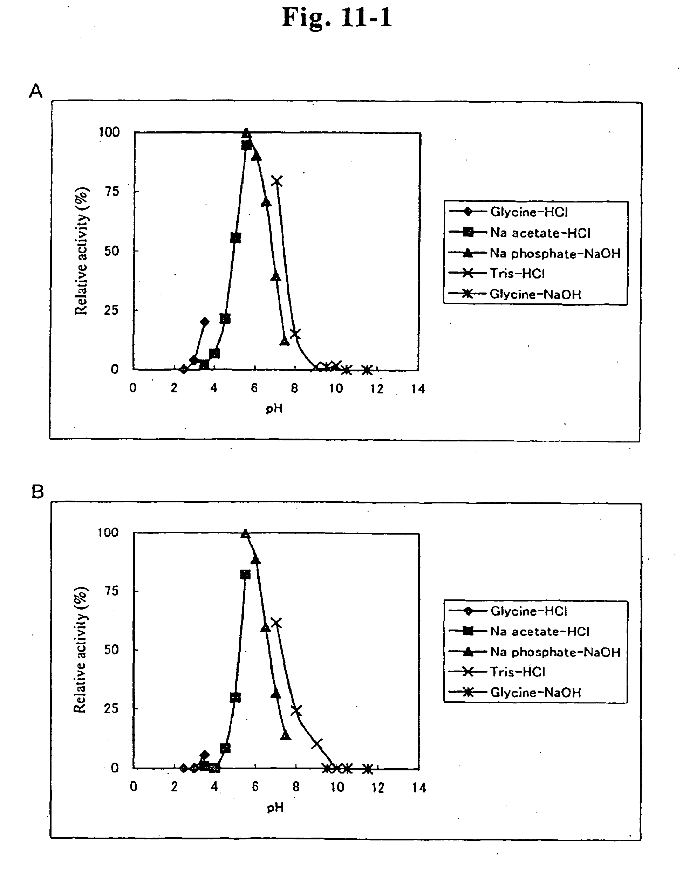

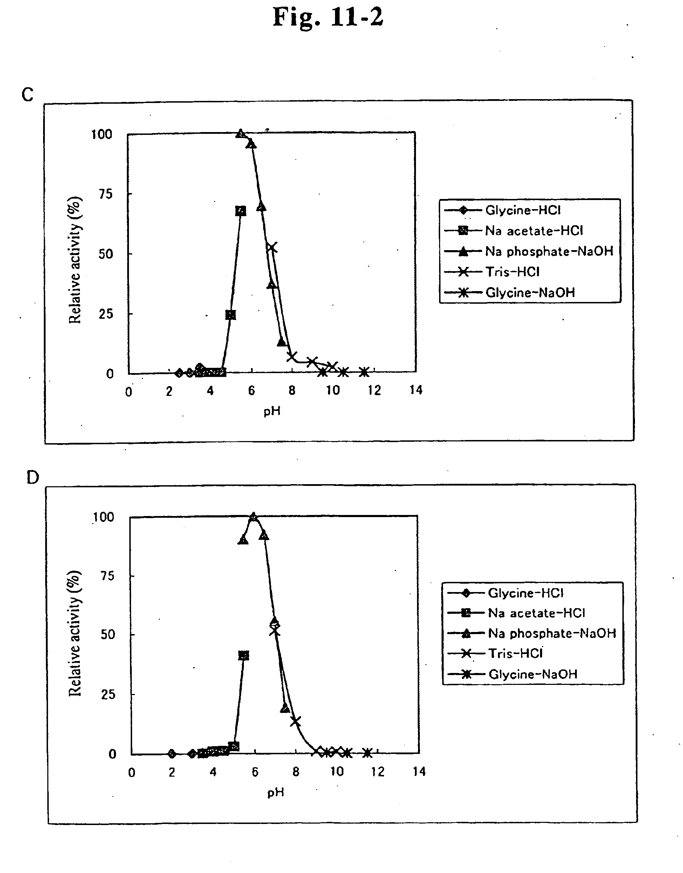

Fig. 11 shows the result of the optimum pH of the neutral phenol oxidase II. Fig. 11 is shown by a relative activity determined when the maximum activity is defined as 100. In addition, in Fig. 11, panels A shows the case where 2,6-dimethoxyphenol was used as a substrate, panel B being the case where ortho-aminophenol was used as a substrate, panel C being the case where para-phenylenediamine was used as a substrate, and panel D being the case where syringaldazine was used as a substrate.

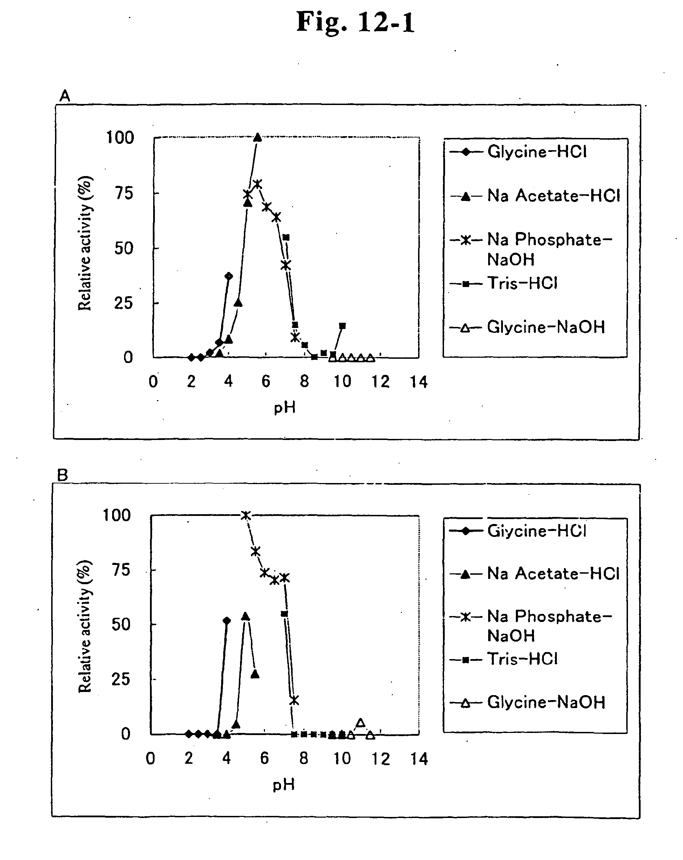

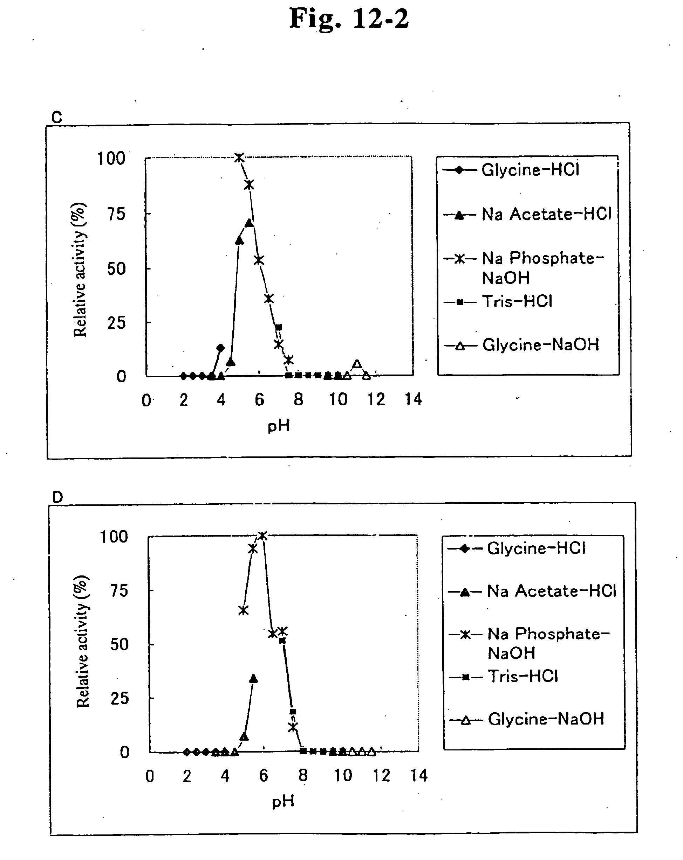

Fig. 12 shows the result of the optimum pH of the neutral phenol oxidase III. Fig. 12 is shown by a relative activity determined when the maximum activity is defined as 100. In addition, panels A shows the case where 2,6-dimethoxyphenol was used as a substrate, panel B being the case where ortho-aminophenol was used as a substrate, panel C being the case where para-phenylenediamine was used as a substrate, and panel D being the case where syringaldazine was used as a substrate.

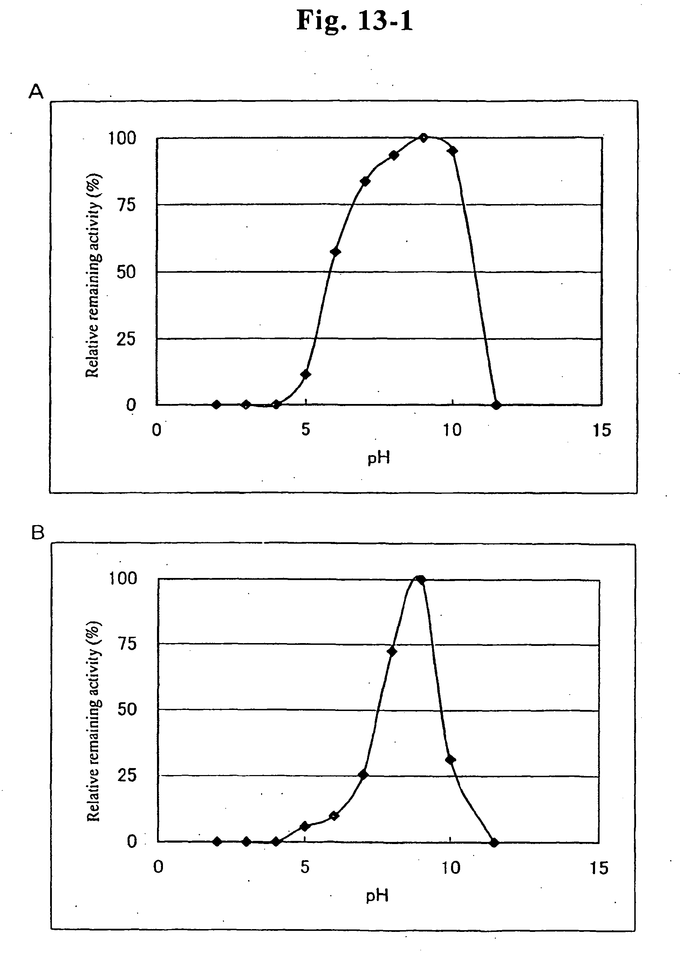

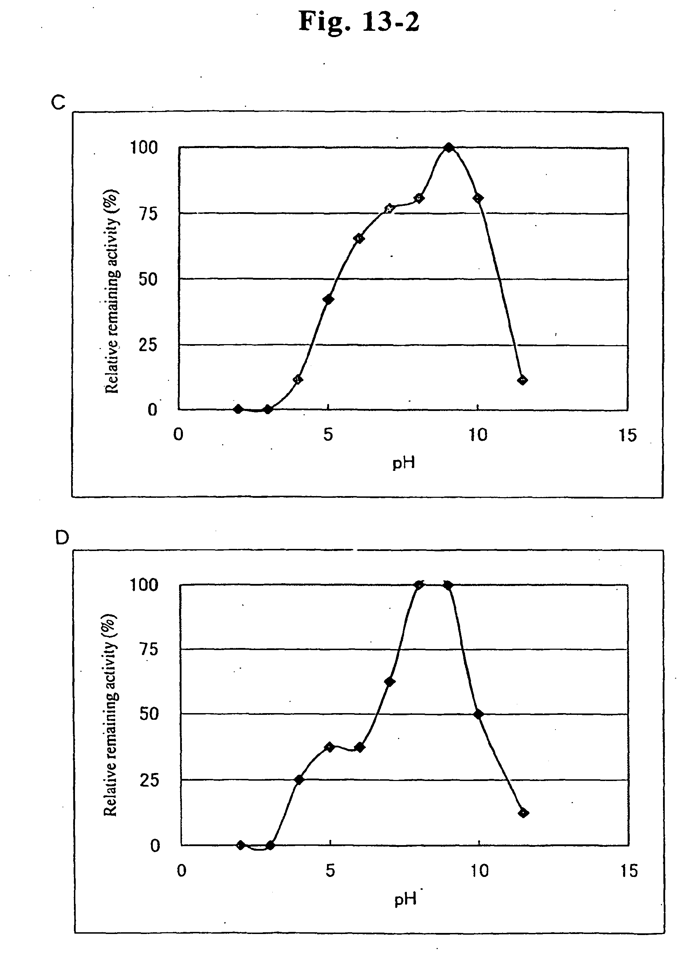

Fig. 13 shows the result of the pH stability of the neutral phenol oxidase I. Fig. 13 is shown by a relative remaining activity determined when the maximum activity is defined as 100. In addition, panels A and B show cases where 2,6-dimethoxyphenol was used as a substrate, and panels C and D being cases where para-phenylenediamine was used as a substrate. Further, in Fig. 13, panels A and C show the pH stability after 1-hour incubation, and panels B and D being the pH stability after 20-hour incubation.

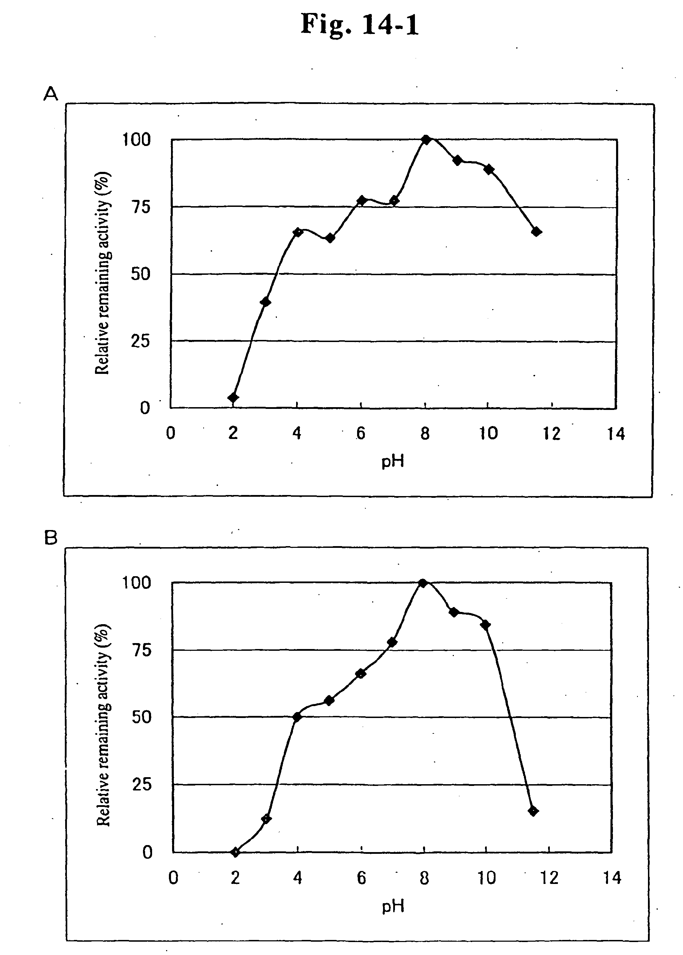

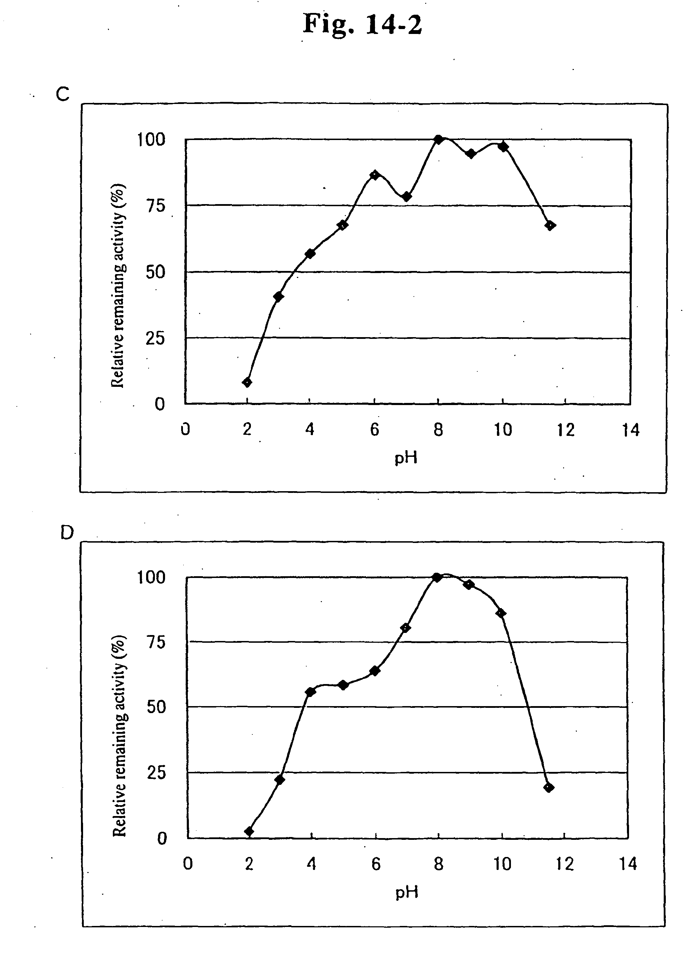

Fig. 14 shows the result of the pH stability of the neutral phenol oxidase II. Fig. 14 is shown by a relative remaining activity determined when the maximum activity is defined as 100. In addition, panels A and B show cases where 2,6-dimethoxyphenol was used as a substrate, and panels C and D being cases where para-phenylenediamine was used as a substrate. Further, in Fig. 14, panels A and C show the pH stability after 1-hour incubation, and panels B and D being the pH stability after 20-hour incubation.

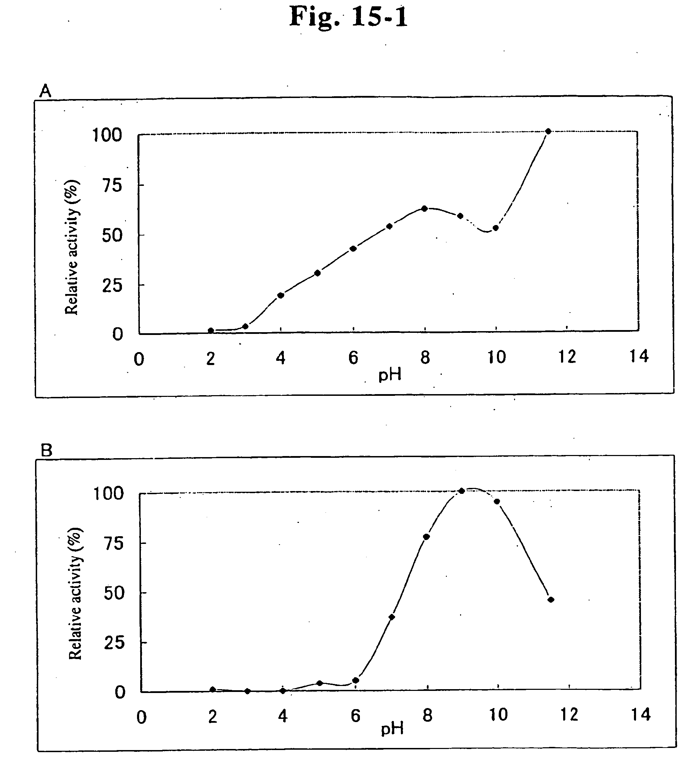

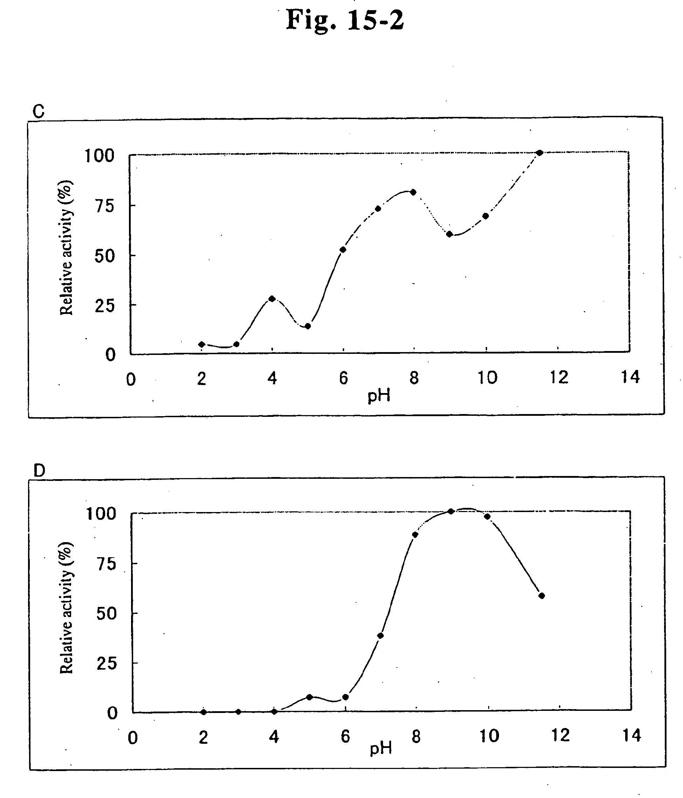

Fig. 15 shows the result of the pH stability of the neutral phenol oxidase III. Fig. 15 is shown by a relative remaining activity determined when the maximum activity is defined as 100. In addition, in Fig. 15, panels A and B show cases where 2,6-dimethoxyphenol was used as a substrate, and panels C and D being cases where para-phenylenediamine was used as a substrate. Further, in Fig. 15, panels A and C show the pH stability after 1-hour incubation, and panels B and D being the pH stability after 20-hour incubation.

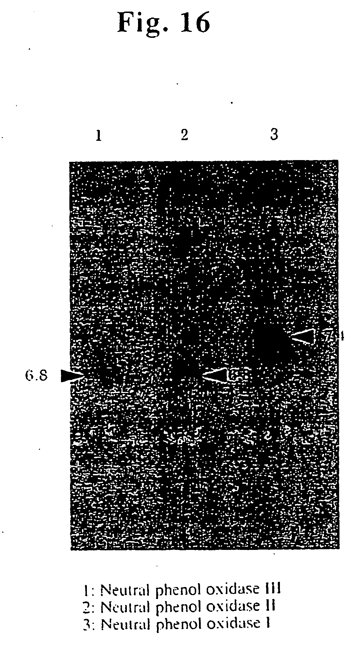

Fig. 16 shows the result of isoelectric focusing of the neutral phenol oxidase I, the neutral phenol oxidase II, and the neutral phenol oxidase III.

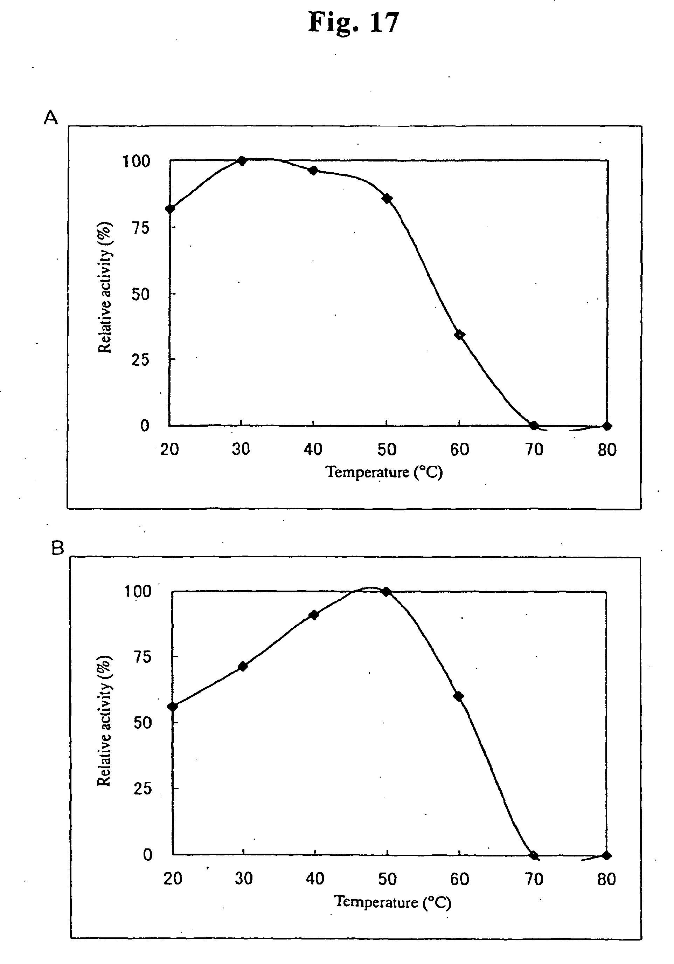

Fig. 17 shows the result of the optimum temperature of the neutral phenol oxidase I. Fig. 17 is shown by a relative activity determined when the maximum activity is defined as 100. In addition, in Fig. 17, panel A shows the case where 2,6-dimethoxyphenol was used as a substrate, and the panel B being the case where para-phenylenediamine was used as a substrate.

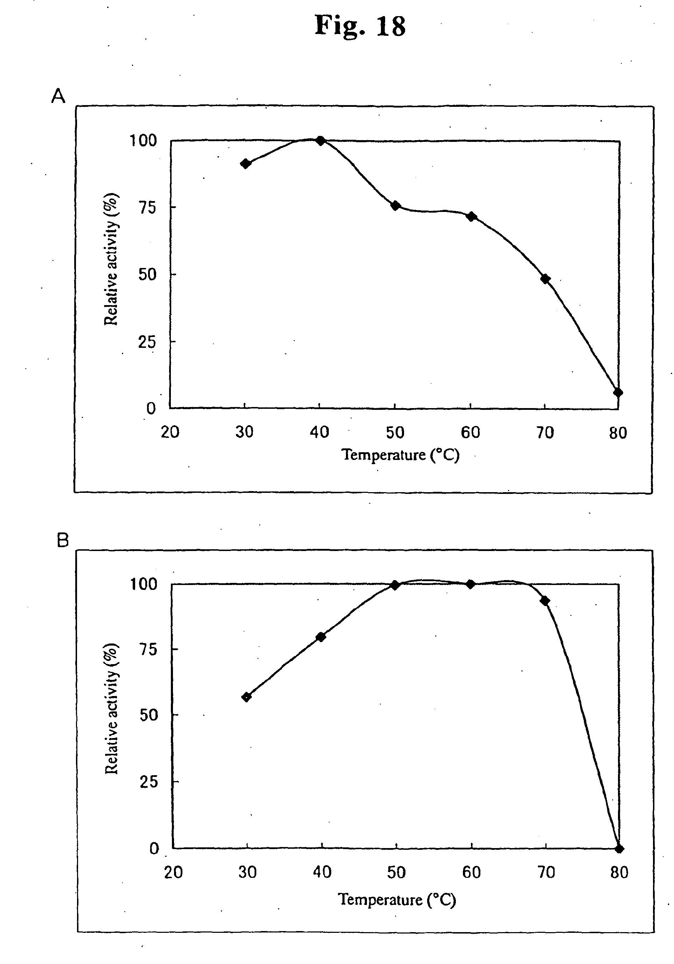

Fig. 18 shows the result of the optimum temperature of the neutral phenol oxidase II. Fig. 18 is shown by a relative activity determined when the maximum activity is defined as 100. In addition, in Fig. 18, panel A shows the case where 2,6-dimethoxyphenol was used as a substrate, and panel B being the case where para-phenylenediamine was used as a substrate.

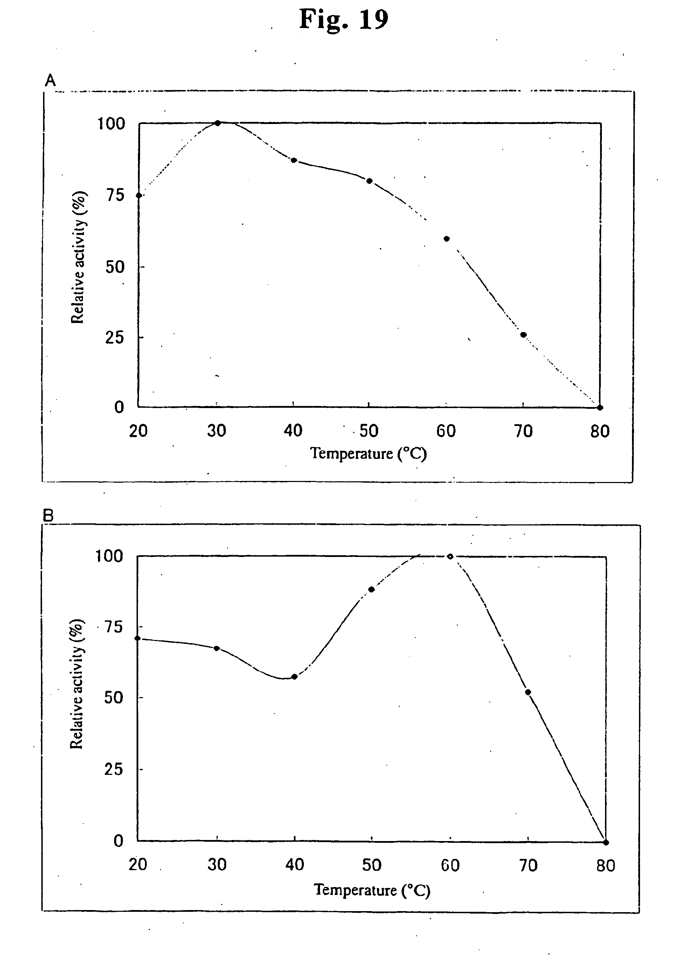

Fig. 19 shows the result of the optimum temperature of the neutral phenol oxidase III. Fig. 19 is shown by a relative activity determined when the maximum activity is defined as 100. In addition, in Fig. 19, panel A shows the case where 2,6-dimethoxyphenol was used as a substrate and panel B being the case where para-phenylenediamine was used as a substrate.

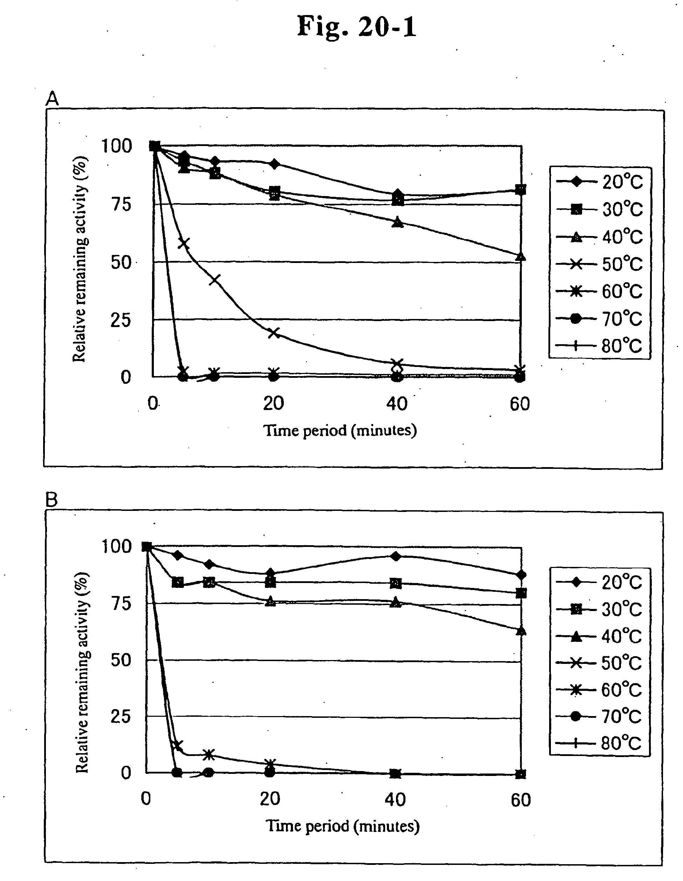

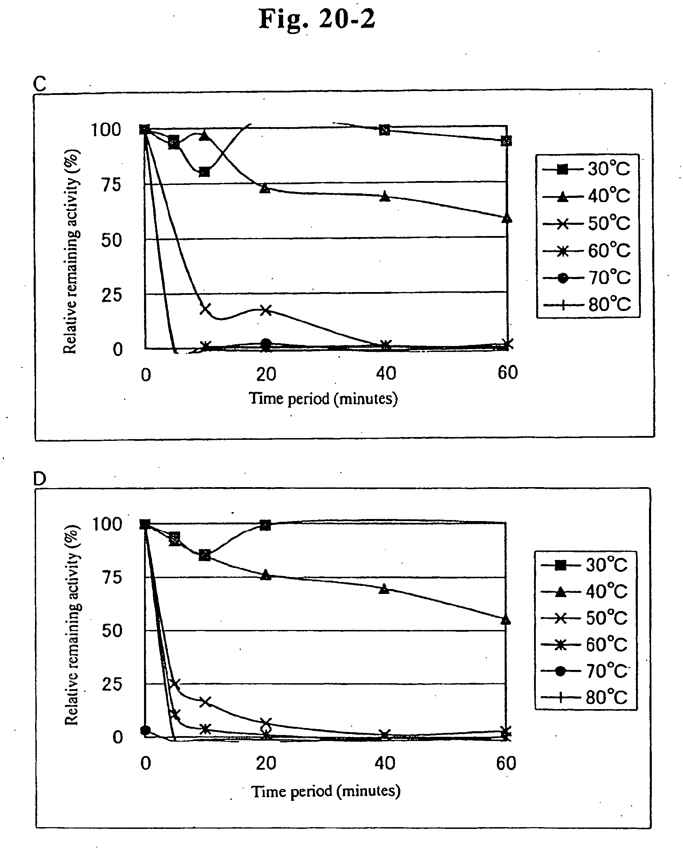

Fig. 20 shows the result of the thermal stability of the neutral phenol oxidase I. Fig. 20 is shown by a relative remaining activity determined when the maximum activity is defined as 100. In addition, in Fig. 20, panels A and B show thermal stability at pH 7.0, and panels C and D being thermal stability at pH 9.0. Further, panels A and C show cases where 2,6-dimethoxyphenol was used as a substrate, and panels B and D being cases where para-phenylenediamine was used as a substrate.

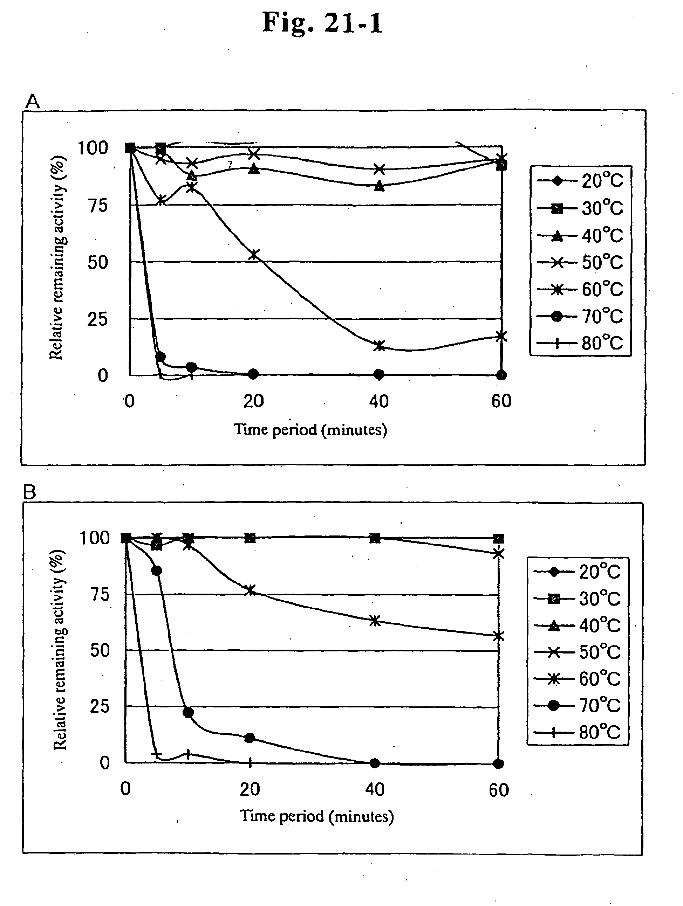

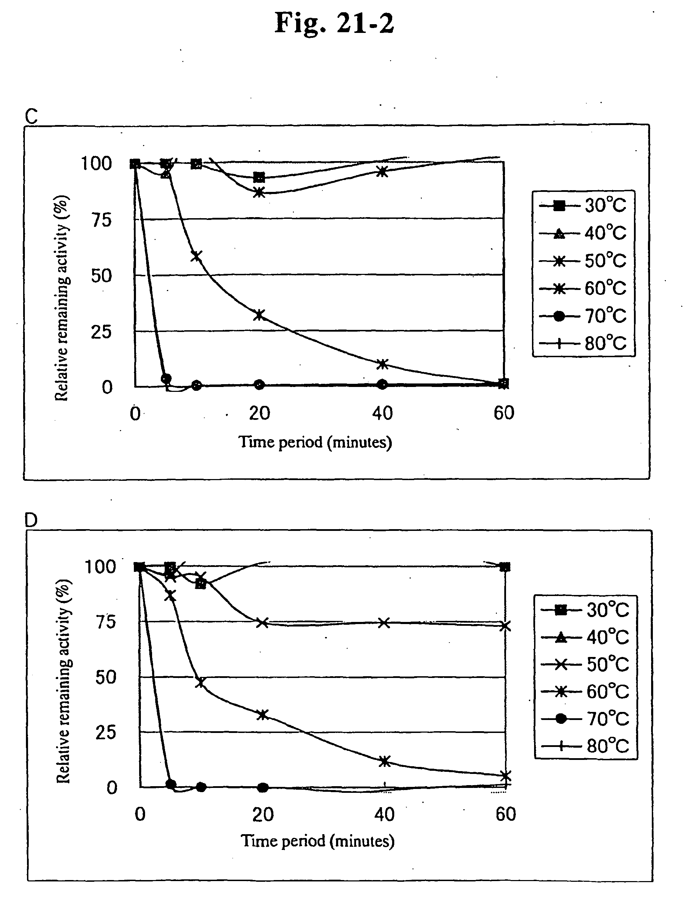

Fig. 21 shows the result of the thermal stability of the neutral phenol oxidase II. Fig. 21 is shown by a relative remaining activity determined when the maximum activity is defined as 100. In addition, in Fig. 21, panels A and B show thermal stability at pH 7.0, and panels C and D being thermal stability at pH 9.0. Further, in Fig. 21, panels A and C show cases where 2,6-dimethoxyphenol was used as a substrate, and panels B and D being cases where para-phenylenediamine was used as a substrate.

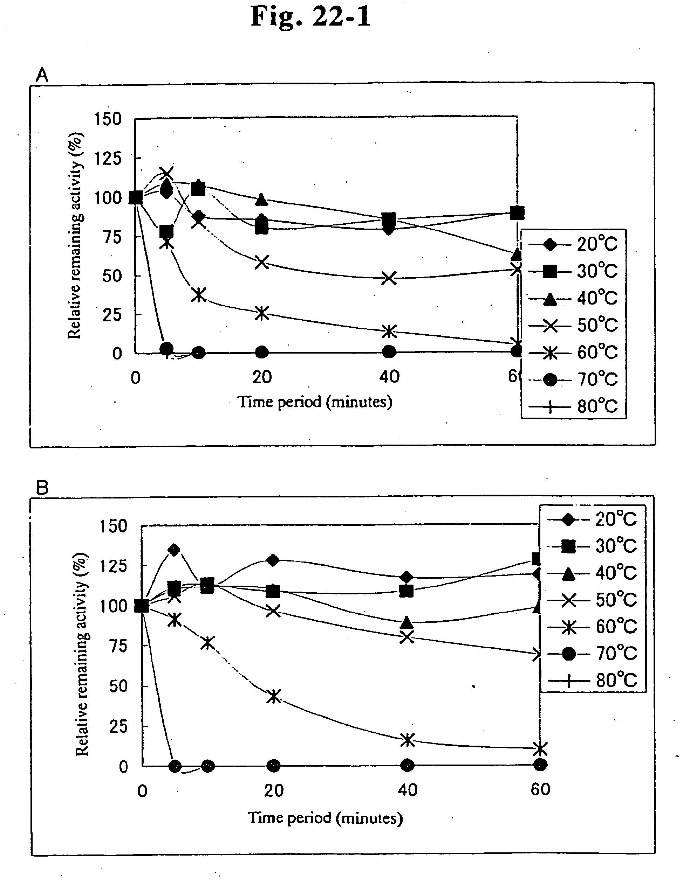

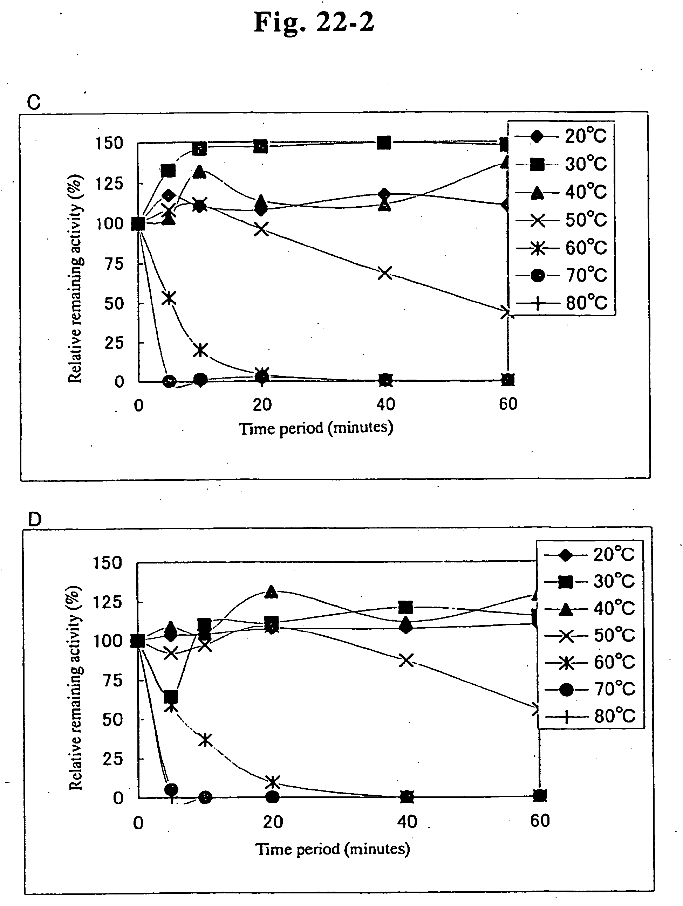

Fig. 22 shows the result of the thermal stability of the neutral phenol oxidase III. Fig. 22 is shown by a relative remaining activity determined when the enzymatic activity before incubation is defined as 100. In addition, in Fig. 22, panels A and B show thermal stability at pH 7.0, and panels C and D being thermal stability at pH 9.0. Further, in Fig. 22, panels A and C show cases where 2,6-dimethoxyphenol was used as a substrate, and panels B and D being cases where para-phenylenediamine was used as a substrate.

The neutral phenol oxidase of the present invention is a phenol oxidase having the following properties:
(1) optimum pH: 5.0 to 7.0;
(2) substrate specificity:
   i) catalyzing a coloring reaction by an oxidation of each of N,N-dimethyl-para-phenylenediamine, ortho-aminophenol, 2,6-dimethoxyphenol, 1,3-dihydroxynaphthol, and 4-hydroxyindole at pH around 6.5; and
   ii) catalyzing the oxidative polymerization reaction of alkali extract of lignin
(3) 28 kDa (calculated by SDS-PAGE),
(4) pH stability: maintaining a relative remaining activity of at least 70 % under incubation conditions at a pH of 8.0 to 9.0 for 20 hours at 30°C,
(5) optimum temperature: 30 to 50°C,
(6) thermal stability:
   I) maintaining a relative remaining activity of at least 80 % under incubation conditions at 0 to 30°C for 1 hour at a pH of 7.0, as compared with an activity before incubation, and
   II) maintaining a relative remaining activity of at least 90 % under incubation conditions at 0 to 30°C for 1 hour at a pH of 9.0, as compared with an activity before incubation, and
(7) isoelectric point: 7.4.

In the following claims neutral phenol oxidase, which is according to the present invention, is referred to as "neutral phenol oxidase I".

The neutral phenol oxidase of the present invention has excellent properties that the changes in optimum pH depending on kinds of substrates are little. Therefore, the neutral phenol oxidase of the present invention exhibits excellent effects such that the reactions for many substrates can be carried out under substantial the same reaction pH condition. In addition, the neutral phenol oxidase of the present invention exhibits excellent effects such that the simplification of the step of changing a reaction pH condition even when the plural number of compounds are used as substrates. Therefore, by using the neutral phenol oxidase of the present invention in, for example, dyeing fibers or hair, the process of changing a reaction pH condition can be simplified.

In the following two further neutral phenol oxidases, which are not according to the present invention, are described .In addition to the above-mentioned characteristics (1) and (2), they further have the following properties (3') to (7'):
(3') 35 kDa (calculated by SDS-PAGE),
(4') pH stability: maintaining a relative remaining activity of at least 75 % under incubation conditions at a pH of 7.0 to 10.0 for 20 hours at 30°C,
(5') optimum temperature: 30 to 60°C,
(6') thermal stability:
   I') maintaining a relative remaining activity of at least 90 % under incubation conditions at 0 to 50°C for 1 hour at a pH of 7.0 , as compared with an activity before incubation, and
   II') maintaining a relative remaining activity of at least 70 % under incubation conditions at 0 to 50°C for 1 hour at a pH of 9.0 , as compared with an activity before incubation, and
(7') isoelectric point: about 6.8;
   or the following properties (3") to (7"):
   (3") 45 kDa (calculated by SDS-PAGE),
   (4") pH stability: maintaining a relative remaining activity of at least 70 % under incubation conditions at 30°C for 20 hours at a pH of 8.0 to 10.0,
   (5") optimum temperature: 30 to 60°C
   (6") thermal stability:
      I") maintaining a relative remaining activity of at least 80 % under incubation conditions at 0 to 30°C for 1 hour at a pH of 7.0 , as compared with an activity before incubation, and
      II") maintaining a relative remaining activity of at least 90 % under incubation conditions at 0 to 40°C for 1 hour at a pH of 9.0 , as compared with an activity before incubation, and
   (7") isoelectric point: about 6.8. In addition, in the present specification, a neutral phenol oxidase having the above properties (1), (2), and (3) to (7) refers to "neutral phenol oxidase I", a neutral phenol oxidase having the above properties (1), (2), and (3') to (7') refers to "neutral phenol oxidase II", and a neutral phenol oxidase having the above-mentioned characteristics (1), (2'), and (3") to (7") refers to "neutral phenol oxidase III".

In the present specification, the "phenol oxidase" refers to an oxidation enzyme oxidizing catalytically and directly substrates such as phenolic compounds, aminophenol compounds, diamine compounds, and heterocyclic compounds in the presence of oxygen. More specifically, the above-mentioned "phenol oxidase" refers to a phenol oxidase oxidizing directly para-phenylenediamine, 2,6-dimethoxyphenol, catechol, ABTS, and syringaldazine but not oxidizing directly L-tyrosine.

Further, in the present specification, the "neutral phenol oxidase" refers to a phenol oxidase having an optimum pH of 5.0 to 7.0, preferably 5.5 to 7.0.

The phenolic compounds include, for example, 2-methoxyphenol, 2,6-dimethoxyphenol, catechol, pyrogallol, gallic acid, propyl gallate, 1-naphthol, catechin, and the like. The aminophenol compounds include, for example, ortho-aminophenol, para-aminophenol, and the like. The diamine compounds include, for example, ortho-phenylenediamine, para-phenylenediamine, and the like. The heterocyclic compounds include, for example, 4-hydroxyindole, 5-hydroxyindole, and the like.

The enzymatic activity of the neutral phenol oxidases can be evaluated by measuring oxidation activity for a substrate, decolorizing activity for a dye, and the like.

The oxidation activity of the neutral phenol oxidases for a substrate can be determined by monitoring the direct oxidation reaction of the substrate by which oxygen molecules are reduced with a phenolic compound, an aminophenol compound, a diamine compound or the like as a hydrogen donor. The hydrogen donor includes 2,6-dimethoxyphenol, ortho-aminophenol, para-phenylenediamine, and the like. The oxidation activity is determined on the basis of, for example, changes in the absorbance of syringaldazine (530 nm), 2,6-dimethoxyphenol, para-phenylenediamine (470 mm), or ortho-aminophenol (420 nm).

Concretely, 0.1 ml of a 0.05 M aqueous solution of 2,6-dimethoxyphenol is added to 0.896 ml of a 0.2 M sodium phosphate buffer (pH 7.0), to thereby obtain a substrate solution. The resulting substrate solution and 0.004 ml of an enzyme solution of the neutral phenol oxidase I or the neutral phenol oxidase II are mixed, to initiate the oxidation reaction of 2,6-dimethoxyphenol. Then, the oxidation reaction of 2,6-dimethoxyphenol is monitored by measuring a change in the absorbance at 470 nm, to thereby determine the oxidation activity. Regarding the oxidation activity, the enzyme amount for increasing the absorbance by 1 per one minute at a given wavelength depending on each of the substrates is defined as 1 unit (U).

Further, the decolorizing activity for a dye by the neutral phenol oxidases is determined by measuring a decrease in the maximum value (λmax) of absorption spectrum of the dye in the presence of the neutral phenol oxidase I or the neutral phenol oxidase II. The dye includes, for example, Acid Violet 17, Evans Blue, Acid Blue 80, and the like. The decolorizing activity for a dye is determined on the basis of, for example, a decrease in the absorbance of Acid Violet 17 (550 nm) or Evans Blue or Acid Blue 80 (630 nm).

Specifically, when Acid Violet 17 is used as a dye, the decolorizing activity is determined by measuring a decrease in the absorbance at 550 nm of a mixture, wherein the mixture is obtained by adding 0.1 ml of a 1.0 M sodium phosphate buffer (pH 6.5) to 0.8 ml of an enzyme solution containing the neutral phenol oxidase I or the neutral phenol oxidase II and mixing the resultant with 0.1 milliliter of a 0.2 mg/ml aqueous solution of Acid Violet 17. Here, regarding the enzymatic activity for the above decolorizing reaction, an amount for increasing the absorbance by 1 per one minute at given wavelength for each of dyes is defined as 1 unit (U).

Each of the neutral phenol oxidase I and the neutral phenol oxidase II has a molecular weight of about 72 kDa when calculated by a gel filtration method. In addition, the neutral phenol oxidase I has a molecular weight of 28 kDa and the neutral phenol oxidase II has a molecular weight of 35 kDa, when calculated by SDS-PAGE. Regarding the neutral phenol oxidase III a molecular weight of about 45 kDa calculated by a gel filtration method corresponds to a molecular weight of 45 kDa when calculated by SDS-PAGE. In the present specification, the molecular weight means a value calculated by a gel filtration method using gel filtration chromatography or by SDS-PAGE. The molecular weight is calculated by, for example, comparing a calibration curve prepared using molecular weight markers each having a known molecular weight, with a measurement value obtained by subjecting a target protein to gel filtration chromatography to thereby measure the volume of eluate at a constant flow velocity, or with a measurement value obtained by subjecting a target protein to SDS-PAGE to thereby measure the mobility thereof, and then comparing the resulting measurement value therewith, to determine the molecular weight of the target protein.

Each of the neutral phenol oxidase I, the neutral phenol oxidase II, and the neutral phenol oxidase III has high enzymatic activity at a pH of 5.0 to 7.0, and exhibits a relative activity of at least 50 % at the pH, compared to the maximum activity.

The neutral phenol oxidases exhibit an excellent enzymatic activity in the above-mentioned pH. Therefore, the neutral phenol oxidases excellent effects such that a water-based reaction solution can be used and particular pH adjustment is not required, in order to carry out efficiently an enzymatic reaction. In addition, for the neutral phenol oxidases a water-based reaction solution can be used. Specifically, a reaction can be efficiently carried out, for example, in an aqueous solution containing a substrate and an enzyme without particular pH adjustment. Therefore, the neutral phenol oxidases exhibit excellent effects such that effects on the human body and the environment by a reaction solution are little. Further, the neutral phenol oxidases do not show large changes in optimum pH for various substrates, especially for phenolic compounds, aminophenol compounds, and diamine compounds, and can efficiently oxidize a substrate at a pH around neutral region.

Specifically, the neutral phenol oxidases have the optimum pH in around neutral region, specifically pH 5.0 to 7.0, preferably pH 5.5 to 7.0, in an oxidation reaction where a phenolic compound, an aminophenol compound, or a diamine compound is used as a substrate.

More specifically, when 2,6-dimethoxyphenol is used as a substrate, the optimum pH of the neutral phenol oxidase I is a pH of about 5.0 to about 7.0, and more optimal range of the optimum pH is a pH of about 5.5 to about 7.0. When ortho-aminophenol is used as a substrate, the optimum pH of the neutral phenol oxidase I is a pH of about 5.5 to about 7.0, and more optimal range of the optimum pH is a pH of about 5.5 to about 6.5. When para-phenylenediamine is used as a substrate, the optimum pH of the neutral phenol oxidase I is a pH of about 5.5 to about 7.0, more optimal range of the optimum pH being a pH of about 5.5 to about 6.5, further more optimal range of the optimum pH being a pH of about 5.5 to about 6.0. When syringaldazine is used as a substrate, the optimum pH of the neutral phenol oxidase I is a pH of about 5.5 to about 7.0, more optimal range of the optimum pH being a pH of about 5.5 to about 6.5, particularly about 6.5. On the other hand, when 2,6-dimethoxyphenol is used as a substrate, the optimum pH of the neutral phenol oxidase II is a pH of about 5.0 to about 7.0, more optimal range of the optimum pH being a pH of about 5.5 to about 7.0, further more optimal range of the optimum pH being a pH of about 5.5 to about 6.0. When ortho-aminophenol is used as a substrate, the optimum pH of the neutral phenol oxidase II is a pH of about 5.5 to about 7.0, more optimal range of the optimum pH being a pH of about 5.5 to about 6.0. When para-phenylenediamine is used as a substrate, the optimum pH of the neutral phenol oxidase II is a pH of about 5.5 to about 7.0, more optimal range of the optimum pH being a pH of about 5.5 to about 6.5, further more optimal range of the optimum pH being a pH of about 5.5 to about 6.0. When syringaldazine is used as a substrate, the optimum pH of the neutral phenol oxidase II is a pH of about 5.5 to about 7.0, more optimal range of the optimum pH being a pH of about 5.5 to about 6.5. In addition, when 2,6-dimethoxyphenol is used as a substrate, the optimum pH of the neutral phenol oxidase III is a pH of about 5.0 to about 7.0, more optimal range of the optimum pH being a pH of about 5.5. When ortho-aminophenol is used as a substrate, the optimum pH of the neutral phenol oxidase III is a pH of about 5.0 to 7.0. When para-phenylenediamine is used as a substrate, the optimum pH of the neutral phenol oxidase III is a pH of about 5.0 to 7.0, more optimal range of the optimum pH being a pH of about 5.0 to 6.0. When syringaldazine is used as a substrate, the optimum pH of the neutral phenol oxidase III is a pH of about 5.0 to 7.0, more optimal range of the optimum pH being a pH of about 6.0.

Each of the neutral phenol oxidase I, the neutral phenol oxidase II, and the neutral phenol oxidase III shows the following substrate specificity:
i) catalyzing the coloring reaction by oxidation of each of N,N-dimethyl-para-phenylenediamine, ortho-aminophenol, 2,6-dimethoxyphenol, 1,3-dihydroxynaphthol, and 4-hydroxyindole at a pH of around 6.5; and
ii) catalyzing the oxidative polymerization reaction of alkali extract of lignin. Specifically, each of the neutral phenol oxidase I and the neutral phenol oxidase II shows the following substrate specificity under neutral conditions (pH 6.5):
   1) catalyzing the oxidation reaction of diamine compounds such as ortho-phenylenediamine, para-phenylenediamine, N,N-dimethyl-para-phenylenediamine, tolylene-3,4-diamine, para-aminodiphenylamine, 2-chloro-1,4-phenylenediamine, 2,5-diaminotoluene, and the like, and particularly, catalyzing strongly the oxidation reaction of N,N-dimethyl-para-phenylenediamine;
   2) catalyzing the oxidation reaction of aminophenol compounds such as ortho-aminophenol, para-aminophenol, 5-amino-2-methylphenol, and the like, and particularly, catalyzing strongly the oxidation reaction of ortho-aminophenol;
   3) catalyzing the oxidation reaction of phenolic compounds such as 2-methoxyphenol, 2,6-dimethoxyphenol, catechol, protocatechuic acid, and the like, and particularly, catalyzing strongly the oxidation reaction of 2,6-dimethoxyphenol;
   4) catalyzing the oxidation reaction of naphthol compounds such as 1-naphthol, 1,3-dihydroxynaphthol, 1,5-dihydroxynaphthol, and the like, and particularly, catalyzing strongly the oxidation reaction of 1,3-dihydroxynaphthalene;
   5) catalyzing the oxidation reaction of indole compounds such as 4-hydroxyindole, 5-hydroxyindole, and the like;
   6) catalyzing the oxidation reaction of compounds (extracts) such as catechin (green tea extract), alkali extract of lignin (derived from rice plant), alkali extract of lignin (derived from a conifer) and the like. Regarding the above-mentioned substrate specificity, the neutral phenol oxidase I of the present invention has properties such that the neutral phenol oxidase I exhibits a strong catalytic activity under neutral conditions (pH 6.5) and under alkaline conditions (pH 9.0), when alkali extract of lignin (derived from a conifer) is used as a substrate, and that specific activity thereof under neutral conditions (pH 6.5) is higher than the specific activity under alkaline conditions (pH 9.0) when compounds other substances such as diamine compounds, aminophenol compounds, phenolic compounds, naphthol compounds, indole compounds, catechin (green tea extract) or alkali extract of lignin (derived from rice plant) are used as substrates. On the other hand, the neutral phenol oxidase II has properties such that specific activity thereof under neutral conditions (pH 6.5) is higher than the specific activity under alkaline conditions (pH 9.0) for various substrates, except for the case where alkali extract of lignin (a conifer) is used as a substrate. Further, the neutral phenol oxidase III has properties such that the neutral phenol oxidase III exhibits higher specific activity under neutral conditions (pH 6.5) than under alkaline conditions (pH 9.0) for various substrates.

In addition, each of the neutral phenol oxidase I, the neutral phenol oxidase II, and the neutral phenol oxidase III oxidizes phenol oxidase mediators, ABTS and NNS, as a substrate.

The neutral phenol oxidases have excellent properties that the neutral phenol oxidases show high pH stability in a wide pH range from neutral to weakly alkaline pH. Specifically, the neutral phenol oxidase I maintains a relative remaining activity of at least 25 % under incubation conditions at a pH of 7.0 to 10.0 for 20 hours at 30°C, as compared with the activity before incubation, preferably at least 70 % under incubation conditions at a pH of 8.0 to 9.0 for 20 hours at 30°C, as compared with the activity before incubation. On the other hand, the neutral phenol oxidase II maintains a relative remaining activity of at least 50 % under incubation conditions at a pH of 4.0 to 10.0 for 20 hours at 30°C, as compared with the activity before incubation, preferably at least 75 % under incubation conditions at a pH of 7.0 to 10.0 for 20 hours at 30°C, as compared with the activity before incubation. The neutral phenol oxidase III maintains a relative remaining activity of at least 30 % under incubation conditions at a pH of 7.0 to 11.5 for 20 hours at 30°C, as compared with the maximum activity, preferably at least 70 % under incubation conditions at a pH of 8.0 to 10.0 for 20 hours at 30°C, as compared with the maximum activity.

Each of the neutral phenol oxidase I, the neutral phenol oxidase II, and the neutral phenol oxidase III exhibits high enzymatic activity at 30 to 50°C, 30 to 60°C, and 30 to 60°C, respectively. Namely, since each of the neutral phenol oxidase I, the neutral phenol oxidase II, and the neutral phenol oxidase III has high enzymatic activity at temperatures within the above-mentioned range, each of the neutral phenol oxidase I, the neutral phenol oxidase II, and the neutral phenol oxidase III exhibits high enzymatic activity at usual living temperatures (e.g., room temperature, water temperature, body temperature, atmospheric temperature, and the like) without adjusting particular temperature conditions. Therefore, according to the neutral phenol oxidases, dyeing, waste water treatment, synthesis of high polymer compounds and the like can be easily carried out. Specifically, the neutral phenol oxidase I of the present invention exhibits the following thermal stability:
I) maintaining a relative remaining activity of at least 50 % under incubation conditions at 0 to 40°C for 1 hour at a pH of 7.0, as compared with the activity before incubation, preferably at least 80 % under incubation conditions at 0 to 30°C for 1 hour at a pH of 7.0, as compared with the activity before incubation; and
II) maintaining a relative remaining activity of at least 50 % under incubation conditions at 0° to 40°C for 1 hour at a pH of 9.0, as compared with the activity before incubation, preferably at least 90 % under incubation conditions at 0 to 30°C for 1 hour at a pH of 9.0, as compared with the activity before incubation. On the other hand, the neutral phenol oxidase II exhibits the following thermal stability:
   I') maintaining a relative remaining activity of at least 15 % under incubation conditions at 0° to 60°C for 1 hour at a pH of 7.0, as compared with the activity before incubation, preferably at least 90 % under incubation conditions at 0° to 50°C for 1 hour at a pH of 7.0, as compared with the activity before incubation; and
   II') maintaining a relative remaining activity of at least 70 % under incubation conditions at 0° to 50°C for 1 hour at a pH of 9.0, as compared with the activity before incubation, preferably at least 90 % under incubation conditions at 0° to 40°C for 1 hour at a pH of 9.0, as compared with the activity before incubation.

In addition, the neutral phenol oxidase III exhibits the following thermal stability:
I") maintaining a relative remaining activity of at least 50 % under incubation conditions at 0° to 50°C for 1 hour at a pH of 7.0, as compared with the activity before incubation, preferably at least 80 % under incubation conditions at 0° to 30°C for 1 hour at pH 7.0, as compared with the activity before incubation; and
II") maintaining a relative remaining activity of at least 40 % under incubation conditions at 0° to 50°C for 1 hour at a pH of 9.0, as compared with the activity before incubation, preferably at least 90 % under incubation conditions at 0° to 40°C for 1 hour at a pH of 9.0, as compared with the activity before incubation.

The neutral phenol oxidase I, the neutral phenol oxidase II, and the neutral phenol oxidase III are produced by a basidiomycete belonging to the genus *Flammulina.* Specifically, the basidiomycete belonging to the genus *Flammulina* includes a basidiomycete belonging to *Flammulina velutipes*, more specifically *Flammulina velutipes* IFO 30601 strain. Since the neutral phenol oxidase I, the neutral phenol oxidase II, and the neutral phenol oxidase II can be obtained from *Flammulina velutipes*, the supply source of them is easily available and is easy to handle.

The neutral phenol oxidase I, the neutral phenol oxidase II, and the neutral phenol oxidase III can be produced by culturing a basidiomycete belonging to the genus *Flammulina.* The present invention encompasses such a method for producing the neutral phenol oxidases.

One of the features of the production method of the present invention resides in that a basidiomycete belonging to the genus *Flammulina* is cultured at a pH of 6.0 to 12.0.

Since in the production method of the present invention, a basidiomycete is cultured at a pH of 6.0 to 12.0, there can be exhibited excellent effects such that the neutral phenol oxidase I, the neutral phenol oxidase II, and the neutral phenol oxidase III can be induced and that the neutral phenol oxidases can be obtained surprisingly efficiently in high yield.

Specifically, the production method of the present invention includes a method comprising the steps of:
1) culturing a basidiomycete belonging to the genus *Flammulina* at a pH of 6.0 to 12.0; and
2) separating a neutral phenol oxidase from a culture supernatant of a culture obtained in the step 1) or an extract obtained from the culture.

The pH of the culture medium may a pH suitable to sufficiently grow a basidiomycete belonging to the genus *Flammulina* and to produce the neutral phenol oxidase I, the neutral phenol oxidase II, and the neutral phenol oxidase III. It is desired that the pH of the culture medium is adjusted to a pH of 6.0 to 12.0, preferably, a pH of 7.0 to 11.0, more preferably a pH of 8.0 to 10.0, and thereafter the medium is sterilized to be use.

In the present invention, from the viewpoint of increasing the yield of the neutral phenol oxidase, there can be carried out the steps of:
i) a culturing step for increasing the mycelium of the basidiomycete, and
ii) a culturing step for inducing the expression of an enzyme, to increase the yield of the neutral phenol oxidase, in the mycelium obtained in the culturing step i), in the step 1) of the production method of the present invention. In this case, culture may be carried out in the culturing steps i) at a pH suitable for the growth of mycelium, specifically, for example, at a pH of 2.0 to 13.0, preferably a pH of 4.0 to 10.0, particularly preferably a pH of 5.2, and then carried out in the culturing step ii) at a pH suitable for the production of the neutral phenol oxidase I or the neutral phenol oxidase II, concretely at a pH of 6.0 to 12.0, preferably a pH of 7.0 to 11.0, more preferably a pH of 8.0 to 10.0. In addition, in the culturing step i), a procedure for scale-up of the amount of culture of mycelium may be appropriately carried out.

In the step 1) described above, the basidiomycete belonging to the genus *Flammulina* can be cultured in any of liquid culture or solid culture.

In the culture of the basidiomycete belonging to the genus *Flammulina,* as long as the basidiomycete can grow, any of usual liquid medium or solid medium may be used.

When the basidiomycete belonging to the genus *Flammulina* is cultured in a liquid medium, aeration culture or shaking culture is desirable.

A carbon source may be any carbon source, as long as the carbon can be assimilated by the basidiomycete belonging to the genus *Flammulina.* The carbon source includes saccharides such as glucose, sucrose, molasses, and starch; bran; orange pulp; and the like. The carbon source can be used alone or in combination of two or more of thereof. In addition, a nitrogen source includes, for example, inorganic nitrogen such as potassium nitrate and ammonium sulfate as well as organic nitrogen sources such as bean curd refuse, peptone, tryptone, casamino acid, yeast extract, malt extract, defatted soybean powder, corn steep liquor, and urea. These nitrogen sources can be used alone or in combination of two or more of them. In addition, a culture medium for producing the neutral phenol oxidase I and the neutral phenol oxidase II may be supplemented, if necessary, with phosphate, magnesium sulfate, magnesium carbonate, sodium carbonate, inorganic salts such as potassium, calcium, iron, copper, zinc, manganese and cobalt; vitamins; and the like. The concentrations of the carbon source, the nitrogen source, and the like in the culture medium may be a concentration at which the basidiomycete belonging to the genus *Flammulina* can be grow sufficiently, to thereby produce the neutral phenol oxidase I, the neutral phenol oxidase II, and the neutral phenol oxidase III. It is desired that the concentration of the carbon source is 0.1 to 20 wt%, preferably 1 to 10 wt%. It is desired that the concentration of the nitrogen source is 0.1 to 10 wt%, preferably 1 to 5 wt%.

The culture temperature may be a temperature at which filamentous fungi can grow, and is practically 10 to 40°C, preferably 20 to 35°C.

The culture time is varied depending on various culture conditions, and in the case of aeration culture, it is desired that the culture time is 2 to 10 days. The culture time can be set by using as an index the timing when the value of the enzymatic activity of a culture solution becomes a maximum value.

A concrete example of the above-mentioned step 1) includes, for example, a process comprising the steps of:
- inoculating a basidiomycete belonging to the genus *Flammulina* into a liquid culture medium 1 {composition: 2.4 wt% potato dextrose broth (manufactured by Difco), and balance water (pH 5.2); sterilized at 121°C for 15 minutes}, carrying out reciprocal shaking cultivation (150 strokes/minute) for 7 days at 25°C, adding all the thus obtained culture solution to 500 ml of a liquid culture medium 1 contained in a 2-liter Erlenmeyer flask, and then carrying out reciprocal shaking cultivation (100 strokes/minute) for 3 weeks at 25°C (this step corresponds to the above-mentioned culture step i) described above), and
- standing the culture solution to precipitate the resulting pellet-like mycelium, removing a supernatant of the culture solution, adding 500 ml of a liquid culture medium 2 {composition: 1.0 wt% glucose, 0.1 wt% yeast extract, 0.14 wt% (NH₄)₂SO₄, 0.36 wt% K₂HPO₄, 0.02 wt% MgSO₄.7H₂O, 0.10 wt% mineral mixture (composition: 1.0 wt% CuSO₄•5H₂O, 1.0 wt% ZnCl₂, 0.7 wt% FeGl₃•6H₂O, 0.5 wt% CoSO₄•7H₂O, 0.5 wt% MnCl₂•4H₂O), pH 9.2; sterilized at 121°C for 15 minutes} to the residual mycelium, and further culturing for 3 days at 25°C (this step corresponds to the culture step ii) described above), and the like.
   Thereafter, the step of separating a neutral phenol oxidase from a culture supernatant of a culture obtained in the step 1) or an extract obtained from the culture {the step 2)}.
   The neutral phenol oxidase is produced in secretion outside of the fungus bodies and accumulated in the culture solution. Therefore, in the step 2), a culture supernatant containing the neutral phenol oxidase I or the neutral phenol oxidase II can be obtained by removing insoluble matter such as fungus bodies from the culture obtained in the step 1), by centrifugation or filtration through a filter paper or filter cloth.
   In addition, in the step 2), an extract containing the neutral phenol oxidase I, the neutral phenol oxidase II, and the neutral phenol oxidase III may also be obtained by carrying out extraction from a culture bed or a waste culture bed after cultivation of the basidiomycete belonging to the genus *Flammulina.*
   The neutral phenol oxidase I, the neutral phenol oxidase II, and the neutral phenol oxidase III can be separated and purified from the resulting culture supernatant or extract by using the purification technique of protein generally used, such as decolorization, concentration, salting out (e.g., ammonium sulfate fractionation), dialysis, various chromatography techniques, and gel filtration chromatography.
   For example, the fractions containing any of the purified neutral phenol oxidase I, the purified neutral phenol oxidase II, and the purified neutral phenol oxidase III having high specific activity can be obtained by freeze-drying and re-thawing the resulting culture supernatant or extract, filtering the resulting products to remove insoluble substances, and then successively subjecting the resultant to ion-exchange chromatography, gel filtration chromatography, and the like.
   Concretely, the neutral phenol oxidase I or the neutral phenol oxidase II can be obtained by, for example,
- adjusting the pH of the resulting culture solution to 7.5, adding 5 g (dry weight) of DEAE-cellulose (manufactured by Sigma) carrier per 1 L of the culture solution after pH adjustment, thereby making them to adsorb proteins,
- eluting the proteins adsorbed to the carrier with a 3-fold volume of 0.1 M sodium phosphate buffer (pH 6.5) containing 1 M sodium chloride, and then freeze-drying the protein solution,
- dissolving the resulting lyophilized product in 0.05 M Tris-HCl buffer (pH 8.0),
- applying the resulting solution to Q-Sepharose column (trade name, 2.6 × 10 cm; manufactured by Pharmacia) equilibrated with 0.05 M Tris-HCl buffer (pH 8.0), eluting the neutral phenol oxidase I and the neutral phenol oxidase II separately, by means of gradient elution with 0 to 1 M sodium chloride, and then freeze-drying them separately,
- dissolving the resulting lyophilized product in 0.05 M Tris-HCl buffer (pH 8.0) containing 0.1 M sodium chloride, and
- applying the resulting product to Sephacryl S-100 HR column (trade name, 1.6 × 60 cm; manufactured by Pharmacia) equilibrated with 0.05 M Tris-HCl buffer (pH 8.0) containing 0.1 M sodium chloride, and then carrying out elution with 0.05 M Tris-HCl buffer (pH 8.0) containing 0.1 M sodium chloride.
   On the other hand, the neutral phenol oxidase III can be obtained by, for example,
- concentrating the above-mentioned culture solution, and then dialyzing the resulting concentrate against 0.05 M sodium phosphate buffer (pH 7.0) overnight at 4°C,
- applying the resulting filtrate to DEAE-cellulose column (2.6 × 60 cm; manufactured by Sigma) equilibrated with 0.05 M sodium phosphate buffer (pH 7.0),
- eluting the neutral phenol oxidase III adsorbed to the column by means of gradient elution with a range of concentration from 0 to 1 M sodium chloride,
- dialyzing the resulting fraction against 0.05 M Tris-HCl buffer (pH 8.0) overnight at 4°C,
- applying the resulting solution to Q-Sepharose column (trade name, 2.6 × 10 cm; manufactured by Pharmacia) equilibrated with 0.05 M Tris-HCl buffer (pH 8.0), and then eluting the neutral phenol oxidase III by means of gradient elution with a range of concentration from 0.2 to 0.5 M sodium chloride,
- dialyzing the resulting fraction against 0.05 M sodium phosphate buffer (pH 7.0) for 3 hours at 4°C, and
- applying the resulting solution to DEAE-cellulose column (1.5 × 5.5 cm; manufactured by Sigma) equilibrated with 0.05 M sodium phosphate buffer (pH 7.0), and then eluting the neutral phenol oxidase III with 0.05 M sodium phosphate buffer (pH 7.0) containing 1 M sodium chloride.

In the present specification, the degree of purification of each of the neutral phenol oxidase I, the neutral phenol oxidase II, and the neutral phenol oxidase III is evaluated by polyacrylamide gel electrophoresis (native-PAGE), SDS-polyacrylamide electrophoresis (SDS-PAGE), and activity staining of gel after such electrophoresis.

In the present specification, each of native-PAGE and SDS-PAGE is carried out without heating samples for electrophoresis.

When activity staining using para-phenylenediamine as a substrate is carried out after native-PAGE, for example, staining may be carried out by is stirring a gel after native-PAGE with shaking for 20 minutes in 0.1 M sodium phosphate buffer (pH 7.0), and thereafter stirring the resulting gel with shaking for 5 minutes in 0.1 M sodium phosphate buffer (pH 7.0) containing 0.001 M para-phenylenediamine.

When staining of protein is carried out using Coomassie Brilliant Blue after native-PAGE, for example, staining may be carried out by immersing a gel after electrophoresis in the staining solution obtained by dissolving 0.5 g of Coomassie Brilliant Blue R250 in a mixed solution of 500 ml of methanol and 100 ml of acetic acid (total 600 ml), and adjusting the volume of the resulting solution to 1,000 ml with distilled ion-exchange water. Thereafter, decolorization is carried out using a decoloring solution prepared by adjusting a mixed solution of 250 ml of methanol and 70 ml of acetic acid (total 320 ml) so as to give a total volume of 1000 ml by using distilled ion-exchange water.

When activity staining is carried out with para-phenylenediamine after SDS-PAGE, for example, staining may be carried out by stirring a gel after SDS-PAGE with shaking for 60 minutes in 0.1 M sodium phosphate buffer (pH 7.0) containing 2.5 w/v% Triton X-100, and then stirring the resulting gel with shaking for 5 minutes in 0.1 M sodium phosphate buffer (pH 7.0) containing 0.001 M para-phenylenediamine.

When staining of protein is carried out using Coomassie Brilliant Blue after SDS-PAGE, for example, staining may be carried out by immersing a gel after electrophoresis in the staining solution obtained by dissolving 0.5 g of Coomassie Brilliant Blue R 250 in a mixed solution of 500 ml of methanol and 100 ml of acetic acid (total 600 milliliters), and then adjusting the volume of the resulting solution to 1000 ml with distilled ion-exchange water. Thereafter, decolorization is carried out using a decoloring solution prepared by adjusting a mixed solution of 250 ml of methanol and 70 ml of acetic acid (total 320 ml) so as to give a total volume of 1000 ml by using distilled ion-exchange water.

Since each of the neutral phenol oxidase I, the neutral phenol oxidase II, and the neutral phenol oxidase III shows a substantially uniform degree of purification in purity evaluation carried out by means of Coomassie Brilliant Blue staining after SDS-PAGE, the present specification can provide an antibody against the neutral phenol oxidase.

The antibody may be either a polyclonal antibody or a monoclonal antibody, as long as the antibody has the ability to bind specifically to the neutral phenol oxidase I, the neutral phenol oxidase II, and the neutral phenol oxidase III.

The antibody can be easily produced by immunizing animals such as rabbits and mice with each of the neutral phenol oxidase I, the neutral phenol oxidase II and the neutral phenol oxidase III according to a method described in, for example, John E. Coligan ed., "Current Protocols in Immunology", John Wiley & Sons, Inc. (1992) and then carrying out purification by means of a conventional method.

In addition, the antibody may be a part of the monoclonal antibody, that is, an antibody fragment, as long as the antibody fragment binds to the neutral phenol oxidase. The antibody fragment includes Fab, F(ab')₂, Fab', Fv, and the like. Such an antibody fragment can be obtained by digesting a monoclonal antibody with, for example, peptidase and the like. For example, Fab fragment can be obtained by treating the monoclonal antibody with papain, and F(ab')₂ fragment can be obtained by treating the monoclonal antibody with pepsin.

The antibody can be evaluated by, for example, measuring the binding property and the affinity of the antibody to each of the neutral phenol oxidase I, the neutral phenol oxidase II, and the neutral phenol oxidase III , by means of ELISA, Ouchterlony method, immunoelectrophoresis, and the like.

The antibody can be used for, for example, affinity chromatography for purification of the neutral phenol oxidase, screening of the neutral phenol oxidase, and the like.

According to the neutral phenol oxidase, dyeing of keratin fibers, and the like can be carried out by using various dyes, pigments, and the like at the same time. Therefore, the present specification can provide a dyeing method and a dyeing composition.

In addition, according to the present specification, dyeing can be carried out by bringing a subject to be dyed into contact with a dye in the presence of the neutral phenol oxidases. According to the neutral phenol oxidase, there are exhibited excellent effects such that dyeing of fibers or hair and the like can be carried out under external environmental conditions without particular adjustment of temperature conditions or pH conditions while reducing effects on the human body, the environment, and the like.

The subject to be dyed to which the dyeing method of the present invention is applicable includes, for example, cotton, diacetate, flax, linen, lyocell, polyacryl, polyamide, polyester, ramie, rayon, tencel, triacetate, fur, fell, leather, fabrics made of silk or wool, yarns, fibers, garments, films, wood, hair, and the like.

The dye includes oxidized dyes described in manufacturing material specification of quasi-drug, indoline and indole compounds, and the like. The dyes can be used alone or in combination of two or more of thereof. In addition, coupling agents can be used. Further, direct dyes can also be used.

Indoline and indoline compounds include, concretely, indoline, 5,6-dihydroxyindoline, N-methyl-5,6-dihydroxyindoline, N-ethyl-5,6-dihydroxyindoline, N-butyl-5,6-dihydroxyindoline, 4-hydroxy-5-methoxyindoline, 6-hydroxy-7-methoxyindoline, 6,7-dihydroxyindoline, 4,5-dihydroxyindoline, 4-methoxy-6-hydroxyindoline, N-hexyl-5,6-dihydroxyindoline, 2-methyl-5,6-dihydroxyindoline, 3-methyl-5,6-dihydroxyindoline, 4-hydroxyindoline, 2,3-dimethyl-5,6-dihydroxyindoline, 2-methyl-5-ethyl-6-hydroxyindoline, 2-methyl-5-hydroxy-6-β-hydroxyethylindoline, 4-hydroxypropylindoline, 2-hydroxy-3-methoxyindoline, 6-hydroxy-5-methoxyindoline, 6-hydroxyindoline, 5-hydroxyindoline, 7-hydroxyindoline, 7-aminoindoline, 5-aminoindoline, 4-aminoindoline, 5,6-dihydroxyindoline carboxylic acid, 1-methyl-5,6-dihydroxyindoline, and salts thereof.

Indole compounds includes, concretely, 4-hydroxyindole, 5-hydroxyindole, 5,6-dihydroxyindole, 5,6-dihydroxyindole-2-carboxylic acid, 5,6-tri(t-butoxycarbonyloxy)indole, 5,6-di(t-butoxycarbonyloxy)indole, 5-t-butoxycarbonyloxy-6-hydroxyindole, 6-*t*-butoxycarbonyloxy-5-hydroxyindole, 5,6-di(ethoxycarbonyloxy)indole, 5,6-di(ethylcarbamoyloxy)indole, 1-pivaloyl-5-(pivaloyloxymethoxy)-6-pivaloyloxyindole, 1-pivaloyl-5-pivaloyloxymethoxy-6-hydroxyindole, 5,6-(oxycarbonylmethoxy)indole, and the like.

Oxidized dyes described in manufacturing material specification of quasi-drug include, concretely, 5-amino-*o*-cresol, *o*-aminophenol, *m*-aminophenol, *p*-aminophenol, 2,6-diaminopyridine, 5-(2-hydroxyethylamino)-2-methylphenol, N,N-bis(β-hydroxy)-*p-*phenylenediamine sulfate, *p*-nitro-*o*-phenylenediamine, *p*-nitro-2',4'-diaminoazobenzene sodium sulfate, toluene-2,5-diamine, 5-amino-*o*-cresol sulfate, *p*-aminophenol sulfate, *o-*chloro-*p*-phenylenediamine sulfate, 4,4'-diaminodiphenylamine sulfate,p-methylaminophenol sulfate, *p*-phenylenediamine sulfate, *m*-phenylenediamine sulfate, toluene-2,5-diamine sulfate, 2,4-diaminophenoxyethanol hydrochloride, toluene-2,5-diamine hydrochloride, *m*-phenylenediamine hydrochloride, 2,4-diaminophenol hydrochloride, 3,3'-iminodiphenol, *p*-phenylenediamine hydrochloride, N-phenyl-*p-*phenylenediamine hydrochloride, N-phenyl-*p*-phenylenediamine acetate, 1,5-dihydroxynaphthalene, tolylene-3,4-diamine,*p*-methylaminophenol, N,N'-bis(4-aminophenyl)-2,5-diamino-1,4-quinonediimine, *o*-aminophenol sulfate, 2,4-diaminophenol sulfate, m-aminophenol sulfate, and the like.

In the present invention, direct dyes such as 2-amino-4-nitrophenol, 2-amino-5-nitrophenol, 1-amino-4-methylaminoanthraquinone, nitro-*p*-phenylenediamine hydrochloride, 1,4-diaminoanthraquinone, nitro-*p*-phenylenediamine, picramic acid, sodium picramate, 2-amino-5-nitrophenol sulfate, resorcinol, nitro-*p*-phenylenediamine sulfate, *p*-nitro-*o*-phenylenediamine sulfate, *p*-nitro-*m*-phenylenediamine sulfate, and the like can also be used.

The contact of a subject to be dyed with a dye in the presence of each of the neutral phenol oxidase I, the neutral phenol oxidase II, and the neutral phenol oxidase III is carried out by, for example, mixing the dye and the neutral phenol oxidase I, the neutral phenol oxidase II, or the neutral phenol oxidase III when used, to contact the resulting mixture with the subject to be dyed, or contacting a mixture preserved under anaerobic conditions of the dye with the neutral phenol oxidase I, the neutral phenol oxidase II, and the neutral phenol oxidase III with the subject to be dyed in the atmosphere in use. The dyeing method can be carried out by using the neutral phenol oxidase as a dyeing composition.

The dyeing composition includes a composition comprising the neutral phenol oxidase and a dye, and a combination of a composition A comprising the neutral phenol oxidase and a composition B comprising a dye, and the like.

In the dyeing composition, reducing agents such as thiolactic acid, sodium sulfite, and N-acetyl-L-cysteine can be mixed, to the extent that enzymatic activity is not inhibited. In addition, in the dyeing composition, surfactants, oily ingredients, silicones, thickeners, solvents, water, chelating agents, fragrant materials, and the like can be appropriately mixed, in addition to the above-mentioned components, to the extent that the effects of the present invention are not prevented.

The neutral phenol oxidases exhibit excellent effects such that the neutral phenol oxidases show high stability and efficiently acts on various substrates, especially phenolic compounds, aminophenol compounds, and diamine compounds at pH around neutral region, without the optimum pH thereof being changed widely depending on the substrates. In addition, according to the method for producing the neutral phenol oxidases, there are excellent effects such that the neutral phenol oxidases can be obtained efficiently and easily in large quantity at low cost. Further, according to the antibody, there are exhibited excellent effects such that the neutral phenol oxidases can be collected and purified easily. Moreover, according to the dyeing method, there are exhibited excellent effects such that dyeing of fibers or hair can be dyed easily and efficiently with various dyes, concretely, with phenolic compounds, aminophenol compounds, diaminophenol compounds, or the like and that effects on the environment, the human body, and the like are reduced. Further, the dyeing composition exhibits excellent effects such that handling is easy and that fibers or hair can be dyed easily and efficiently. Furthermore, the dyeing composition exhibits excellent effects of reducing effects on the environment, the human body, and the like.

The present invention will be described hereinbelow more specifically by means of Examples, without intending to limit the present invention to these Examples.

### EXAMPLES

The examples relating to neutral phenol oxidase I are according to the invention, while the examples relating to neutral phenol oxidase II and neutral phenol oxidase III are reference examples.

### Example 1 Search for and preparation of phenol oxidase from Flammulina velutipes IFO 30601 strain

### (1) Investigation of culture conditions

Among culture conditions for inducing phenol oxidase, the investigation of incubation pH conditions is described as an example.

Regarding a method for producing laccase, Japanese Patent Publication No. Sho 60-156385 discloses a method comprising culturing *Irpex lacteus* ATCC20123 for 3 days at pH 6.0, to thereby obtain acid laccase having an optimum pH of around 4.5.

In this example, incubation pH conditions were examined by carrying out cultivation using culture media with arbitrary pH values of 4.0 to 9.0, in order to determine a culture method for inducing phenol oxidase in *Flammulina velutipes* IFO 30601 strain.

Each of operations such as inoculation, cutting of mycelium, and addition of culture media as shown below was carried out in a clean bench.

*Flammulina velutipes* IFO 30601 strain was sown to a sterilized petri dish containing 10 ml of a solid culture agar medium {composition: 2.4% by weight potato dextrose broth (manufactured by Difco), 2.0% by weight agar, and balance water} in an amount equivalent to a single loop of platinum, and cultured at 25°C for 10 days. Thereafter, the hyphae grown on the whole agar medium were cut into small pieces of 5 mm each side with a sterilized loop of platinum.

Ten pieces of the above-mentioned small pieces were sown to a liquid culture medium 1 {composition: 2.4% by weight potato dextrose broth (manufactured by Difco), and balance water (pH 5.2); sterilized at 121°C for 15 minutes}, and culture with reciprocal shaking (150 rpm) was carried out at 25°C for 7 days. In addition, as the liquid culture medium 1, the medium prepared so as to have any pH of 4.0 to 9.0 was used.

The difference in induction of an enzyme according to incubation pH conditions was clarified by sampling each of the culture solutions with different incubation pH was sampled every day, to thereby determine a change in the enzymatic activity of the culture solution.

The enzymatic activity (oxidative activity) was determined by measuring a change in the absorbance at 470 nm using sodium acetate buffer (pH 5.0) or Tris-HCl buffer (pH 8.0) as a buffer and para-phenylenediamune as a substrate.

Concretely, in each well of a 96-well microplate (manufactured by Coming, Coster (registered trademark) 3368), 0.08 ml of a 0.025 M aqueous solution of para-phenylenediamine was added to 0.1 ml of the 0.2 M buffer to thereby prepare a substrate solution. The enzymatic activity was determined by mixing the resulting substrate solution with 0.02 ml of each of the sampled culture solution, and measuring a change in the absorbance at 470 nm of the obtained mixture using a Multi Spectrometer (manufactured by Dainippon Pharmaceutical Co., Ltd.; trade name: Viento). Here, regarding the enzymatic activity, the enzyme amount for increasing the absorbance by 1 per minute at 470 nm per one minute was defined as one unit (U).

As a result of culture for 7 days under each of incubation pH conditions, the measurement of the enzymatic activity of each of the culture solutions at reaction pHs of 5.0 and 8.0, in either case, showed its highest enzymatic activity on the 7th day of the culture time period, and an increase of the enzymatic activities with the passage of the culture time period in each of the incubation pH conditions.

As a result of the measurement of the enzymatic activity at a reaction pH of 5.0, apparent enzymatic activity was not found in each of the culture solutions cultured at different pH in the range of 4.0 to 9.0. Concretely, although the culture solution obtained by culturing for 7 days at an incubation pH of 9.0 showed the highest enzymatic activity, a change in the absorbance at 470 nm after a lapse of 17 hours from the initiation of reaction was only 0.12. In addition, , a change in the absorbance at 470 nm after a lapse of 17 hours from the initiation of reaction for the culture solution obtained by culturing for 7 days at an incubation pH of 6.0 was 0.06.

On the other hand, as a result of the measurement of the enzymatic activity at a reaction pH of 8.0, apparent enzymatic activity was found in each of the culture solutions cultured at different pH in the range of 5. In addition, although the culture solution cultured at an incubation pH of 4.0 showed no apparent enzymatic activity, an increase of the enzymatic activity with the passage of the culture time period was found.

Further, the culture solution for which incubation pH was higher pH showed higher enzymatic activity. The culture solution showing the highest enzymatic activity is a culture solution obtained by culturing for 7 days at an incubation pH of 9.0, of which changes in the absorbance at 470 nm after a lapse of 17 hours is 1.27. A change in the absorbance at 470 nm after a lapse of 17 hours from the initiation of reaction the culture solution obtained by culturing for 7 days at an incubation pH of 6.0 was 0.78.

From the above-mentioned results, it was found that the phenol oxidase induced in *Flammulina velutipes* IFO 30601 strain under the above-mentioned culture conditions, was a neutral phenol oxidase, and that acidic laccase or acidic phenol oxidase having optimum pH in the neutral region was not induced. In addition, it was found that the large amounts of the enzymes were induced in *Flammulina velutipes* IFO 30601 strain under the above-mentioned culture conditions. Regarding the enzymatic activity at pH 8.0, it was found that the enzymatic activity of the culture solution obtained by culturing for 7 days at an incubation pH of 9.0 was about 1.6 times as high as that of the culture solution obtained by culturing for 7 days at an incubation pH of 6.0.

As described above, under the above-mentioned culture conditions, it is possible to induce a neutral phenol oxidase and produce the neutral phenol oxidase in good yield.

### (2) Investigation of culture method

Each of operations such as inoculation, cutting of mycelium, and addition of culture media as shown below was carried out in a clean bench. Regarding culture, it was examine a method comprising carrying out firstly, culture for growing fungus bodies, and then carrying out culture for increasing the yield of an enzyme.

*Flammulina velutipes* IFO 30601 strain was sown to a sterilized petri dish containing 10 ml of a solid culture agar medium {composition: 2.4% by weight potato dextrose broth (manufactured by Difco), 2.0% by weight agar, and balance water} in an amount equivalent to a single loop of platinum, and cultured at 25°C for 10 days. Thereafter, the hyphae grown on the whole agar medium were cut into small pieces of 5 mm each side with a sterilized loop of platinum.

Ten pieces of the above-mentioned small pieces were sown to a liquid culture medium 1 {composition: 2.4% by weight potato dextrose broth (manufactured by Difco), and balance water (pH 5.2); sterilized at 121°C for 15 minutes}, and culture with reciprocation shaking (150 reciprocations/min.) was carried out at 25°C for 7 days.

Total volume of the culture solution was added to 500 ml of the above-mentioned liquid culture medium 1 contained in a 2-liter Erlenmeyer flask, and thereafter reciprocal shaking culture (100 strokes/minute) was carried out for 3 weeks at 25°C.

Thereafter, the grown pellet-like mycelium was allowed to stand to be precipitated, to remove a culture supernatant. In addition, 500 ml of liquid culture medium 2 {composition: 1.0% by weight glucose, 0.1% by weight yeast extract, 0.14% by weight (NH₄)₂SO₄, 0.36% by weight K₂HPO₄, 0.02% by weight MgSO₄•7H₂O, 0.1% mineral mixed solution (composition: 1.0% by weight CuSO₄•5H₂O, 1.0% by weight ZnCl₂, 0.7% by weight FeCl₃•6H₂O, 0.5% by weight CoSO₄•7H₂O, 0.5% by weight MnCl₂•4H₂O), pH 9.2; sterilized at 121°C for 15 minutes} was added to the remaining hyphae, and culture was further carried out at 25°C for 3 days.

Thereafter, the grown pelletal hyphae were allowed to stand to be precipitated. The culture was collected by decantation.

The resulting culture was a pale yellow or yellowish brown, transparent or turbid liquid. The collected culture solution had a total volume of 3060 ml, a total titer of 13,100 U, a total amount of protein of 778 mg, and a specific activity of 16.8 U/mg protein.

### (3) Preparation of crude enzyme solution

The culture solution collected in the above item (2) was filtered under reduced pressure, to thereby obtain filtrate.

The pH of the filtrate was adjusted to pH 7.5 with a 1 M aqueous solution of sodium hydroxide. Five-gram (dry weight) of DEAE-cellulose (manufactured by Sigma) carrier was added per 1 L of the filtrate after pH adjustment, and thereafter the resulting mixture was stirred with shaking for 30 minutes at 4°C. Thereafter, the mixture was allowed to stand for 10 minutes, and then a supernatant was removed to collect the DEAE-cellulose carrier.

The collected DEAE-cellulose carrier was added to 0.1 M sodium phosphate buffer (pH 6.5) containing 1 M sodium chloride in an amount of 3 times the volume of the carrier. The resulting mixture was stirred with shaking for 5 minutes to elute a protein adsorbed to the DEAE-cellulose carrier. The eluent obtained by DEAE-cellulose was collected by means of filtration under reduced pressure and dialyzed against deionized water at 4°C. The resulting product was lyophilized, to thereby obtain a lyophilized product (total weight: 630 mg, total titer: 7326 U, total amount of protein: 110 mg, specific activity: 66.4 U/mg protein). Regarding the titer of the lyophilized product, the enzymatic activity is determined by dissolving 1 mg of the lyophilized product in 1 ml of 0.1 M sodium phosphate buffer (pH 7.0), mixing 0.01 milliliter of the thus obtained solution with a substrate solution obtained by adding 0.1 ml of 0.05 M 2,6-dimethoxyphenol (final concentration: 0.005 mM) to 0.089 ml of 0.1 M sodium phosphate buffer (pH 7.0), and measuring a change in the absorbance at 470 nm in the same manner as in the above item (1) for the thus obtained mixture. Further, the amount of protein was quantified by Folm-Lowry method using an aqueous solution of the lyophilized product.

A solution in which the lyophilized product obtained by the DEAE-cellulose was again dissolved was subjected to native-PAGE. A gel after electrophoresis was stirred with shaking for 20 minutes in 0.1 M sodium phosphate buffer (pH 7.0). Thereafter, the gel was stirred with shaking for 5 minutes in 0.1 M sodium phosphate buffer (pH 7.0) containing any one of 0.005 M 2,6-dimethoxyphenol, 0.005 M ortho-aminophenol, and 0.001 M para-phenylenediamine, to thereby carry out activity staining. The result of activity staining is shown in Fig. 1.

As shown in Fig. 1, from the result of activity staining, it was found that the culture solution produced by culturing *Flammulina velutipes* IFO 30601 strain according to the culture method described in the item (2) contains at least three kinds of neutral phenol oxidases.

### (4) Purification of neutral phenol oxidase {Ion-exchange chromatography (Q-Sepharose column)}

Six-hundred -and- thirty milligrams of the lyophilized product obtained in the above item (3) was dissolved in 10 ml of 0.05 M Tris-HCl buffer (pH 8.0). Thereafter, insoluble matter was removed by means of centrifugal filtration using DISMIC-13P (trade name, manufactured by ADVANTEC), to thereby obtain filtrate.

Forty nine milliliters of the filtrate was applied to Q-Sepharose column (trade name, 2.6 × 10 cm; manufactured by Pharmacia) equilibrated with 0.05 M Tris-HCl buffer (pH 8.0) by the use of FPLC system (manufactured by Pharmacia). Two kinds of neutral phenol oxidases (the neutral phenol oxidase I and the neutral phenol oxidase II) out of neutral phenol oxidases adsorbed to the column were eluted by means of gradient elution with a range of concentration from a 0 to 1 M aqueous solution of sodium chloride. A fraction of the neutral phenol oxidase I or the neutral phenol oxidase II was determined by measuring the enzymatic activity of each of the resulting elution fractions for 2,6-dimethoxyphenol at 470 nm and then carrying out activity staining for each of the resulting elution fractions with para-phenylenediamine, after native-PAGE.

The enzymatic activity in the case where 2,6-dimethoxyphenol was used as a substrate was determined by adding 0.1 ml of a 0.05 M aqueous solution of 2,6-dimethoxyphenol to 0.896 ml of 0.2 M sodium phosphate (pH 7.0), to thereby obtain a substrate solution, mixing the resulting substrate solution with 0.004 ml of the elution fraction, and measuring a change in the absorbance at 470 nm of the resulting mixture.

The chromatogram of ion-exchange chromatography is shown in Fig. 2. In Fig. 2, a change in the absorbance at 280 nm (A280 in Fig. 2) represents a change in an amount of protein contained in each fraction, a change in the absorbance at 400 nm (A400 in Fig. 2) being a change in an amount of glucide contained in each fraction, and a change in the absorbance at 470 nm (A470 in Fig. 2) being a change in the enzymatic activity in each fraction.

The result of activity staining with para-phenylenediamine after native-PAGE for the fraction of the neutral phenol oxidase I and the fraction of the neutral phenol oxidase II each showed a single band, but the result of Coomassie Brilliant Blue staining after native-PAGE for each of them showed plural bands. Each of the fraction of the neutral phenol oxidase I and the fraction of the neutral phenol oxidase II was collected, and then lyophilized after dialysis.

The resulting fraction of the neutral phenol oxidase I had a total volume of 7.5 ml, a total titer of 2505 U, a total amount of protein of 5.4 mg, and a specific activity of 463.9 U/mg protein. On the other hand, the resulting fraction of the neutral phenol oxidase II had a total volume of 10.5 ml, a total titer of 3630 U, a total amount of protein of 41.0 mg, and a specific activity of 88.5 U/mg protein.

### (5) Purification of neutral phenol oxidase I and neutral phenol oxidase II (gel filtration chromatography)

The lyophilized product of the neutral phenol oxidase I obtained in the above item (3) was dissolved in 1.5 ml of 0.05 M Tris-HCl buffer (pH 8.0) containing 0.1 M sodium chloride. The resulting product was applied to Sephacryl S-100 HR column (trade name, 1.6 × 60 cm; manufactured by Pharmacia) equilibrated with 0.05 M Tris-HCl buffer (pH 8.0) containing 0.1 M sodium chloride by the use of FPLC system (manufactured by Pharmacia). Then, the neutral phenol oxidase I adsorbed to the column was eluted with 0.05 M Tris-HCl buffer (pH 8.0) containing 0.1 M sodium chloride.

The chromatogram of gel filtration chromatography for the neutral phenol oxidase I is shown in Fig. 3. In Fig. 3, a change in the absorbance at 280 nm (A280 in Fig. 3) shows a change in an amount of protein contained in each fraction, and a change in the absorbance at 470 nm (A470 in Fig. 3) being a change in the enzymatic activity in each fraction.

A fraction of the neutral phenol oxidase I was determined by carrying out enzymatic activity assay of each of the fractions thus obtained for 2,6-dimethoxyphenol at 470 nm, activity staining with para-phenylenediamine after SDS-PAGE and Coomassie Brilliant Blue staining after SDS-PAGE. As the results of activity staining with para-phenylenediamine and Coomassie Brilliant Blue staining after SDS-PAGE, a single band was found.

The enzymatic activity in the case where 2,6-dimethoxyphenol was used as a substrate was determined by adding 0.1 ml of a 0.05 M aqueous solution of 2,6-dimethoxyphenol to 0.896 ml of 0.2 M sodium phosphate (pH 7.0), to thereby obtain a substrate solution, mixing the resulting substrate solution with 0.004 ml of the fraction of the neutral phenol oxidase I, and measuring a change in the absorbance at 470 nm of the resulting mixture.

A fraction which showed enzymatic activity and was stained as a result of activity staining was collected as the neutral phenol oxidase I. The resulting fraction of the neutral phenol oxidase I had a total volume of 10 ml, a total titer of 1530 U, a total amount of protein of 1.6 mg, and a specific activity of 956.3 U/mg protein.

On the other hand, the lyophilized product of the neutral phenol oxidase II obtained in the above item (3) was dissolved in 1.5 ml of 0.05 M Tris-HCl buffer (pH 8.0) containing 0.1 M sodium chloride. The resulting product was applied to Sephacryl S-100 HR column (trade name, 1.6 × 60 cm; manufactured by Pharmacia) equilibrated with 0.05 M Tris-HCl buffer (pH 8.0) containing 0.1 M sodium chloride by the use of FPLC system (manufactured by Pharmacia). Then, the neutral phenol oxidase II of the present invention adsorbed to the column was eluted with 0.05 M Tris-HCl buffer (pH 8.0) containing 0.1 M sodium chloride.

The chromatogram of gel chromatography for the neutral phenol oxidase II is shown in Fig. 4. In Fig. 4, a change in the absorbance at 280 nm (A280 in Fig. 4) represents a change in an amount of protein contained in each fraction, and a change in the absorbance at 470 nm (A470 in Fig. 4) being a change in the enzymatic activity in each fraction.

A fraction of the neutral phenol oxidase II of the present invention was determined by carrying out enzymatic activity assay of each of the resulting fractions for 2,6-dimethoxyphenol at 470 nm, activity staining with para-phenylenediamine after SDS-PAGE and Coomassie Brilliant Blue staining after SDS-PAGE. As the results of activity staining with para-phenylenediamine and Coomassie Brilliant Blue staining after SDS-PAGE, a single band was found.

The enzymatic activity in the case where 2,6-dimethoxyphenol was used as a substrate was determined by adding 0.1 ml of a 0.05 M aqueous solution of 2,6-dimethoxyphenol to 0.896 ml of 0.2 M sodium phosphate (pH 7.0), to thereby obtain a substrate solution, mixing the resulting substrate solution with 0.004 ml of the fraction of the neutral phenol oxidase II, and measuring a change in the absorbance at 470 nm of the resulting mixture.

A fraction which showed enzymatic activity and was stained as a result of activity staining was collected as the neutral phenol oxidase II. The resulting fraction of the neutral phenol oxidase II had a total volume of 11.5 ml, a total titer of 742 U, a total amount of protein of 5.3 mg, and a specific activity of 140 U/mg protein.

The scheme of the above-mentioned extraction and purification is shown in Fig. 5. The degree of purification and specific activity of the neutral phenol oxidase I and the neutral phenol oxidase II in each of the purification processes are shown in Table 1.

**Table 1**

| Purification Step | | Total amount of proteins | Total activity | Specific activity | Yield |
|---|---|---|---|---|---|
| | | (mg) | (U) | (U/mg protein) | (%) |
| Culture | | 778 | 13100 | 16.8 | 100.0 |
| DEAE-cellulose | | 110 | 7326 | 66.4 | 55.9 |
| Q-Sepharose | Neutral phenol oxidase I | 5.4 | 2505 | 463.9 | 19.1 |
| | Neutral phenol oxidase II | 41.0 | 3630 | 88.5 | 27.7 |
| Sephacryl S-100 HR | Neutral phenol oxidase I | 1.6 | 1530 | 956.3 | 11.7 |
| | Neutral phenol oxidase II | 5.3 | 742 | 140.0 | 5.7 |

### (6) Purification of neutral phenol oxidase III {Ion-exchange chromatography (DEAE-cellulose column)}

A culture solution was collected in the same manner as in the above item (2) of Example 1. The collected culture solution had a total volume of 4000 ml, a total titer of 26800 U, a total amount of protein of 1188 mg, and a specific activity of 22.5 U/mg protein.

The collected culture solutions were respectively poured into each of dialysis membranes {trade name: Dialysis membrane, Size 36 (manufactured by Wako Pure Chemical Industries, Ltd.)} so that each of the dialysis membranes had 250 ml of the culture solution. Each of the prepared tubes was put into a plastic bag, and then the tube was covered with about 120 g of polyethylene glycol 2000 (manufactured by Wako Pure Chemical Industries, Ltd.). The plastic bags each containing the tube therein were incubated in the ice for about 48 hours, to thereby concentrate the culture solution. The concentrated culture solution was centrifuged at 10000 rpm for 20 minutes at 4°C using a small-sized high-speed refrigerated centrifuge (Rotor No. 8.1; manufactured by TOMY), to thereby remove a precipitate. The resulting concentrate of the culture solution had a total volume of 229 ml, a total titer of 9,814 U, a total amount of protein of 193.6 mg, and a specific activity of 50.7 U/mg protein.

The concentrate of the culture solution was dialyzed overnight against 0.05 M sodium phosphate buffer (pH 7.0) at 4°C. A supernatant obtained by centrifugation at 12,000 rpm for 30 minutes at 4°C using a small-sized high-speed refrigerated centrifuge (Rotor No. 8.1; manufactured by TOMY) was filtered using Disposable syringe filter unit (trade name)(cellulose acetate, 0.45 µm; manufactured by ADVANTEC) to remove insoluble matter, thereby giving filtrate.

Two-hundred and twenty nine milliliters of the filtrate was applied to DEAE-cellulose column (2.6 × 60 cm; manufactured by Sigma) equilibrated with 0.05 M sodium phosphate buffer (pH 7.0). Then, the neutral phenol oxidase III adsorbed to the column was eluted by means of gradient elution with a range of concentration from 0 to 1 M sodium chloride. A fraction of the neutral phenol oxidase III was determined by measuring enzymatic activity assay of each of the obtained elution fractions for 2,6-dimethoxyphenol at 470 nm, and activity staining with para-phenylenediamine after SDS-PAGE.

The chromatogram of DEAE-cellulose column chromatography for the neutral phenol oxidase III is shown in Fig. 6. In Fig. 6, a change in the absorbance at 280 nm (A280 in Fig. 6) represents a change in an amount of protein contained in each fraction, and a change in the absorbance at 470 nm (A470 in Fig. 6) being a change in the enzymatic activity in each fraction.

The enzymatic activity in the case where 2,6-dimethoxyphenol was used as a substrate was determined by adding 0.1 ml of a 0.05 M aqueous solution of 2,6-dimethoxyphenol to 0.895 ml of 0.2 M sodium phosphate buffer (pH 7.0), to thereby obtain a substrate solution, mixing the resulting solution with 0.005 ml of the elution fraction, and measuring a change in the absorbance at 470 nm of the resulting mixture.

The resulting fraction of the neutral phenol oxidase III had a total volume of 18 ml, a total titer of 1102 U, a total amount of protein of 3.6 mg, and a specific activity of 303 U/mg protein.

### (7) Purification of neutral phenol oxidase III {Ion-exchange chromatography (Q-Sepharose column)}

The resulting fraction of the neutral phenol oxidase III was collected, dialyzed overnight against 0.05 M Tris-HCl buffer (pH 8.0) at 4°C, and filtered using Disposable syringe filter unit (trade name)(cellulose acetate, 0.45 µm; manufactured by ADVANTEC) to remove insoluble matter, thereby giving filtrate.

To Q-Sepharose column (trade name, 2.6 × 10 cm; manufactured by Pharmacia) equilibrated with 0.05 M Tris-HCl buffer (pH 8.0) was applied 21.5 ml of the filtrate by the use of FPLC system (manufactured by Pharmacia). The neutral phenol oxidase III adsorbed to the column was eluted by means of gradient elution with a range of concentration from 0.2 to 0.5 M sodium chloride. A fraction of the neutral phenol oxidase III was determined by an enzymatic activity assay of each of the resulting elution fractions for 2,6-dimethoxyphenol at 470 nm, and activity staining with para-phenylenediamine after SDS-PAGE.

The chromatogram of Q-Sepharose column chromatography for the neutral phenol oxidase III is shown in Fig. 7. In Fig. 7, a change in the absorbance at 280 nm (A280 in Fig. 7) represents a change in an amount of protein contained in each fraction, and a change in the absorbance at 470 nm (A470 in Fig. 7) being a change in the enzymatic activity in each fraction.

The enzymatic activity in the case where 2,6-dimethoxyphenol was used as a substrate was determined by adding 0.1 ml of a 0.05 M aqueous solution of 2,6-dimethoxyphenol to 0.895 ml of 0.2 M sodium phosphate buffer (pH 7.0), to thereby obtain a substrate solution, mixing the substrate solution with 0.005 ml of the elution fraction, and measuring a change in the absorbance at 470 nm of the thus obtained mixture.

The resulting fraction of the neutral phenol oxidase III had a total volume of 18 ml, a total titer of 115 U, a total amount of protein of 1 mg, and a specific activity of 117.4 U/mg protein.

The fraction of the neutral phenol oxidase III was dialyzed against 0.05 M sodium phosphate buffer (pH 7.0) at 4°C for 3 hours. A filtrate was obtained by filtration using Disposable syringe filter unit (trade name)(cellulose acetate, 0.45 µm; manufactured by ADVANTEC) to remove insoluble matters. The resulting filtrate was applied to DEAE-cellulose column (manufactured by Sigma; 1.5 × 5.5 cm) equilibrated with 0.05 M sodium phosphate buffer (pH 7.0). Then, the neutral phenol oxidase III adsorbed to the column was eluted with 0.05 M sodium phosphate buffer (pH 7.0) containing 1 M sodium chloride.

A fraction of the neutral phenol oxidase III was determined by enzymatic activity assay of each of the resulting fractions for 2,6-dimethoxyphenol, and activity staining with para-phenylenediamine after SDS-PAGE and Coomassie Brilliant Blue staining after SDS-PAGE. As a result of activity staining with para-phenylenediamine after SDS-PAGE, one major band and two minor bands were found. As a result of Coomassie Brilliant Blue staining after SDS-PAGE, a single band was found.

Regarding the enzymatic activity in the case where 2,6-dimethoxyphenol was used as a substrate, a change in the color of the resulting mixture was visually confirmed, wherein the mixture is obtained by adding 0.02 ml of a 0.05 M aqueous solution of 2,6-dimethoxyphenol to 0.178 ml of 0.2 M sodium phosphate buffer (pH 7.0) in each well of a 96-well microplate {manufactured by Coming; Coster (trade mark) 3368}, to thereby obtain a substrate solution, and mixing the resulting substrate solution with 0.002 ml of the elution fraction.

A fraction which showed enzymatic activity and which was stained as a result of activity staining was collected as the neutral phenol oxidase III. The resulting fraction of the neutral phenol oxidase III had a total volume of 3.3 ml, a total titer of 242 U, a total amount of protein of 0.6 mg, and a specific activity of 423.3 U/mg protein.

The scheme of the extraction and purification of the neutral phenol oxidase III is shown in Fig. 8. The degree of purification and specific activity of the neutral phenol oxidase III in each of the purification processes are shown in Table 2.

**Table 2**

| Purification Step | Total amount of Total | | Specific Activity | Yield |
|---|---|---|---|---|
| | protein (mg) | activity (U) | (U/mg proteins) | (%) |
| PEG concentrate of culture | 194 | 9814 | 50.7 | 100.0 |
| DEAE-Sepharose | 3.6 | 1102 | 303.0 | 11.2 |
| Q-Scpharose | 1.0 | 115 | 117.4 | 1.2 |
| DEAE-cellulose | 0.6 | 242 | 423.3 | 2.5 |

### Example 2 Molecular weight of each of neutral phenol oxidase I, neutral phenol oxidase II, and neutral phenol oxidase III

The molecular weight of each of the neutral phenol oxidase I and the neutral phenol oxidase II obtained in Example 1 was measured by a gel filtration using gel filtration chromatography.

In the gel filtration chromatography, there is used Sephacryl S-100 HR column (1.6 × 60 cm; manufactured by Pharmacia) equilibrated with 0.05 M Tris-HCl buffer (pH 8.0) containing 0.1 M sodium chloride, by the use of FPLC system (manufactured by Pharmacia).

Three milliliters of the solution of the neutral phenol oxidase I or the neutral phenol oxidase II obtained in the above item (5) of Example 1 was applied to Sephacryl S-100 HR column (trade name, 1.6 × 60 cm; manufactured by Pharmacia) equilibrated with 0.05 M Tris-HCl buffer (pH 8.0) containing 0.1 M sodium chloride by the use of FPLC system (manufactured by Pharmacia). Gel filtration chromatography was carried out with 0.05 M Tris-HCl buffer (pH 8.0) containing 0.1 M sodium chloride by using the column, to elute the neutral phenol oxidase I or the neutral phenol oxidase II, and thereafter the volume of eluate was measured. The molecular weight of each of the neutral phenol oxidase I and the neutral phenol oxidase II was determined by carrying out gel filtration chromatography separately. In addition, under the same conditions as those described above, four kinds of proteins each having known molecular weights (about 67 kDa, 43 kDa, 25 kDa, and 13.7 kDa) as molecular weight markers were separately added to measure the volume of eluate of each of the molecular weight markers.

The molecular weight of each of the neutral phenol oxidase I and the neutral phenol oxidase II was determined from the elution time of the neutral phenol oxidase I or the neutral phenol oxidase II and a calibration curve prepared using the volume of eluate of each of the molecular weight markers.

As a result, each of the neutral phenol oxidase I and the neutral phenol oxidase II had a molecular weight of about 72 kDa.

Further, the molecular weight of the neutral phenol oxidase III obtained in the above item (7) of Example 1 was measured by a gel filtration using gel filtration chromatography.

The fraction of the neutral phenol oxidase III obtained in the above item (7) of Example 1 after Q-Sepharose column (total volume: 12 ml, total titer: 262 U, total amount of protein: 3.8 mg, specific activity: 69 U/mg protein) was concentrated by centrifugal filtration using trade name: Ultrafree (trade mark)-MC 30,000 NMWL Filter Unit (manufactured by MILLIPORE).

To a column (2.6 × 60 cm) filled with Sephadex G-100 (trade name, manufactured by Amersham Biosciences) and equilibrated with 0.05 M sodium phosphate buffer (pH 7.0) was applied 0.855 ml of the resulting concentrate. Then, gel filtration chromatography was carried out using 0.05 M sodium phosphate buffer (pH 7.0), to elute the neutral phenol oxidase III, thereby measuring the volume of eluate. Further, under the same conditions as those described above, five kinds of proteins each having known molecular weight (about 73 kDa, 61 kDa, 47 kDa, 37 kDa, and 24 kDa) were separately added as molecular weight markers, to measure the volume of eluate.

The chromatogram of gel filtration chromatography for the neutral phenol oxidase III is shown in Fig. 9. In Fig. 9, a change in the absorbance at 280 nm (A280 in Fig. 9) represents a change in an amount of protein contained in each fraction, and a change in the absorbance at 470 nm (A470 in Fig. 9) being a change in the enzymatic activity in each fraction.

The enzymatic activity in the case where 2,6-dimethoxyphenol was used as a substrate was calculated by adding 0.1 ml of a 0.05 M aqueous solution of 2,6-dimethoxyphenol to 0.895 ml of 0.2 M sodium phosphate buffer (pH 7.0), to thereby obtain a substrate solution, mixing the resulting substrate solution with 0.005 ml of the elution fraction, and measuring a change in the absorbance at 470 nm of the resulting mixture.

As a result, the neutral phenol oxidase III had a molecular weight of about 45 kDa.

Five microliters of the solution of each of the neutral phenol oxidase I and the neutral phenol oxidase II obtained in the above item (5) of Example 1, and the neutral phenol oxidase III obtained in the above item (7) of Example 1, and 15 microliters of a molecular weight marker (manufactured by Amersham Biosciences; trade name: LMW calibration kit) were subjected to 12.5 % SDS-PAGE at the same time.

Activity staining was carried out by stirring the gel after SDS-PAGE in 0.1 M sodium phosphate buffer (pH 7.0) containing 2.5 % Triton (trade mark) X-100 with shaking for 60 minutes, and then stirring the gel in 0.1 M sodium phosphate buffer (pH 7.0) containing 1 mM para-phenylenediamine with shaking for 5 minutes. Thereafter, staining is carried out by washing the gel after electrophoresis with distilled water for 5 minutes, and then immersing the gel in a staining solution {obtained by dissolving 0.5 g of Coomassie Brilliant Blue R250 in a mixed solution of 500 ml of methanol and 100 ml of acetic acid (total 600 ml) and adding distilled ion-exchange water to the mixed solution so as to give a total volume of 1,000 ml}. Then, decolorization was carried out using a decolorizing solution (obtained by adding distilled ion-exchange water to the mixed solution of 250 ml of methanol and 70 ml of acetic acid (total 320 ml) so as to give a total volume of 1,000 ml).

As a result of SDS-PAGE, each of the neutral phenol oxidase I, the neutral phenol oxidase II, and the neutral phenol oxidase III of the present invention showed a single band. The molecular weights of the neutral phenol oxidase I, the neutral phenol oxidase II and the neutral phenol oxidase III of the present invention as determined by SDS-PAGE were 28 kDa, 35 kDa, and 45 kDa, respectively.

### Example 3 Optimum pH of each of neutral phenol oxidase I, neutral phenol oxidase II, and neutral phenol oxidase III

In the reaction of the neutral phenol oxidase I or the neutral phenol oxidase II with a substrate, 0.2 M glycine-NaOH buffer (pHs 11.5, 10.5, and 9.5), 0.2 M Tris-HCl buffer (pHs 10.0, 9.0, 8.0, and 7.0), 0.2 M sodium phosphate buffer (pHs 7.5, 7.0, 6.5, 6.0, and 5.5), 0.2 M sodium acetate buffer (pHs 5.5, 5.0, 4.5, 4.0, and 3.5), or 0.2 M glycine-HCl buffer (pHs 3.5, 3.0, and 2.0) was used as a buffer depending on pH.

When 2,6-dimethoxyphenol was used as a substrate, an enzymatic activity was evaluated by mixing adequately 0.02 ml of the fraction of the neutral phenol oxidase I or the neutral phenol oxidase II obtained in the above item (5) of Example 1, 0.88 ml of any one of the buffers mentioned above, and 0.1 ml of a 0.05 M aqueous solution of 2,6-dimethoxyphenol in a microcuvette, and then measuring a change in the absorbance at 470 nm of the thus obtained mixture using a spectrophotometer (manufactured by Jasco; trade name: v-530).

When ortho-aminophenol was used as a substrate, enzymatic activity was evaluated in the same manner as that of the above-mentioned evaluation method in which 2,6-dimethoxyphenol was used as a substrate, except that a 0.05 M dimethylsulfoxide solution of ortho-aminophenol was used instead of the 0.05 M aqueous solution of 2,6-dimethoxyphenol and that a change in the absorbance was measured at 420 nm.

When para-phenylenediamine was used as a substrate, enzymatic activity was evaluated by measuring a change in the absorbance at 470nm in the same manner as that of the above-mentioned evaluation method in which 2,6-dimethoxyphenol was used as a substrate, except that a 0.05 M aqueous solution of para-phenylenediamine was used instead of the 0.05 M aqueous solution of 2,6-dimethoxyphenol.

When syringaldazine was used as a substrate, enzymatic activity was evaluated in the same manner as that of the above-mentioned evaluation method in which 2,6-dimethoxyphenol was used as a substrate, except that a 0.005 M ethanol solution of syringaldazine was used instead of the 0.05 M aqueous solution of 2,6-dimethoxyphenol and that a change in the absorbance was measured at 530 nm.

Here, regarding the enzymatic activity, an amount at appropriate wavelength for each of substrates is defined as 1 unit (U).

The results of the optimum pH of the neutral phenol oxidase I and the optimum pH of the neutral phenol oxidase II are shown in Fig. 10 and Fig. 11, respectively. Figs. 10 and 11 are respectively shown by a relative activity determined when the maximum activity is defined as 100. In each of Figs. 10 and 11, panel A shows the case where 2,6-dimethoxyphenol was used as a substrate, panel B being the case where ortho-aminophenol was used as a substrate, panel C being the case where para-phenylenediamine was used as a substrate, and panel D being the case where syringaldazine was used as a substrate.

As a result, as shown in Fig. 10, regarding the neutral phenol oxidase I, it was found that the optimum pH was about 5.5 to 7.0 when 2,6-dimethoxyphenol was used as a substrate, that the optimum pH was about 5.5 to 7.0 when ortho-aminophenol was used as a substrate, that the optimum pH was about 5.5 to 6.0 when para-phenylenediamine was used as a substrate, and that the optimum pH was about 6.5 when syringaldazine was used as a substrate.

As shown in Fig. 11, regarding the neutral phenol oxidase II, it was found that the optimum pH of was about 5.5 to 6.0 when 2,6-dimethoxypphenol was used as a substrate, that the optimum pH was about 5.5 to 7.0 when ortho-aminophenol was used as a substrate, that the optimum pH was about 5.5 to 6.0 when para-phenylenediamine was used as a substrate, and that the optimum pH of the neutral phenol oxidase II was about 6.5 when syringaldazine was used as a substrate.

In the reaction of the neutral phenol oxidase III with each of substrates, 0.2 M glycine-NaOH buffer (pH 11.5, 11, 10.5, 10, and 9.5), 0.2 M Tris-HCl buffer (pH 10, 9.5, 9, 8.5, 8, 7.5, and 7), 0.2 M sodium phosphate buffer (pH 7.5, 7, 6.5, 6, 5.5, and 5), 0.2 M sodium acetate buffer (pH 5.5, 5, 4.5, 4, and 3.5), or 0.2 M glycine-HCl buffer (pH 4, 3.5, 3, 2.5, and 2) was used as a buffer depending on pH.

When 2,6-dimethoxyphenol was used as a substrate, enzymatic activity was evaluated by mixing adequately 0.04 ml of the fraction of the neutral phenol oxidase III obtained in the above item (7) of Example 1 with 0.86 ml of any one of the above buffers and 0.1 ml of a 0.05 M aqueous solution of 2,6-dimethoxyphenol in a microcuvette, and then measuring a change in the absorbance at 470 nm of the resulting mixture using a spectrophotometer (manufactured by Jasco; trade name: v-530).

When ortho-aminophenol was used as a substrate, enzymatic activity was evaluated by mixing adequately 0.005 ml of the fraction of the neutral phenol oxidase III obtained in the above item (7) of Example 1 with 0.895 ml of any one of the above buffers and 0.1 ml of a 0.05 M dimethylsulfoxide solution of ortho-aminophenol in a microcuvette, and then measuring a change in the absorbance at 420 nm of the resulting mixture using a spectrophotometer (manufactured by Jasco; trade name: v-530).

When para-phenylenediamine was used as a substrate, enzymatic activity was evaluated by 0.01 ml of the fraction of the neutral phenol oxidase III obtained in the above item (7) of Example 1 with 0.89 ml of any one of the above buffers and 0.1 ml of a 0.05 M aqueous solution of para-phenylenediamine in a microcuvette, and then measuring a change in the absorbance at 470 nm of the resulting mixture using a spectrophotometer (manufactured by Jasco; trade name: v-530).

When syringaldazine was used as a substrate, by mixing adequately 0.02 ml of the fraction of the neutral phenol oxidase III obtained in the above item (7) of Example 1 with 0.96 ml of any one of the above buffers and 0.02 ml of a 0.005 M ethanol solution of syringaldazine in a microcuvette, and then measuring a change in the absorbance at 530 nm of the resulting mixture using a spectrophotometer (manufactured by Jasco; trade name: v-530).

Here, regarding the enzymatic activity for the above decolorizing reaction, an amount at appropriate wavelength for each of substrates is defined as 1 unit (U).

The result of the optimum pH of the neutral phenol oxidase III is shown in Fig. 12. Fig. 12 is shown by a relative activity determined when the maximum activity is defined as 100. In addition, panels A in the Fig. 2 shows the case where 2,6-dimethoxyphenol was used as a substrate, panel B being the case where ortho-aminophenol was used as a substrate, panel C being the case where para-phenylenediamine was used as a substrate, and panel D being the case where syringaldazine was used as a substrate.

As the results, as shown in Fig. 12, it was found that the optimum pH of the neutral phenol oxidase III was about 5.5 when 2,6-dimethoxyphenol was used as a substrate, that the optimum pH was about 5.0 to 7.0 when ortho-aminophenol was used as a substrate, that the optimum pH was about 5.0 to 6.0 when para-phenylenediamine was used as a substrate, and that the optimum pH of the neutral phenol oxidase III was about 6.0 when syringaldazine was used as a substrate.

### Example 4 pH stability of each of neutral phenol oxidase I, neutral phenol oxidase II, and neutral phenol oxidase III.

In the reaction of each of the neutral phenol oxidase I, the neutral phenol oxidase II and the neutral phenol oxidase III with each of substrates, 0.2 M glycine-NaOH buffer (pH 11.5), 0.2 M Tris-HCl buffer (pH 10.0, 9.0, or 8.0), 0.2 M sodium phosphate buffer (pH 7.0 or 6.0), 0.2 M sodium acetate buffer (pH 5.0 or 4.0), or 0.2 M glycine-HCl buffer (pH 3.0 or 2.0) was used as a buffer depending on pH.

Pretreatment was carried out by mixing 0.02 ml of the fraction of each of the neutral phenol oxidase I and the neutral phenol oxidase II obtained in the above item (5) of Example 1, and the neutral phenol oxidase III obtained in the above item (7) of Example 1 with 0.18 ml of any one of the above buffers, and then incubating the resulting mixture at 30°C for 1 hour or 20 hours.

Then, enzymatic activity was evaluated by adequately mixing 0.02 ml of the purified enzyme solution after pretreatment, 0.88 ml of the buffer and 0.1 ml of a 0.05 M aqueous solution of 2,6-dimethoxyphenol in a microcuvette, and then measuring a change in the absorbance at 470 nm of the resulting mixture using a spectrophotometer (manufactured by Jasco; trade name: V-530).

When para-phenylenediamine was used as a substrate, enzymatic activity was evaluated by measuring a change in the absorbance at 470nm in the same manner as that of the above-mentioned evaluation method in which 2,6-dimethoxyphenol was used as a substrate, except that a 0.05 M aqueous solution of para-phenylenediamine was used instead of the 0.05 M aqueous solution of 2,6-dimethoxyphenol.

Here, regarding the enzymatic activity, an amount at appropriate wavelength for each of dyes is defined as 1 unit (U).

The result of the pH stability of each of the neutral phenol oxidase I, the neutral phenol oxidase II and the neutral phenol oxidase III is shown in Figs. 13, 14, and 15, respectively. Figs. 13, 14, and 15 are shown by the relative remaining activity determined when the maximum activity is defined as 100. In each of Figs. 13, 14, and 15, panels A and B show cases where 2, 6-dimethoxyphenol was used as a substrate, and panels C and D being cases where para-phenylenediamine was used as a substrate. Further, in each of Figs. 13, 14, and 15, panels A and C show the pH stability after incubation for 1 hour, and panels B and D being the pH stability after incubation for 20 hours.

As the results, as shown in Fig. 13, in the case of treatment for 1 hour, the neutral phenol oxidase I was stable at pH 7.0 to 10.0 and maintained a relative remaining activity of 75 % or more as compared with the maximum activity. In the case of treatment for 20 hours, the neutral phenol oxidase I was stable at pH 8.0 to 9.0 and maintained a relative remaining activity of 70 % or more as compared with the maximum activity.

As shown in Fig. 14, in the case of treatment for 1 hour, the neutral phenol oxidase II was stable at pH 6.0 to 10.0 and maintained a relative remaining activity of 75 % or more as compared with the maximum activity. In the case of treatment for 20 hours, the neutral phenol oxidase II was stable at pH 7.0 to 10.0 and maintained a relative remaining activity of 75 % or more as compared with the maximum activity.

As shown in Fig. 15, in the case of treatment for 1 hour, the neutral phenol oxidase III was stable at pH 7.0 to 11.5 and maintained a relative remaining activity of 50 % or more as compared with the maximum activity. In the case of treatment for 20 hours, the neutral phenol oxidase III was stable at pH 8.0 to 10.0 and maintained a relative remaining activity of 70 % or more as compared with the maximum activity.

### Example 5 Isoelectric point of each of neutral phenol oxidase I, neutral phenol oxidase II and neutral phenol oxidase III

Isoelectric focusing was carried out using a polyacrylamide gel, in order to determine the isoelectric point of each of the neutral phenol oxidase I and the neutral phenol oxidase II obtained in the above item (5) of Example 1, and the neutral phenol oxidase III obtained in the above item (7) of Example 1. Specifically, a polyacrylamide gel having a final concentration of 5 % was prepared by dissolving Pharmalyte (trade name, pH3-10; manufactured by Pharmacia) in acrylamide so as to give a final concentration of 2 %, and then adding ammonium persulfate and N,N,N',N'-tetramethylethylenediamine (TEMED) thereto. As electrode solutions, a 1.0 M aqueous solution of sodium hydroxide was used as a cathode solution and 0.5 M acetic acid was used as an anode solution. Electrophoresis was carried out for 6 hours at a constant voltage of 50 V. Activity staining of the gel was carried out by immersing the gel after electrophoresis in 0.1 M sodium phosphate buffer (pH 7.0) containing 0.001 M para-phenyelnediamine and stirring the gel with shaking. The isoelectric point was measured by, after isoelectric focusing, cutting the both sides of the gel where samples did not electrophorese, to thereby obtain small pieces each having a width of 2.5 mm and measuring the pH of a solution obtained by immersing the small pieces in deionized water at 4°C for extraction.

The result of isoelectric focusing of each of the neutral phenol oxidase I, the neutral phenol oxidase II, and the neutral phenol oxidase III is shown in Fig. 16.

As a result, as shown in Fig. 16, it was determined by isoelectric focusing using a polyacrylamide gel that the isoelectric points of the neutral phenol oxidase I, the neutral phenol oxidase II, and the neutral phenol oxidase III were 7.4, 6.8, and 6.8, respectively.

### Example 6 Optimum temperatures of neutral phenol oxidase I, neutral phenol oxidase II and neutral phenol oxidase III

The enzymatic activity of each of the neutral phenol oxidase I and the neutral phenol oxidase II obtained in the above item (5) of Example 1 and the neutral phenol oxidase III obtained in the above item (7) of Example 1 was separately measured under various temperature conditions (at 20, 30, 40, 50, 60, 70, and 80°C).

Specifically, When 2, 6-dimethoxyphenol was used as a substrate, 0.88 ml of 0.2 M sodium phosphate buffer (pH 6.5), 0.1 ml of a 0.05 M aqueous solution of 2,6-dimethoxyphenol, and 0.02 ml of the fraction of the neutral phenol oxidase I or the neutral phenol oxidase II were mixed in a microcentrifuge tube (manufactured by Porex BioProducts Inc.), and then the resulting mixture was subjected to reaction for 5 minutes at each of the reaction temperatures. Thereafter, 0.1 ml of 2 M glycine-HCl buffer (pH 3.0) was added to the resulting reaction solution, to thereby terminate the enzymatic reaction.

When para-phenylenediamine was used as a substrate, a procedure thereof was carried out in the same manner as that of the above-mentioned evaluation method in which 2,6-dimethoxyphenol was used as a substrate, except that a 0.05 M aqueous solution of para-phenylenediamine was used instead of the aqueous solution of 2,6-dimethoxyphenol.

Then, enzymatic activity was evaluated by measuring the absorbance at 470 nm of the resulting reaction product using a spectrophotometer (manufactured by Jasco; trade name: V-530) in each of the case where 2, 6-dimethoxyphenol was used as a substrate and the case where para-phenylenediamine was used as a substrate.

Here, regarding the enzymatic activity, an amount for increasing the absorbance by 1 per one minute at 470 nm is defined as 1 unit (U).

The result of the optimum temperature of each of the neutral phenol oxidase I and the neutral phenol oxidase II is shown in Figs. 17 and 18, respectively. Figs. 17 and 18 are shown by relative activity determined when the maximum activity is defined as 100. In each of Figs. 17 and 18, panel A shows the case where 2,6-dimethoxyphenol was used as a substrate, and panel B being the case where para-phenylenediamine was used as a substrate.

As the results, as shown in Fig. 17, it was found that when 2,6-dimethoxyphenol was used as a substrate, the optimum temperature of the neutral phenol oxidase I was from 20° to 50°C, wherein the relative activity in the optimum temperature was 80 % or more as compared with the maximum activity, and that when para-phenyelenediamine was used as a substrate, the optimum temperature of the neutral phenol oxidase I was from 30° to 60°C, wherein the relative activity in the optimum temperature is 60 % or more as compared with the maximum activity.

As shown in Fig. 18, it was found that when 2,6-dimethoxyphenol was used as a substrate, the optimum temperature of the neutral phenol oxidase II was from 30° to 60°C, wherein the relative activity in the optimum temperature was 70 % or more as compared with the maximum activity, and that when para-phenylenediamine was used as a substrate, the optimum temperature of the neutral phenol oxidase II was from 40° to 70°C, wherein the relative activity in the optimum temperature is 80 % or more as compared with the maximum activity.

On the other hand, the enzymatic activity of the neutral phenol oxidase III obtained in the above item (7) of Example 1 was measured under various temperature conditions (at 20, 30, 40, 50, 60, 70, and 80°C).

Concretely, when 2,6-dimethoxyphenol was used as a substrate, 0.88 ml of 0.2 M sodium phosphate buffer (pH 6.5) and 0.1 ml of a 0.05 M aqueous solution of 2,6-dimethoxyphenol were mixed in a cuvette, and thereafter the obtained mixture was heated at each of the reaction temperatures for 10 minutes, to thereby obtain a substrate solution.

The fraction of the neutral phenol oxidase III obtained in the above item (7) of Example 1 was diluted 10 times with distilled ion-exchange water, to thereby obtain an enzyme solution. To the substrate solution was added 0.02 ml of the enzyme solution, and thereafter, the resulting mixture was adequately mixed.

The resulting mixture was subjected to reaction at each of the reaction temperatures for 5 minutes. Then, the enzymatic activity was evaluated by measuring the absorbance at 470 nm of the resulting reaction product using a spectrophotometer (manufactured by Jasco; trade name: v-530).

When para-phenylenediamine was used as a substrate, the enzymatic activity was evaluated by carrying out the same procedures in the case where the 2,6-dimethoxyphenol was used as a substrate, except that a 0.05 M aqueous solution of para-phenylenediamine was used instead of the 0.05 M aqueous solution of 2,6-dimethoxyphenol to measure a change in the absorbance at 470nm in the same manner.

Here, regarding the enzymatic activity, an amount for increasing the absorbance by 1 per one minute at 470 nm is defined as 1 unit (U).

The result of the optimum temperature of the neutral phenol oxidase III is shown in Fig. 19. Fig. 19 is shown by relative activity determined when the maximum activity is defined as 100. In Fig. 19, panel A shows the case where 2,6-dimethoxyphenol was used as a substrate, and panel B being the case where para-phenylenediamine was used as a substrate.

As the results, as shown in Fig. 19, it was found that when 2,6-dimethoxyphenol was used as a substrate, the optimum temperature of the neutral phenol oxidase III is from 20 to 50°C, wherein the relative activity in the optimum temperature is 70 % or more as compared with the maximum activity, and that when para-phenyelenediamine was used as a substrate, the optimum temperature of the neutral phenol oxidase III is from 20 to 60°C, wherein the relative activity in the optimum temperature is 60 % or more as compared with the maximum activity.

### Example 7 Thermal stability of each of neutral phenol oxidase I, neutral phenol oxidase II, and neutral phenol oxidase III

The thermal stability of each of the neutral phenol oxidase I and the neutral phenol oxidase II was examined at pH 7.0 and pH 9.0.

Pretreatment was carried out by incubating 0.005 ml of the fraction of the neutral phenol oxidase I or the neutral phenol oxidase II obtained in the above item (5) of Example 1 in 0.045 ml of 0.2 M sodium phosphate buffer (pH 7.0) (total volume 0.005 ml) for various periods of time (5, 10, 20, 40, and 60 minutes) at various temperatures (20, 30, 40, 50, 60, 70, and 80°C).

When 2,6-dimethoxyphenol was used as a substrate, the enzymatic activity was evaluated by mixing adequately 0.02 ml of the pretreated enzyme solution, 0.88 ml of 0.2 M sodium phosphate buffer (pH 6.5), and 0.1 ml of a 0.05 M aqueous solution of 2,6-dimethoxyphenol in a microcuvette, and then measuring a change in the absorbance at 470 nm of the resulting mixture using a spectrophotometer (manufactured by Jasco; trade name: V-530).

When para-phenylenediamine was used as a substrate, the enzymatic activity was evaluated by carrying out the same procedure in the case where 2,6-dimethoxyphenol was used as a substrate, except that a 0.05 M aqueous solution of para-phenylenediamine was used instead of the aqueous solution of 2,6-dimethoxyphenol, and then measuring a change in the absorbance at 470nm in the same manner.

Here, regarding the enzymatic activity, an amount for increasing the absorbance by 1 per one minute at 470 nm is defined as 1 unit (U).

When the thermal stability at pH 9.0 of each of the neutral phenol oxidase I and the neutral phenol oxidase II was determined, pretreatment was carried out by incubating 0.048 ml of the fraction of the neutral phenol oxidase I or the neutral phenol oxidase II obtained in the above item (5) of Example 1 in 0.012 ml of 0.5 M Tris-HCl buffer (pH 9.0) at various temperatures (30, 40, 50, 60, 70, and 80°C) for various periods of time (5, 10, 20, 40, and 60 minutes), instead of the pretreatment at pH 7.0 described above.

The results of the thermal stabilities of the neutral phenol oxidase I and the neutral phenol oxidase II are shown in Figs. 20 and 21, respectively. Figs. 20 and 21 are shown by relative remaining activity determined when the maximum activity is defined as 100. In each of Figs. 20 and 21, panels A and B show thermal stability at pH 7.0, and the panels C and D being thermal stability at pH 9.0. Further, in each of Figs. 20 and 21, panels A and C show cases where 2,6-dimethoxyphenol was used as a substrate, and panels B and D show cases where para-phenylenediamine being used as a substrate.

As the results, as shown in Fig. 20, it was found that as the thermal stability at pH 7.0, the neutral phenol oxidase I had 80 % or more after 1-hour incubation at temperatures of up to 30°C, as compared with the maximum activity. Further, it was found that as the thermal stability at pH 9.0, the neutral phenol oxidase I had a relative remaining activity of 90 % or more after 1-hour incubation at temperatures of up to 30°C, as compared with the maximum activity.

In addition, as shown in Fig. 21, it was found that as the thermal stability at pH 7.0, the neutral phenol oxidase II had a relative remaining activity of 90 % or more after 1-hour incubation at temperatures of up to 50°C, as compared with the maximum activity. Further, it was found that as thermal stability at pH 9.0, the neutral phenol oxidase II had a relative remaining activity of 70 % or more after 1-hour incubation at temperatures of up to 50°C, as compared with the maximum activity.

In addition, the thermal stability of the neutral phenol oxidase III was examined at pH 7.0 and pH 9.0.

When the thermal stability at pH 7.0 of the neutral phenol oxidase III was examined, pretreatment was carried out by incubating 0.0125 ml of the fraction of the neutral phenol oxidase III obtained in the above item (7) of Example 1 in 0.0375 ml of 0.2 M sodium phosphate buffer (pH 7.0) (total volume 0.05 milliliter) at various temperatures (20, 30, 40, 50, 60, 70, and 80°C) for various periods of time (5, 10, 20, 40, and 60 minutes).

When 2,6-dimethoxyphenol was used as a substrate, the enzymatic activity was evaluated by mixing adequately 0.02 ml of the pre-treated enzyme solution, 0.88 ml of 0.2 M sodium phosphate buffer (pH 6.5), and 0.1 ml of a 0.05 M aqueous solution of 2,6-dimethoxyphenol in a cuvette, and then measuring a change in the absorbance at 470 nm of the resulting mixture using a spectrophotometer (manufactured by Jasco; trade name: v-530).

When para-phenylenediamine was used as a substrate, enzymatic activity was evaluated by carrying out procedures in the same manner as those of the above-mentioned evaluation method in which 2,6-dimethoxyphenol was used as a substrate, except that a 0.05 M aqueous solution of para-phenylenediamine was used instead of the aqueous solution of 2,6-dimethoxyphenol, and measuring a change in the absorbance at 470nm in the same manner.

Here, regarding the enzymatic activity, an amount for increasing the absorbance by 1 per one minute at 470 nm is defined as 1 unit (U).

When the thermal stability at pH 9.0 of the neutral phenol oxidase III was determined, pretreatment was carried out by incubating 0.0125 ml of the fraction of the neutral phenol oxidase III obtained in the above item (7) of Example 1 in 0.0375 ml of 0.2 M Tris-HCl buffer (pH 9.0) (total volume 0.05 ml) at various temperatures (20, 30, 40, 50, 60, 70, and 80°C) for various periods of time (5, 10, 20, 40, and 60 minutes) instead of the pretreatment at pH 7.0.

The result of the thermal stability of the neutral phenol oxidase III is shown in Fig. 22. Fig. 22 is shown by relative remaining activity determined when the enzymatic activity before incubation is defined as 100. Panels A and B of Fig. 22 show thermal stability at pH 7.0, and panels C and D show thermal stability at pH 9.0. In addition, in Fig. 22, panels A and C show cases where 2,6-dimethoxyphenol was used as a substrate, and panels B and D being cases where para-phenylenediamine was used as a substrate.

As shown in Fig. 22, it was found that as a thermal stability, the neutral phenol oxidase III had a relative remaining activity of 80 % or more after 1-hour incubation at temperatures of up to 30°C at pH 7.0, as compared with the enzymatic activity before incubation. In addition, it was found that as a thermal stability, the neutral phenol oxidase III had a relative remaining activity of 90 % or more after 1-hour incubation at temperatures of up to 40°C at pH 9.0, as compared with the enzymatic activity before incubation.

### Example 8 Substrate specificity of each of neutral phenol oxidase I, neutral phenol oxidase II, and neutral phenol oxidase III of the present invention

In order to examine the substrate specificity of each of the neutral phenol oxidase I, the neutral phenol oxidase II, and the neutral phenol oxidase III of the present invention, the substrate specificity was examined using para-phenylenediamine, 4-hydroxyindole, 2-methoxyphenol, 2,6-dimethoxyphenol, catechol, para-aminophenol, ortho-aminophenol, syringaldazine, bilirubin, ABTS, NNS, and L-tyrosine as substrates.

When para-phenylenediamine, 4-hydroxyindole, 2-methoxyphenol, 2,6-dimethoxyphenol, catechol, ABTS, or NNS was used as a substrate for the neutral phenol oxidase I and the neutral phenol oxidase II of the present invention, the enzymatic activity was evaluated by mixing adequately 0.02 ml of the fraction of the neutral phenol oxidase I or the neutral phenol oxidase II obtained in the above item (5) of Example 1, 0.88 ml of 0.1 M sodium phosphate buffer (pH 6.5), and 0.1 ml of a 0.05 M aqueous substrate solution in a microcuvette, and then measuring a change in the absorbance at a wavelength pre-determined depending on the substrate of the obtained mixture using a spectrophotometer (manufactured by Jasco; trade name: V-530).

When para-aminophenol or ortho-aminophenol was used as a substrate, enzymatic activity was evaluated by carrying out procedures in the same manner as those of the above-mentioned method for evaluating enzymatic activity, except that a 0.05 M dimethylsulfoxide solution was used instead of the 0.05 M aqueous substrate solution, and then measuring a change in the absorbance at a wavelength give for each of substrates.

When syringaldazine was used as a substrate, using 0.005 M ethanol solution of syringaldazine instead of the 0.05M aqueous substrate solution, enzymatic activity was evaluated by mixing 0.02 ml of the fraction of the neutral phenol oxidase I or the neutral phenol oxidase II obtained in the above item (5) of Example 1, 0.96 ml of 0.1 M sodium phosphate buffer (pH 6.5), and 0.02 ml of a 0.005 M ethanol solution of syringaldazine in a microcuvette, and then measuring a change in the absorbance at 530 nm of the thus obtained mixture using a spectrophotometer (manufactured by Jasco; trade name: V-530).

When bilirubin was used as a substrate, enzymatic activity was evaluated by carrying out procedures in the same manner as those of the above-mentioned method for evaluating enzymatic activity, except that a 0.01 M dimethylsulfoxide solution were used instead of the 0.05M aqueous substrate solution, and then measuring a change in the absorbance at 450 nm.

When L-tyrosine was used as a substrate, using 0.2 M sodium phosphate buffer (pH 6.5) containing 0.001 M L-tyrosine instead of the 0.05M aqueous substrate solution, enzymatic activity was evaluated by mixing 0.02 ml of the fraction of the neutral phenol oxidase I or the neutral phenol oxidase II obtained in the above item (5) of Example 1 and 0.98 ml of 0.2 M sodium phosphate buffer (pH 6.5) containing 0.001 M L-tyrosine in a microcuvette, and then measuring a change in the absorbance at 490 nm of the resulting mixture.

As for the neutral phenol oxidase III, when para-phenylenediamine, 4-hydroxyindole, 2-methoxyphenol, 2,6-dimethoxyphenol, catechol, or ABTS was used as a substrate, enzymatic activity was evaluated by mixing adequately 0.04 ml of the fraction of the neutral phenol oxidase III obtained in the above item (7) of Example 1, 0.86 ml of 0.2 M sodium phosphate buffer (pH 6.5), and 0.1 ml of a 0.05 M aqueous substrate solution in a cuvette, and then measuring a change in the absorbance at a wavelength given for each of substrates for the resulting mixture using a spectrophotometer (manufactured by Jasco; trade name: v-530).

When para-aminophenol or ortho-aminophenol was used as a substrate, enzymatic activity was evaluated by carrying out procedures in the same manner as that of the above method for evaluating enzymatic activity, except that a 0.05 M dimethylsulfoxide solution of the substrate was used instead of the 0.05 M aqueous substrate solution, and then measuring a change in the absorbance at a wavelength given for each of substrates.

When NNS was used as a substrate, enzymatic activity was evaluated by mixing adequately 0.04 ml of the fraction of the neutral phenol oxidase III obtained in the above item (7) of Example 1, 0.94 ml of 0.2 M sodium phosphate buffer (pH 6.5), and 0.02 ml of a 0.005 M aqueous NNS solution in a cuvette, and then measuring a change in the absorbance at 410 nm of the resulting mixture.

When syringaldazine was used as a substrate, enzymatic activity was evaluated by mixing adequately 0.04 ml of the fraction of the neutral phenol oxidase III obtained in the above item (7) of Example 1, 0.86 ml of 0.2 M sodium phosphate buffer (pH 6.5), and 0.1 ml of a 0.005 M ethanol solution of syringaldazine in a cuvette, and then measuring a change in the absorbance at 530 nm of the resulting mixture.

When bilirubin was used as a substrate, enzymatic activity was evaluated by mixing adequately 0.04 ml of the fraction of the neutral phenol oxidase III obtained in the above item (7) of Example 1, 0.94 ml of 0.2 M sodium phosphate buffer (pH 6.5), and 0.02 ml of a 0.0005 M dimethylsulfoxide solution of bilirubin in a cuvette, and then measuring a change in the absorbance at 450 nm of the resulting mixture.

Here, regarding the enzymatic activity, an amount for increasing the absorbance by 1 per one minute is defined as 1 unit (U).

The wave length for measurement and the results for the neutral phenol oxidase I, the neutral phenol oxidase II, and the neutral phenol oxidase III are shown in Tables 3, 4, and 5, respectively.

**Table 3**

| Neutral phenol oxidase I | | |
|---|---|---|
| Substrate | Wavelength for measurement (nm) | Activity(U) |
| Para-phenylenediamine | 470 | 0.038 |
| 4-hydroxy indole | 470 | 0.005 |
| 2-methoxy phenol | 470 | 0.022 |
| 2,6-dimethoxy phenol | 470 | 0.234 |
| Catechol | 400 | 0.016 |
| Para-amino phenol | 400 | 0.032 |
| Ortho-amino phenol | 420 | 0.072 |
| Syringaldazine | 530 | 0.596 |
| Bilirubin | 530 | -0.215 |
| ABTS | 420 | 0.320 |
| NNS | 490 | -0.017 |
| L-tyrosine | 490 | 0.000 |

**Table 4**

| Neutral phenol oxidase II | | |
|---|---|---|
| Substrate | Wavelength for measurement (nm) | Activity(U) |
| Para-phenylenediamine | 470 | 0.070 |
| 4-hydroxy indole | 470 | 0.016 |
| 2-methoxy phenol | 470 | 0.080 |
| 2,6-dimethoxy phenol | 470 | 0.676 |
| Catechol | 400 | 0.032 |
| Para-amino phenol | 400 | 0.068 |
| Ortho-amino phenol | 420 | 0.130 |
| syringaldazine | 530 | 1.014 |
| Bilirubin | 530 | -0.458 |
| ABTS | 420 | 0.752 |
| NNS | 490 | -0.032 |
| L-tyrosine | 490 | 0.000 |

**Table 5**

| Neutral phenol oxidase III | | |
|---|---|---|
| Substrate | Wavelength for measurement (nm) | Activity(U) |
| Para-phenylenediamine | 470 | 0.080 |
| 4-hydroxy indole | 470 | 0.022 |
| 2-methoxy phenol | 470 | 0.075 |
| 2,6-dimethoxy phenol | 470 | 0.557 |
| Catechol | 400 | 0.025 |
| Para-amino phenol | 400 | 0.072 |
| Ortho-amino phenol | 420 | 0.174 |
| syringaldazine | 530 | 1.142 |
| Bilirubin | 530 | -0.100 |
| ABTS | 420 | 0.847 |
| NNS | 490 | -0.015 |
| L-tyrosine | 490 | 0.000 |

From the results shown in Tables 3, 4, and 5, it was found that the neutral phenol oxidase I, the neutral phenol oxidase II, and the neutral phenol oxidase III possess phenol oxidase activity. More specifically, it is shown that since the neutral phenol oxidase I, the neutral phenol oxidase II, and the neutral phenol oxidase III catalyze the direct oxidation (coloring) reaction of syringaldazine or ABTS, the neutral phenol oxidase I, the neutral phenol oxidase II, and the neutral phenol oxidase III have laccase activity. In addition, it is also shown that since the neutral phenol oxidase I, the neutral phenol oxidase II, and the neutral phenol oxidase III catalyze the decomposition reaction of bilirubin due to direct oxidation, the neutral phenol oxidase I, the neutral phenol oxidase II, and the neutral phenol oxidase III have bilirubin oxidase activity. However, since the neutral phenol oxidase I and the neutral phenol oxidase II do not catalyze the direct oxidation (coloring) reaction of L-tyrosine, it is found that the neutral phenol oxidase I and the neutral phenol oxidase II do not possess tyrosinase activity.

In addition, since the neutral phenol oxidase I, the neutral phenol oxidase II, and the neutral phenol oxidase III oxidize ABTS and NNS which are phenol oxidase mediators, by the neutral phenol oxidase I, the neutral phenol oxidase II, and the neutral phenol oxidase III, it is possible to enhance an enzymatic reaction through the intermediation of these substrates as mediators, and to perform efficiently reactions which are conventionally difficult to catalyze (or reactions which are not to catalyze).

### Example 9 Substrate specificity of neutral phenol oxidase I, neutral phenol oxidase II, and neutral phenol oxidase III (Direct oxidation reaction)

The substrate specificity (direct oxidation reaction) to various compounds was determined using a coloring reaction caused by the oxidative polymerization of a substrate for each of the neutral phenol oxidase I and the neutral phenol oxidase II obtained in the above item (5) of Example 1.

As substrates, diamine compounds, aminophenol-based compounds, phenolic compounds, and other compounds (extracts) were used. As a substrate solution, a 0.01 M aqueous substrate solution (in the cases of other compounds, a 2.0 mg/ml aqueous substrate solution) containing 1.0 % (v/v) dimethylsulfoxide {10 %(v/v), in the cases of para-aminophenol, trimethylhydroquinone, naphthol compounds, and indole compounds} was used.

Concretely, 0.1 ml of 1.0 M sodium phosphate buffer (pH 6.5) or 0.1 ml of 1.0 M Tris-HCl buffer (pH 9.0) was added to 0.8 ml of an enzyme solution containing the above enzyme, and thereafter 0.1 ml of the 0.01 M aqueous substrate solution (2.0 mg/ml aqueous substrate solution in the cases of other compounds) was mixed with the resulting mixture. Then, a change in the absorbance of the resulting mixture was measured using an ultraviolet visible spectrophotometer (manufactured by Shimadzu; trade name: UV-2450) to determine enzymatic activity (coloring activity). Here, regarding the enzymatic activity, an amount for increasing the absorbance by 1 per one minute is defined as 1 unit (U). For a comparison, the substrate specificity of a bilirubin oxidase derived from a fungus of the genus *Myrothecium* was also examined at pH 6.5 and pH 9.0 in the same manner as described above.

The substrate specificity (direct oxidation reaction) of the neutral phenol oxidase III obtained in the above item (7) of Example 1 to various compounds was determined by a coloring reaction caused by the oxidative polymerization of a substrate.

As substrates, diamine compounds, aminophenol compounds, phenolic compounds, naphthol compounds, indole compounds, and other compounds (extracts) were used. As a substrate solution, a 0.01 M aqueous substrate solution (a 2.0 mg/ml aqueous substrate solution for other compounds) containing 1 % (v/v) dimethylsulfoxide {10 %(v/v) in the cases of para-aminophenol, trimethylhydroquinone, naphthol compounds, and indole compounds} was used.

Concretely, 0.005 ml of the fraction of the neutral phenol oxidase III obtained in the above item (7) of Example 1, 0.5 ml of 0.2 M sodium phosphate buffer (pH 6.5) or 0.5 ml of 0.2 M Tris-HCl buffer (pH 9.0), 0.395 ml of distilled ion-exchange water, and 0.1 ml of the above substrate aqueous solution were adequately mixed. Then, enzymatic activity was evaluated by measuring a change in the absorbance at a wavelength given for each of substrates for the resulting mixture using an ultraviolet visible spectrophotometer (manufactured by Shimadzu; trade name: UV-2450).

Here, regarding the enzymatic activity, an amount for increasing the absorbance by 1 per one minute at given wavelength for each of substrates is defined as 1 unit (U).

In Table 6, the result of direct oxidation reaction is shown by specific activity.

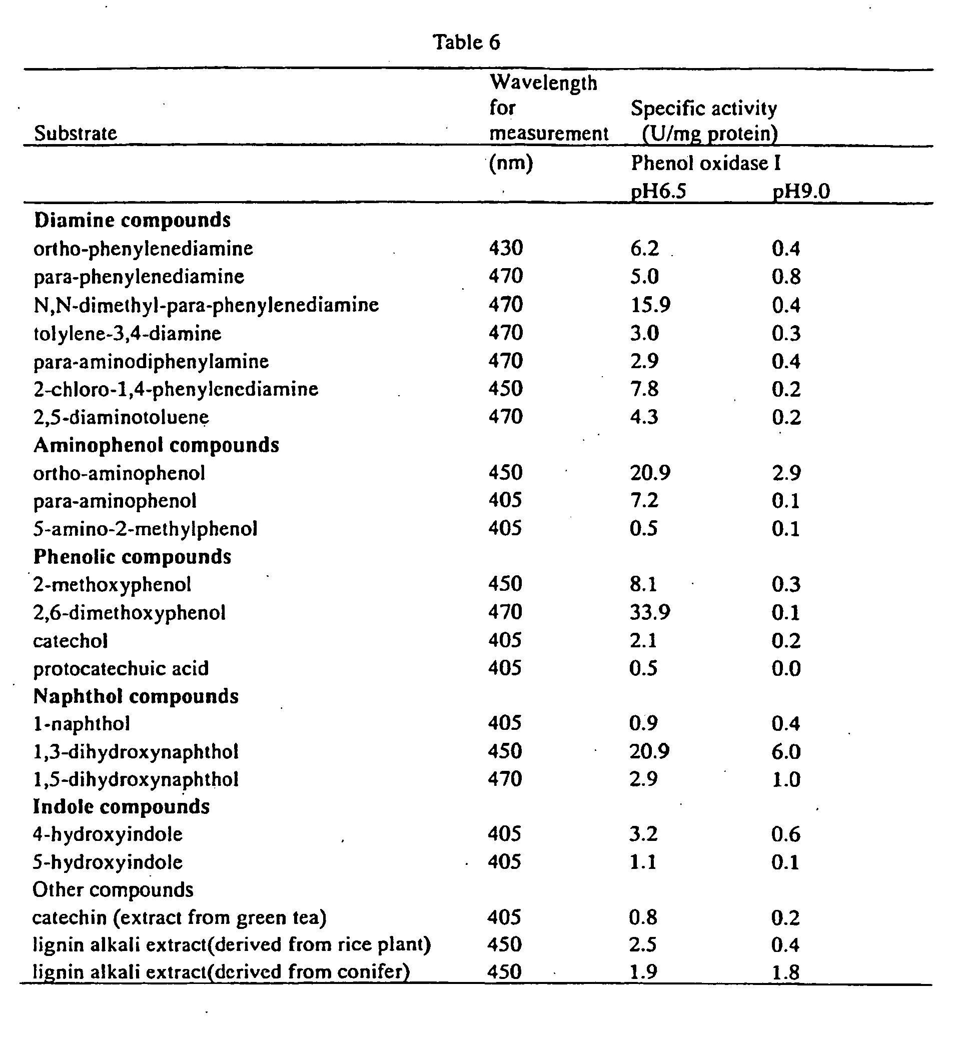

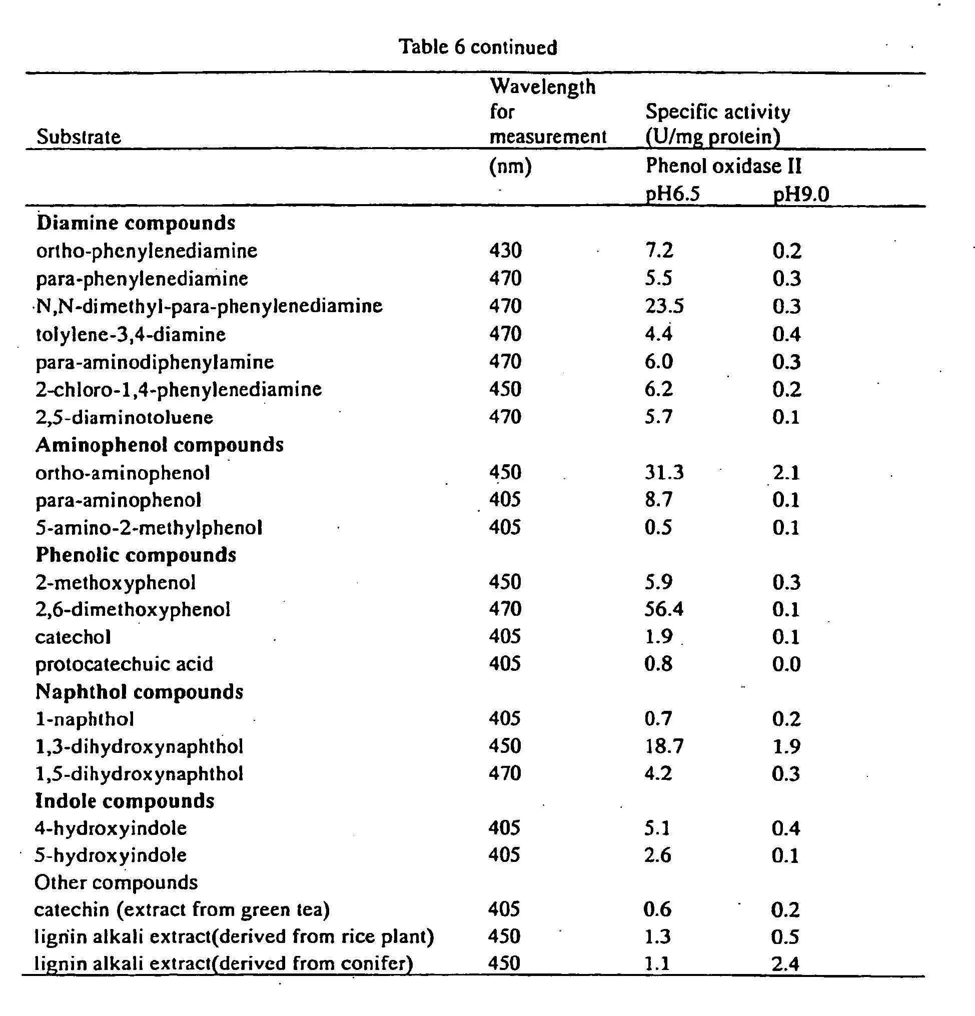

| Substrate | Wavelength for measurement | Specific activity (U/mg protein) | |
|---|---|---|---|
| | (nm) | Phenol oxidase III pH6.5 | pH9.0 |
| **Diamine compounds** | | | |
| ortho-phenylenediamine | 430 | 8.7 | 0.2 |
| para-phenylenediamine | 470 | 7.0 | 0.2 |
| N,N-dimethyl-para-phenylenediamine | 470 | 3.6 | 0.0 |
| tolylene-3,4-diamine | 470 | 0.8 | 0.0 |
| para-aminodiphenylamine | 470 | 2.9 | 0.0 |
| 2-chloro-1,4-phenylenediamine | 450 | 10.1 | 0.0 |
| 2,5-diaminotoluene | 470 | 5.2 | 0.0 |
| **Aminophenol compounds** | | | |
| ortho-aminophenol | 450 | 24.1 | 2.3 |
| para-aminophenol | 405 | 10.4 | 0.0 |
| 5-amino-2-methylphenol | 405 | 1.4 | 0.0 |
| **Phenolic compounds** | | | |
| 2-methoxyphenol | 450 | 2.0 | 0.2 |
| 2,6-dimethoxyphenol | 470 | 63.3 | 0.2 |
| catechol | 405 | 4.4 | 0.0 |
| protocatechuic acid | 405 | 2.1 | 0.9 |
| **Naphthol compounds** | | | |
| 1-naphthol | 405 | 0.7 | 0.1 |
| 1,3-dihydroxynaphthol | 450 | 15.5 | 1.9 |
| 1,5-dihydroxynaphthol | 470 | 2.8 | 0.0 |
| **Indole compounds** | | | |
| 4-hydroxyindole | 405 | 3.8 | 0.0 |
| 5-hydroxyindole | 405 | 2.6 | 0.0 |
| Other compounds | | | |
| catechin (extract from green tea) | 405 | 0.7 | 0.0 |
| lignin alkali extract(derived from rice plant) | 450 | 3.3 | 0.0 |
| lignin alkali extract(derived from conifer) | 450 | 3.7 | 0.0 |

| Substrate | Wavelength for measurement | Specific activity (U/mg protein) | |
|---|---|---|---|
| | (nm) | Bilirubin oxidase pH6.5 | pH9.0 |
| | | | |
| **Diamine compounds** | | | |
| ortho-phenylenediamine | 430 | 0.6 | 0.0 |
| para-phenylenediamine | 470 | 3.4 | 0.5 |
| N,N-dimethyl-para-phenylenediamine | 470 | 42.4 | 2.8 |
| tolylene-3,4-diamine | 470 | 0.6 | 0.1 |
| para-aminodiphenylamine | 470 | 15.4 | 1.2 |
| 2-chloro-1,4-phenylenediamine | 450 | 2.6 | 0.0 |
| 2,5-diaminotoluene | 470 | 3.2 | 0.1 |
| **Aminophenol compounds** | | | |
| ortho-aminophenol | 450 | 1.8 | 9.3 |
| para-aminophenol | 405 | 0.5 | 0.2 |
| 5-amino-2-methylphenol | 405 | 0.1 | 0.1 |
| **Phenolic compounds** | | | |
| 2-methoxyphenol | 450 | 0.0 | 0.4 |
| 2,6-dimethoxyphenol | 470 | 0.4 | 1.7 |
| catechol | 405 | 0.3 | 0.2 |
| protocatechuic acid | 405 | 0.6 | 0.6 |
| **Naphthol compounds** | | | |
| 1-naphthol | 405 | 0.1 | 1.0 |
| 1,3-dihydroxynaphthol | 450 | 12.6 | 16.7 |
| 1,5-dihydroxynaphthol | 470 | 3.3 | 5.1 |
| **Indole compounds** | | | |
| 4-hydroxyindole | 405 | 1.8 | 2.7 |
| 5-hydroxyindole | 405 | 0.1 | 0.1 |
| Other compounds | | | |
| catechin (extract from green tea) | 405 | 0.6 | 1.4 |
| lignin alkali extract(derived from rice plant) | 450 | 0.6 | 1.6 |
| lignin alkali extract(derived from conifer) | 450 | 3.5 | 3.8 |

As a result, as shown in Table 6, the neutral phenol oxidase I, the neutral phenol oxidase II, and the neutral phenol oxidase III strongly catalyzed the oxidation reactions of various substrates such as diamine compounds, aminophenol compounds, and phenolic compounds under neutral conditions (pH 6.5).

Further, the neutral phenol oxidase I showed the following substrate specificity:
1) When diamine compounds were used as substrates, the neutral phenol oxidase I catalyzed the oxidation reactions of all the substrates under neutral conditions (pH 6.5). Among them, the neutral phenol oxidase I particularly strongly catalyzed the oxidation reaction of N,N-dimethyl-paraphenylenediamine.
2) When aminophenol compounds were used as substrates, the neutral phenol oxidase I catalyzed the oxidation reaction of all the substrates under neutral conditions (pH 6.5). Among them, the neutral phenol oxidase I particularly strongly catalyzed the oxidation reaction of ortho-amiophenol.
3) When phenolic compounds were used as substrates, the neutral phenol oxidase I catalyzed the oxidation reactions of all the substrates under neutral conditions (pH 6.5). Among them, the neutral phenol oxidase I particularly strongly catalyzed the oxidation reaction of 2,6-dimethoxyphenol.
4) When naphthol compounds were used as substrates, the neutral phenol oxidase I catalyzed the oxidation reactions of all the substrates under neutral conditions (pH 6.5). Among them, the neutral phenol oxidase I particularly strongly catalyzed the oxidation reaction of 1,3-dihydroxynaphthalene.
5) When indole compounds were used as substrates, the neutral phenol oxidase I catalyzed the oxidation reactions of all the substrates under neutral conditions (pH 6.5).
6) When other compounds (extracts) were used as substrates, the neutral phenol oxidase I catalyzed the oxidation reactions of all the substrates under neutral conditions (pH 6.5).

Further, it was found that the neutral phenol oxidase I shows higher specific activities to various substrates under neutral conditions (pH 6.5) than under alkaline conditions (pH 9.0), when the specific activities under neutral conditions (pH 6.5) were compared with the specific activities under alkaline conditions (pH 9.0).

Also, when an alkali extract of lignin (derived from a conifer) was used as a substrate, the neutral phenol oxidase I showed a strong catalytic activity not only under neutral conditions (pH 6.5) but also comparable strong catalytic activity under alkaline conditions (pH 9.0).

In addition, since the bilirubin oxidase derived from a fungus of the genus *Myrothecium* shows higher specific activities to the substrates other than diamine compounds and catechol under alkaline conditions (pH 9.0) than under neutral conditions (pH 6.5), when the specific activities under neutral conditions (pH 6.5) were compared with the specific activities under alkaline conditions (pH 9.0), it was found that the bilirubin oxidase derived from a fungus of the genus *Myrothecium* is greatly different from the neutral phenol oxidase I.

The neutral phenol oxidase II showed the following substrate specificity:
1) When diamine compounds were used as substrates, the neutral phenol oxidase II catalyzed the oxidation reactions of all the substrates under neutral conditions (pH 6.5). Among them, the neutral phenol oxidase II particularly strongly catalyzed the oxidation reaction of N,N-dimethyl-paraphenylenediamine.
2) When aminophenol compounds were used as substrates, the neutral phenol oxidase II catalyzed the oxidation reactions of all the substrates under neutral conditions (pH 6.5). Among them, the neutral phenol oxidase II particularly strongly catalyzed the oxidation reaction of ortho-amiophenol.
3) When phenolic compounds were used as substrates, the neutral phenol oxidase II catalyzed the oxidation reactions of all the substrates under neutral conditions (pH 6.5). Among them, the neutral phenol oxidase II particularly strongly catalyzed the oxidation reaction of 2,6-dimethoxyphenol.
4) When naphthol compounds were used as substrates, the neutral phenol oxidase II catalyzed the oxidation reactions of all the substrates under neutral conditions (pH 6.5). Among them, the neutral phenol oxidase II particularly strongly catalyzed the oxidation reaction of 1,3-dihydroxynaphthalene.
5) When indole compounds were used as substrates, the neutral phenol oxidase II catalyzed the oxidation reactions of all the substrates under neutral conditions (pH 6.5).
6) When other compounds (extracts) were used as substrates, the neutral phenol oxidase II catalyzed the oxidation reactions of all the substrates under neutral conditions (pH 6.5).

Further, regarding the neutral phenol oxidase II, when the specific activities under neutral conditions (pH 6.5) were compared with the specific activities under alkaline conditions (pH 9.0), it was found that the specific activities to these various substrates under neutral conditions (pH 6.5) were higher than the specific activities under alkaline conditions (pH 9.0), except for alkali extract of lignin (derived from a conifer).

In addition, when alkali extract of lignin (derived from a conifer) was used as a substrate, the neutral phenol oxidase II showed a strong catalytic activity under neutral conditions (pH 6.5) but the neutral phenol oxidase II showed a stronger catalytic activity under alkaline conditions (pH 9.0).

On the other hand, it was found that the bilirubin oxidase derived from a fungus of the genus *Myrothecium* is greatly different from the neutral phenol oxidase II, since when the specific activities under neutral conditions (pH 6.5) were compared with the specific activities under alkaline conditions (pH 9.0), the bilirubin oxidase derived from a fungus of the genus *Myrothecium* shows the specific activities to the substrates other than diamine compounds and catechol under alkaline conditions (pH 9.0) were higher than the specific activities under neutral conditions (pH 6.5).

The neutral phenol oxidase III showed the following substrate specificity:
1) When diamine compounds were used as substrates, the neutral phenol oxidase III catalyzed the oxidation reactions of all the substrates under neutral conditions (pH 6.5). Among them, the neutral phenol oxidase III particularly strongly catalyzed the oxidation reaction of 2-chloro-1,4-phenylenediamine.
2) When aminophenol compounds were used as substrates, the neutral phenol oxidase III catalyzed the oxidation reactions of all the substrates under neutral conditions (pH 6.5). Among them, the neutral phenol oxidase III particularly strongly catalyzed the oxidation reaction of ortho-amiophenol.
3) When phenolic compounds were used as substrates, the neutral phenol oxidase III catalyzed the oxidation reactions of all the substrates under neutral conditions (pH 6.5). Among them, the neutral phenol oxidase III particularly strongly catalyzed the oxidation reaction of 2,6-dimethoxyphenol.
4) When naphthol compounds were used as substrates, the neutral phenol oxidase III catalyzed the oxidation reactions of all the substrates under neutral conditions (pH 6.5). Among them, the neutral phenol oxidase III particularly strongly catalyzed the oxidation reaction of 1,3-dihydroxynaphthalene.
5) When indole compounds were used as substrates, the neutral phenol oxidase III catalyzed the oxidation reactions of all the substrates under neutral conditions (pH 6.5).
6) When other compounds (extracts) were used as substrates, the neutral phenol oxidase III catalyzed the oxidation reactions of all the substrates under neutral conditions (pH 6.5).
   Further, regarding the neutral phenol oxidase III, when the specific activities under neutral conditions (pH 6.5) were compared with the specific activities under alkaline conditions (pH 9.0), it was found that the specific activities under neutral conditions (pH 6.5) were higher than the specific activities under alkaline conditions (pH 9.0).

On the other hand, regarding the bilirubin oxidase derived from a fungus of the genus *Myrothecium,* when the specific activities under neutral conditions (pH 6.5) were compared with the specific activities under alkaline conditions (pH 9.0), the specific activities to the substrates other than diamine compounds and catechol under alkaline conditions (pH 9.0) were higher than under neutral conditions (pH 6.5). Therefore, it was found that the bilirubin oxidase derived from a fungus of the genus *Myrothecium* is greatly different from the neutral phenol oxidase III.

According to each of the neutral phenol oxidase I, the neutral phenol oxidase II, and the neutral phenol oxidase III from the above-mentioned *Flammulina velutipes* IFO 30601 strain, various substrates can be efficiently oxidized under neutral conditions without changing reaction pH conditions depending on the reaction for a desired substrate. Therefore, since a reaction can be carried out under mild conditions that have little effect on the environment, the human body, and the like, it is to be expected that the neutral phenol oxidase I, the neutral phenol oxidase II, and the neutral phenol oxidase III will be widely applied to various fields such as production of phenolic resins, production of artificial lacquer paints, production of adhesives, synthesis of organic compounds, dyeing, waste water treatment, detoxication of toxic compounds, improvement of the quality of wood, manufacture of laminated wood, soil improvement, and the like.

### Example 10 Decolorizing activity of each of neutral phenol oxidase I, neutral phenol oxidase II, and neutral phenol oxidase III

The decolorizing activity of each of the neutral phenol oxidase I and the neutral phenol oxidase II obtained in the above item (5) of Example 1 and the neutral phenol oxidase III obtained in the above item (7) of Example 1 against various dyes was determined by measuring a change in the maximum value of ultraviolet-visible absorption spectrum of each dye.

Concretely, 0.1 ml of 0.1 M sodium phosphate buffer (pH 6.5) was added to 0.8 ml of an aqueous enzyme solution containing the neutral phenol oxidase I or the neutral phenol oxidase II obtained in the above item (5) of Example 1, and thereafter the resulting mixture was mixed with 0.1 ml of a 0.2 mg/mL aqueous dye solution. Then, enzymatic activity (decolorizing activity) was deteremined by measuring a change in the absorbance of the resulting mixture was measured using an ultraviolet visible spectrophotometer (manufactured by Shimadzu; trade name: UV-2450).

The decolorizing activity of the neutral phenol oxidase III obtained in the above item (7) of Example 1 against various dyes was also determined by a change in the maximum value of ultraviolet-visible absorption spectrum of each dye.

Concretely, 0.005 ml of the fraction of the neutral phenol oxidase III obtained in the above item (7) of Example 1, 0.395 ml of distilled ion-exchange water, 0.5 ml of 0.2 M sodium phosphate buffer (pH 6.5), and 0.1 ml of a 0.2 mg/ml aqueous dye solution were adequately mixed. Then, enzymatic activity (decolorizing activity) was determined by measuring a change in the absorbance of the thus obtained mixture was measured using an ultraviolet visible spectrophotometer (manufactured by Shimadzu; trade name: UV-2450).

Here, regarding the enzymatic activity, an amount for increasing the absorbance by 1 per one minute is defined as 1 unit (U). For comparison, the decolorizing activity of the bilirubin oxidase derived from a fungus of the genus *Myrothecium* was also determined.

The wave length for measurement for each dye and the results of decolorizing activity are shown by the specific activity in Table 7.

**Table 7**

| Dye | Wavelength for measurement(nm) | Specific activity (U/mg protein) | | | |
|---|---|---|---|---|---|
| | | Neutral phenol oxidase | | | Bilirubin oxidase derived from a fungus of the genus *Myrothecium* |
| | | I | II | III | |
| Natural Orange 6 | 450 | -0.06 | 0.04 | 0.00 | 0.01 |
| Acid orange 8 | 480 | 0.14 | 0.19 | 0.00 | -0.27 |
| Acid Violet 17 | 550 | -0.17 | -0.12 | 0.55 | 0.09 |
| Remazol Brilliant Blue R | 600 | 0.11 | 0.02 | 0.00 | -0.06 |
| Evans Blue | 630 | -0.86 | -0.23 | -0.31 | 1.03 |
| Acid Blue 80 | 630 | -0.50 | -0.07 | -0.31 | -0.37 |

As a result, as shown in Table 7, the neutral phenol oxidase I showed decolorizing activity against Natural Orange 6, Acid Violet 17, Evans Blue, and Acid Blue 80.

In addition, as shown in Table 7, the neutral phenol oxidase II showed decolorizing activity against Acid Violet 17, Evans Blue, and Acid Blue 80.

Additionally, as shown in Table 7, the neutral phenol oxidase III showed decolorizing activity against Evans Blue and Acid Blue 80.

Further, it was found that the neutral phenol oxidase I is different from the neutral phenol oxidase II and the neutral phenol oxidase III in decolorizing activity against Natural Orange 6.

In addition, it has been found that the neutral phenol oxidase III is different from the neutral phenol oxidase I and the neutral phenol oxidase II in decolorizing activity against Acid Violet 17.

On the other hand, the bilirubin oxidase derived from a fungus of the genus *Myrothecium* was greatly different from the neutral phenol oxidase I in decolorizing activity against Natural Orange 6, Acid Orange 8, Acid Violet 17, Remazol Brilliant Blue R, and Evans Blue. In addition, the bilirubin oxidase derived from a fungus of the genus *Myrothecium* was greatly different from the neutral phenol oxidase II in decolorizing activity against Acid Orange 8, Acid Violet 17, Remazol Brilliant Blue R, and Evans Blue. Also, the bilirubin oxidase derived from a fungus of the genus *Myrothecium* was greatly different from the neutral phenol oxidase III in decolorizing activity against Acid Orange 8, Remazol Brilliant Blue R, and Evans Blue.

The neutral phenol oxidase I, the neutral phenol oxidase II, and the neutral phenol oxidase III can efficiently decolorize dyes under neutral conditions. Therefore, it is to be expected that the neutral phenol oxidase I, the neutral phenol oxidase II, and the neutral phenol oxidase III will be widely applied to various fields such as waste water treatment, decolorization, prevention of color transfer during washing, and the like.

### Example 11 Hair dyeing test using neutral phenol oxidase I and neutral phenol oxidase II

A hair coloring test using the neutral phenol oxidase I or the neutral phenol oxidase II obtained in the above item (5) of Example 1 was carried out by dyeing a tuft of yak hair and wool cloth by means of oxidative polymerization using para-phenylenediamine as a dye.

Concretely, a composition was used in the hair coloring test, wherein the composition was obtained by adding to mix 0.5 g of para-phenylenediamine as a dye, 0.75 g of hydroxyethylcellulose as a thickener, 1.0 g of hydrogenated castor oil as a surfactant, and 0.25 g of cetanol and 0.25 g of myristyl alcohol as higher alcohols, adjusting the pH of the mixture to 7.0 with 0.5 g of lactic acid and monoethanolamine, and then adjusting the weight of the mixture to 50 g with ion-exchange water, to thereby obtain a composition. In addition, a mixture was applied to 1 g of a tuft of yak hair and a piece of wool cloth (2 x 3 cm), and the above tuft of yak hair and the wool cloth were left at 30°C for 1 hour, wherein the mixture was produced through the addition of 2 g of the composition and an enzyme solution of the neutral phenol oxidase I or the neutral phenol oxidase II so as to give an enzymatic activity against para-phenylenediamine of 1.0 U at pH 7.0 was applied to 1 g of a tuft of yak hair and a piece of wool cloth (2 x 3 cm), and they were left at 30°C for 1 hour.

As a result, the tuft of yak hair and the wool cloth were dyed black by the use of the neutral phenol oxidase I or the neutral phenol oxidase II.

The neutral phenol oxidase of the present invention can be used react efficiently and stably with various substrates, especially phenolic compounds, aminophenol compounds, and diamine compounds at pH around neutral region. In addition, according to the method of the present invention for producing the neutral phenol oxidase, it is possible to produce efficiently and easily the neutral phenol oxidase of the present invention in large quantity at low cost. Furthermore, according to the antibody of the present invention, the neutral phenol oxidase can be collected and purified easily. In addition, according to the dyeing method of the present invention, it is possible to dye fibers or to color hair easily and efficiently with various dyes, concretely with phenolic compounds, aminophenol compounds, diaminophenol compounds, and the like while reducing effects on the environment, the human body, and the like. Moreover, the use of the dyeing composition of the present invention makes it possible to dye fibers or to color hair easily and efficiently while reducing effects on the environment, the human body, and the like.

## Claims

1. A neutral phenol oxidase having the following properties:
(1) optimum pH: being from 5.0 to 7.0,
(2) substrate specificity:
i) catalyzing a coloring reaction by an oxidation of each of N,N-dimethyl-para-phenylenediamine, ortho-aminophenol, 2,6-dimethoxyphenol, 1,3-dihydroxynaphthol, and 4-hydroxyindole at a pH of around 6.5; and
ii) catalyzing an oxidative polymerization reaction of alkali extract of lignin;
(3) 28 kDa (calculated by SDS-PAGE);
(4) pH stability: maintaining a relative remaining activity of at least 70 % under incubation conditions at a pH of 8.0 to 9.0 for 20 hours at 30°C;
(5) optimum temperature: being from 30°C to 50°C;
(6) thermal stability:
I) maintaining a relative remaining activity of at least 80 % under incubation conditions at 0°C to 30°C for 1 hour at a pH of 7.0, as compared with an activity before incubation; and
II)maintaining a relative remaining activity of at least 90 % under incubation conditions at 0°C to 30°C for 1 hour at a pH of 9.0, as compared with an activity before incubation; and
(7) isoelectric point: being 7.4.

2. The neutral phenol oxidase according to claim 1, wherein the neutral phenol oxidase is produced by a basidiomycete belonging to the genus *Flammulina*.

3. The neutral phenol oxidase according to claim 1 or 2, wherein the basidiomycete belonging to the genus *Flammulina* is a basidiomycete belonging to *Flammulina velutipes*.

4. The neutral phenol oxidase according to any one of claims 1 to 3, wherein the basidiomycete belonging to *Flammulina velutipes* is *Flammulina velutipes* IFO 30601 strain.

5. A production method of a neutral phenol oxidase, **characterized in** than (A) a basidiomycete belonging to the genus *Flammulina* velutipes IFO 30601 is cultured at a pH of 6.0 to 12.0; and a neutral phenol oxidase is separated from a culture supernatant of a culture obtained in step (A) or an extract obtained from the culture; wherein the neutral phenol oxidase is the neutral phenol oxidase of claim 1.

6. An antibody raised against the neutral phenol oxidase of any one of claims 1 to 4.

7. A dyeing composition comprising the neutral phenol oxidase of any one of claims 1 to 4.

8. The dyeing composition according to claim 7, further comprising a dye.

9. A dyeing method, **characterized in that** a subject to be dyed is brought into contact with a dye in the presence of the neutral phenol oxidase of any one of claims 1 to 4, thereby dyeing the subject to be dyed.

## Patentansprüche

1. Neutrale Phenoloxidase mit den folgenden Eigenschaften:
(1) Optimaler pH-Wert: 5,0 bis 7,0,
(2) Substratspezifität:
i) Katalysieren einer Farbreaktion durch eine Oxidation von jeweils N,N-Dimethylparaphenylendiamin, Orthoaminophenol, 2,6-Dimethoxyphenol, 1,3-Dihydroxynaphthol und 4-Hydroxyindol bei einem pH-Wert von etwa 6,5; und
ii) Katalysieren einer oxidativen Polymerisationsreaktion eines Alkaliextrakts von Lignin;
(3) 28 kDa (berechnet nach SDS-PAGE),
(4) pH-Stabilität: Beibehalten einer relativen Restaktivität von mindestens 70% unter Inkubationsbedingungen bei einem pH-Wert von 8,0 bis 9,0 für 20 Stunden bei 30°C,
(5) Optimale Temperatur: 30°C bis 50°C,
(6) thermische Stabilität:
(I) Beibehalten einer relativen Restaktivität von mindestens 80% unter Inkubationsbedingungen bei 0°C bis 30°C für 1 Stunde bei einem pH-Wert von 7,0, verglichen mit einer Aktivität vor der Inkubation, und
(II) Beibehalten einer relativen Restaktivität von mindestens 90% unter Inkubationsbedingungen bei 0°C bis 30°C für 1 Stunde bei einem pH-Wert von 9,0, verglichen mit einer Aktivität vor der Inkubation, und
(7) Isoelektrischer Punkt: 7,4.

2. Neutrale Phenoloxidase gemäß Anspruch 1, wobei die neutrale Phenoloxidase durch ein zur Gattung *Flammulina* gehörendes Basidiomycet hergestellt wird.

3. Neutrale Phenoloxidase gemäß Anspruch 1 oder 2, wobei das zur Gattung *Flammulina* gehörende Basidiomycet ein zu *Flammulina velutipes* gehörendes Basidiomycet ist.

4. Neutrale Phenoloxidase gemäß einem der Ansprüche 1 bis 3, wobei das zu *Flammulina velutipes* gehörende Basidiomycet der *Flammulina velutipes* IFO 30601-Bakterienstamm ist.

5. Verfahren zur Herstellung einer neutralen Phenoloxidase, **dadurch gekennzeichnet, dass** (A) ein zur Gattung *Flammulina velutipes* IFO 30601 gehörendes Basidiomycet bei einem pH-Wert von 6,0 bis 12,0 kultiviert wird; und (B) eine neutrale Phenoloxidase von einem Kulturüberstand einer im Schritt (A) erhaltenen Kultur oder von einem aus der Kultur erhaltenen Extrakt gewonnen wird, wobei die neutrale Phenoloxidase die neutrale Phenoloxidase gemäß Anspruch 1 ist.

6. Antikörper, gerichtet gegen die neutrale Phenoloxidase gemäß einem der Ansprüche 1 bis 4.

7. Färbezusammensetzung, umfassend die neutrale Phenoloxidase gemäß einem der Ansprüche 1 bis 4.

8. Färbezusammensetzung gemäß Anspruch 7, ferner umfassend einen Farbstoff.

9. Färbeverfahren, **dadurch gekennzeichnet, dass** ein zu färbender Gegenstand in Anwesenheit der neutralen Phenoloxidase gemäß einem der Ansprüche 1 bis 4 mit einem Farbstoff in Kontakt gebracht wird, wobei der zu färbende Gegenstand gefärbt wird.

## Revendications

1. Phénol oxydase neutre ayant les propriétés suivantes :
(1) pH optimal : de 5,0 à 7,0,
(2) spécificité de substrat :
i) catalyse une réaction de coloration par oxydation de chacun des composés choisis parmi N,N-diméthyl-paraphénylènediamine, ortho-aminophénol, 2,6-diméthoxyphénol, 1,3-dihydroxynaphtol, et 4-hydroxyindole à un pH d'environ 6,5, et
ii) catalyse une réaction de polymérisation oxydative d'un extrait alcalin de lignine ;
(3) 28 kDa (calculé par SDS-PAGE),
(4) stabilité au pH : maintient une activité résiduelle relative d'au moins 70 % dans des conditions d'incubation à un pH de 8,0 à 9,0 pendant 20 heures à 30°C,
(5) température optimale : de 30°C à 50°C,
(6) stabilité thermique :
I) maintient une activité résiduelle relative d'au moins 80 % dans des conditions d'incubation à 0°C à 30°C pendant 1 heure à un pH de 7,0 par rapport à une activité avant incubation, et
II) maintient une activité résiduelle relative d'au moins 90 % dans des conditions d'incubation à 0°C à 30°C pendant 1 heure à un pH de 9,0, par rapport à une activité avant incubation, et
(7) point isoélectrique : 7,4.

2. Phénol oxydase neutre selon la revendication 1, dans laquelle la phénol oxydase neutre est produite par un basidiomycète appartenant au genre *Flammulina.*

3. Phénol oxydase neutre selon la revendication 1 ou 2, dans laquelle le basidiomycète appartenant au genre *Flammulina* est un basidiomycète appartenant à *Flammulina velutipes.*

4. Phénol oxydase neutre selon l'une quelconque des revendications 1 à 3, dans laquelle le basidiomycète appartenant à *Flammulina velutipes* est la souche de *Flammulina velutipes* IFO 30601.

5. Procédé de production d'une phénol oxydase neutre, **caractérisé en ce que**
(A) un basidiomycète appartenant au genre *Flammulina velutipes* IFO 30601 est cultivé à un pH de 6,0 à 12,0,
(B) une phénol oxydase neutre est séparée d'un surnageant d'une culture obtenue dans la phase (A) ou d'un extrait obtenu à partir de la culture ;
dans lequel la phénol oxydase neutre est la phénol oxydase neutre de la revendication 1.

6. Anticorps dirigé contre la phénol oxydase neutre selon l'une quelconque des revendications 1 à 4.

7. Composition colorante comprenant la phénol oxydase neutre selon l'une quelconque des revendications 1 à 4.

8. Composition colorante selon la revendication 7, comprenant en outre un colorant.

9. Procédé de coloration, **caractérisé en ce qu'**un sujet à colorer est mis en contact avec un colorant en présence de la phénol oxydase neutre selon l'une quelconque des revendications 1 à 4, colorant ainsi le sujet à colorer.
